(19) Europäisches Patentamt
European Patent Office
Office européen des brevets

(11) EP 3 483 162 B1

(12) **EUROPEAN PATENT SPECIFICATION**

(45) Date of publication and mention of the grant of the patent:
**06.10.2021 Bulletin 2021/40**

(21) Application number: **17820330.3**

(22) Date of filing: **30.06.2017**

(51) Int Cl.:
***C07D 403/04*** (2006.01) ***C07D 413/04*** (2006.01)
***C07D 471/04*** (2006.01) ***C07D 487/04*** (2006.01)
***C07D 513/04*** (2006.01) ***C07D 519/00*** (2006.01)
***A61K 31/4184*** (2006.01) ***A61K 31/423*** (2006.01)
***A61K 31/437*** (2006.01) ***A61K 31/5025*** (2006.01)
***A01N 43/90*** (2006.01) ***A61P 33/00*** (2006.01)

(86) International application number:
**PCT/JP2017/024158**

(87) International publication number:
**WO 2018/003976 (04.01.2018 Gazette 2018/01)**

(54) **N-ALKYLSULFONYL CONDENSED HETEROCYCLIC COMPOUND OR SALT THEREOF, PESTICIDE CONTAINING SAID COMPOUND, AND METHOD FOR USING SAID PESTICIDE**

N-ALKYLSULFONYL-KONDENSIERTE HETEROCYCLISCHE VERBINDUNG ODER SALZ DAVON, PESTIZID MIT BESAGTER VERBINDUNG UND VERFAHREN ZUR VERWENDUNG DES BESAGTEN PESTIZIDS

COMPOSÉ HÉTÉROCYCLIQUE N-ALKYLSULFONYLE FUSIONNÉ OU LEUR SELS, PESTICIDE CONTENANT LEDIT COMPOSÉ ET PROCÉDÉ D'UTILISATION DUDIT PESTICIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.07.2016 JP 2016131280**
**15.09.2016 JP 2016180927**

(43) Date of publication of application:
**15.05.2019 Bulletin 2019/20**

(73) Proprietor: **Nihon Nohyaku Co., Ltd.**
**Tokyo 104-8386 (JP)**

(72) Inventors:
- **ABE, Yutaka**
**Kawachinagano-shi**
**Osaka 586-0094 (JP)**
- **YAMAUCHI, Chiaki**
**Kawachinagano-shi**
**Osaka 586-0094 (JP)**
- **SAKURAI, Yuhji**
**Kawachinagano-shi**
**Osaka 586-0094 (JP)**
- **FUJIHARA, Hirokazu**
**Kawachinagano-shi**
**Osaka 586-0094 (JP)**
- **MATSUO, Soichiro**
**Kawachinagano-shi**
**Osaka 586-0094 (JP)**
- **YONEMURA, Ikki**
**Kawachinagano-shi**
**Osaka 586-0094 (JP)**
- **SUWA, Akiyuki**
**Kawachinagano-shi**
**Osaka 586-0094 (JP)**
- **FUJIE, Shunpei**
**Kawachinagano-shi**
**Osaka 586-0094 (JP)**
- **TANAKA, Ryosuke**
**Kawachinagano-shi**
**Osaka 586-0094 (JP)**

(74) Representative: **Witte, Weller & Partner**
**Patentanwälte mbB**
**Postfach 10 54 62**
**70047 Stuttgart (DE)**

(56) References cited:
**WO-A1-95/18801** **WO-A1-2013/018928**
**WO-A1-2015/000715** **WO-A1-2016/039444**
**WO-A1-2016/091731** **WO-A1-2016/129684**
**WO-A1-2016/142327** **WO-A1-2016/162318**
**WO-A1-2017/061497** **WO-A2-2008/132434**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

## Description

TECHNICAL FIELD

The present invention relates to an insecticide comprising an N-alkylsulfonyl condensed heterocyclic compound or a salt thereof as an active ingredient, and a method for using the insecticide.

BACKGROUND ART

Various compounds have been examined for their potential as agricultural and horticultural insecticides, and among them, certain kinds of condensed heterocyclic compounds have been reported to be useful as insecticides (for example, see Patent Literature 1 to 8). In particular, Patent Literature 8 discloses a compound having two condensed heterocycles bound to each other. However, none of the compounds disclosed in the literature have an N-alkylsulfonyl condensed heterocycle as one of the condensed heterocycles.

CITATION LIST

Patent Literature

Patent Literature 1: JP-A 2009-280574
Patent Literature 2: JP-A 2010-275301
Patent Literature 3: JP-A 2011-79774
Patent Literature 4: JP-A 2012-131780
Patent Literature 5: WO 2012/086848
Patent Literature 6: WO 2013/018928
Patent literature 7: WO 2014/157600
Patent literature 8: WO 2016/091731

SUMMARY OF INVENTION

TECHNICAL PROBLEM

In crop production in the fields of agriculture, horticulture and the like, the damage caused by insect pests etc. is still immense, and insect pests resistant to existing insecticides have emerged. Under such circumstances, the development of novel insecticides and acaricides is desired.

SOLUTION TO PROBLEM

The present inventors conducted extensive research to develop a novel insecticide, particularly an agricultural and horticultural insecticide. As a result, the present inventors found that a compound represented by the general formula (1) of the present invention which has an N-alkylsulfonyl condensed heterocycle or a salt of the compound is highly effective as an insecticide. Based on this finding, the present inventors completed the present invention.

That is, the present invention includes the following.

[1] A compound represented by the general formula (1):

[Chem. 1]

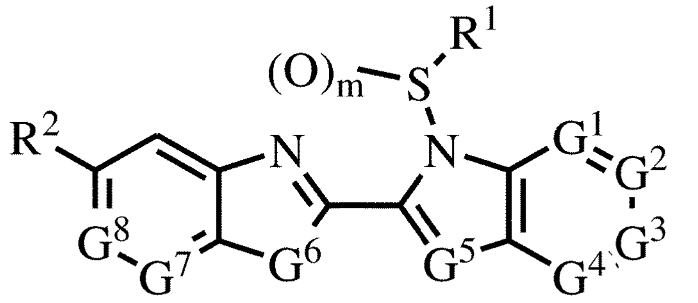

(1)

{wherein

$R^1$ represents

(a1) a $(C_1-C_6)$ alkyl group;
(a2) a $(C_3-C_6)$ cycloalkyl group;
(a3) a $(C_2-C_6)$ alkenyl group; or
(a4) a $(C_2-C_6)$ alkynyl group,

$R^2$ represents

(b1) a halogen atom;
(b2) a cyano group;
(b3) a nitro group;
(b4) a $(C_1-C_6)$ alkyl group;
(b5) a $(C_1-C_6)$ alkoxy group;
(b6) a $(C_2-C_6)$ alkenyloxy group;
(b7) a $(C_2-C_6)$ alkynyloxy group;
(b8) a halo $(C_1-C_6)$ alkyl group;
(b9) a halo $(C_1-C_6)$ alkoxy group;
(b10) a halo $(C_2-C_6)$ alkenyloxy group;
(b11) a halo $(C_2-C_6)$ alkynyloxy group;
(b12) a $(C_1-C_6)$ alkylthio group;
(b13) a $(C_1-C_6)$ alkylsulfinyl group;
(b14) a $(C_1-C_6)$ alkylsulfonyl group;
(b15) a halo $(C_1-C_6)$ alkylthio group;
(b16) a halo $(C_1-C_6)$ alkylsulfinyl group; or
(b17) a halo $(C_1-C_6)$ alkylsulfonyl group,

$G^1$, $G^2$, $G^3$ and $G^4$ independently represent C-$R^3$ or N (wherein each $R^3$ independently represents

(c1) a hydrogen atom;
(c2) a halogen atom;
(c3) a hydroxyl group;
(c4) a formyl group;
(c5) a cyano group;
(c6) a $(C_1-C_6)$ alkyl group;
(c7) a $(C_3-C_6)$ cycloalkyl group;
(c8) a $(C_1-C_6)$ alkoxy group;
(c9) a halo $(C_1-C_6)$ alkyl group;
(c10) a halo $(C_3-C_6)$ cycloalkyl group;
(c11) a halo $(C_1-C_6)$ alkoxy group;
(c12) a $(C_1-C_6)$ alkylthio group;

(c13) a ($C_1$-$C_6$) alkylsulfinyl group;
(c14) a ($C_1$-$C_6$) alkylsulfonyl group;
(c15) a halo ($C_1$-$C_6$) alkylthio group;
(c16) a halo ($C_1$-$C_6$) alkylsulfinyl group;
(c17) a halo ($C_1$-$C_6$) alkylsulfonyl group;
(c18) a ($C_3$-$C_6$) cycloalkyl ($C_1$-$C_6$) alkoxy group;
(c19) an $R^9(R^{10})N$ group (wherein $R^9$ and $R^{10}$ independently represent a hydrogen atom; a ($C_1$-$C_6$) alkyl group; a ($C_3$-$C_6$) cycloalkyl ($C_1$-$C_6$) alkyl group; a halo ($C_1$-$C_6$) alkyl group; a ($C_1$-$C_6$) alkylcarbonyl group; a ($C_1$-$C_6$) alkoxycarbonyl group; or a phenylcarbonyl group);
(c20) a mono ($C_1$-$C_6$) alkylaminocarbonyl NH group;
(c21) a mono ($C_1$-$C_6$) alkylaminothiocarbonyl NH group;
(c22) an $R^{10}ON=C(R^9)$ group (wherein $R^9$ and $R^{10}$ are as defined above);
(c23) a carboxyl group;
(c24) a ($C_1$-$C_6$) alkoxycarbonyl group;
(c25) an $R^9$ ($R^{10}$) N carbonyl group (wherein $R^9$ and $R^{10}$ are as defined above);
(c26) an aryl group;
(c27) an aryl group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c28) an aryl ($C_1$-$C_6$) alkyl group;
(c29) an aryl ($C_1$-$C_6$) alkyl group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c30) an aryl ($C_1$-$C_6$) alkoxy group;
(c31) an aryl ($C_1$-$C_6$) alkoxy group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c32) an aryl ($C_1$-$C_6$) alkyl NH group;
(c33) an aryl ($C_1$-$C_6$) alkyl NH group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c34) a heterocyclic group;
(c35) a heterocyclic group having, on the ring, 1 to 3 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group; or
(c36) a ($C_1$-$C_6$) alkoxycarbonyl NH group),

$G^5$ represents C-$R^4$ or N
(wherein
$R^4$ represents

(d1) a hydrogen atom;
(d2) a halogen atom;
(d3) a ($C_1$-$C_6$) alkyl group;
(d4) a ($C_3$-$C_6$) cycloalkyl group;
(d5) a ($C_2$-$C_6$) alkenyl group;
(d6) a ($C_2$-$C_6$) alkynyl group;
(d7) a ($C_1$-$C_6$) alkoxy group;
(d8) a halo ($C_1$-$C_6$) alkyl group;

(d9) a halo ($C_3$-$C_6$) cycloalkyl group;
(d10) a halo ($C_2$-$C_6$) alkenyl group;
(d11) a halo ($C_2$-$C_6$) alkynyl group;
(d12) a halo ($C_1$-$C_6$) alkoxy group;
(d13) a cyano group;
(d14) an $R^9$ ($R^{10}$) N group (wherein $R^9$ and $R^{10}$ are as defined above) ;
(d15) a ($C_1$-$C_6$) alkylthio group;
(d16) a ($C_1$-$C_6$) alkylsulfinyl group;
(d17) a ($C_1$-$C_6$) alkylsulfonyl group;
(d18) a ($C_1$-$C_6$) alkylthio group;
(d19) a formyl group;
(d20) a ($C_1$-$C_6$) alkoxycarbonyl group;
(d21) an $R^9$ ($R^{10}$) N carbonyl group (wherein $R^9$ and $R^{10}$ are as defined above); or
(d22) an $R^{10}ON=C(R^9)$ group (wherein $R^9$ and $R^{10}$ are as defined above)),

$G^6$ represents N-$R^5$; O; or S
(wherein
$R^5$ represents

(e1) a hydrogen atom;
(e2) a ($C_1$-$C_6$) alkyl group;
(e3) a ($C_3$-$C_6$) cycloalkyl group;
(e4) a ($C_2$-$C_6$) alkenyl group; or
(e5) a ($C_2$-$C_6$) alkynyl group),

$G^7$ and $G^8$ independently represent C-H or N, and
m represents an integer of 0 to 2}, or

a salt thereof.
[2] The compound or the salt according to the above [1], wherein

$R^1$ is (a1) a ($C_1$-$C_6$) alkyl group,
$R^2$ is

(b8) a halo ($C_1$-$C_6$) alkyl group;
(b15) a halo ($C_1$-$C_6$) alkylthio group; or
(b17) a halo ($C_1$-$C_6$) alkylsulfonyl group,

each $R^3$ is independently

(c1) a hydrogen atom;
(c2) a halogen atom;
(c4) a formyl group;
(c5) a cyano group;
(c6) a ($C_1$-$C_6$) alkyl group;
(c7) a ($C_3$-$C_6$) cycloalkyl group;
(c8) a ($C_1$-$C_6$) alkoxy group;
(c9) a halo ($C_1$-$C_6$) alkyl group;
(c11) a halo ($C_1$-$C_6$) alkoxy group;
(c12) a ($C_1$-$C_6$) alkylthio group;
(c13) a ($C_1$-$C_6$) alkylsulfinyl group;
(c14) a ($C_1$-$C_6$) alkylsulfonyl group;
(c15) a halo ($C_1$-$C_6$) alkylthio group;
(c19) an $R^9(R^{10})N$ group (wherein $R^9$ and $R^{10}$ independently represent a hydrogen atom; a ($C_1$-$C_6$) alkyl group; a ($C_3$-$C_6$) cycloalkyl ($C_1$-$C_6$) alkyl group; a halo ($C_1$-$C_6$) alkyl group; a ($C_1$-$C_6$) alkylcarbonyl group; a ($C_1$-$C_6$) alkoxycarbonyl group; or a phenylcarbonyl group);
(c22) an $R^{10}ON=C(R^9)$ group (wherein $R^9$ and $R^{10}$ are as defined above);
(c24) a ($C_1$-$C_6$) alkoxycarbonyl group;

(c25) an $R^9$ ($R^{10}$) N carbonyl group (wherein $R^9$ and $R^{10}$ are as defined above);
(c27) an aryl group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group; or
(c36) a ($C_1$-$C_6$) alkoxycarbonyl NH group,

$R^4$ is

(d1) a hydrogen atom;
(d2) a halogen atom;
(d3) a ($C_1$-$C_6$) alkyl group;
(d4) a ($C_3$-$C_6$) cycloalkyl group;
(d7) a ($C_1$-$C_6$) alkoxy group;
(d12) a halo ($C_1$-$C_6$) alkoxy group;
(d13) a cyano group;
(d14) an $R^9$($R^{10}$)N group (wherein $R^9$ and $R^{10}$ independently represent a hydrogen atom; a ($C_1$-$C_6$) alkyl group; a ($C_3$-$C_6$) cycloalkyl ($C_1$-$C_6$) alkyl group; a halo ($C_1$-$C_6$) alkyl group; a ($C_1$-$C_6$) alkylcarbonyl group; a ($C_1$-$C_6$) alkoxycarbonyl group; or a phenylcarbonyl group);
(d15) a ($C_1$-$C_6$) alkylthio group;
(d16) a ($C_1$-$C_6$) alkylsulfinyl group;
(d17) a ($C_1$-$C_6$) alkylsulfonyl group;
(d18) a ($C_1$-$C_6$) alkylthio group;
(d19) a formyl group;
(d20) a ($C_1$-$C_6$) alkoxycarbonyl group;
(d21) an $R^9$ ($R^{10}$) N carbonyl group (wherein $R^9$ and $R^{10}$ are as defined above); or
(d22) an $R^{10}$ON=C($R^9$) group (wherein $R^9$ and $R^{10}$ are as defined above),

$G^6$ is N-$R^5$ or O, and
$R^5$ is (e2) a ($C_1$-$C_6$) alkyl group.

[3] The compound or the salt according to the above [1] or [2], wherein

$R^2$ is

(b8) a halo ($C_1$-$C_6$) alkyl group or
(b15) a halo ($C_1$-$C_6$) alkylthio group,

each $R^3$ is independently

(c1) a hydrogen atom;
(c2) a halogen atom;
(c8) a ($C_1$-$C_6$) alkoxy group;
(c9) a halo ($C_1$-$C_6$) alkyl group; or
(c11) a halo ($C_1$-$C_6$) alkoxy group, and

$R^4$ is

(d1) a hydrogen atom or
(d2) a halogen atom.

[4] An insecticide comprising the compound or the salt according to any of the above [1] to [3] as an active ingredient.
[5] An agricultural and horticultural insecticide comprising the compound or the salt according to any of the above [1] to [3] as an active ingredient.
[6] An animal ectoparasite control agent comprising the compound or the salt according to any of the above [1] to [3] as an active ingredient.
[7] A method for using an insecticide, comprising treating plants or soil with an effective amount of the insecticide

according to the above [4].

[9] A condensed heterocyclic compound represented by the general formula (1):

[Chem. 2]

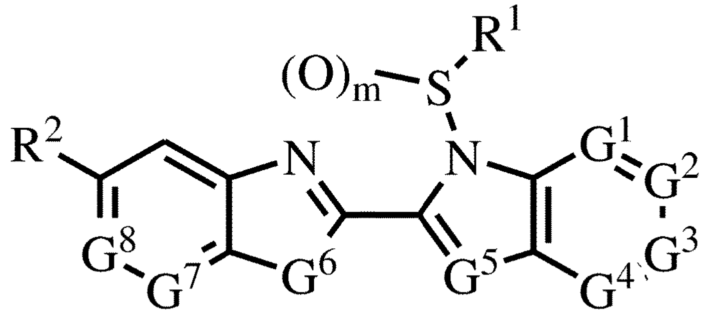

(1)

{wherein

$R^1$ represents

(a1) a ($C_1$-$C_6$) alkyl group;
(a2) a ($C_3$-$C_6$) cycloalkyl group;
(a3) a ($C_2$-$C_6$) alkenyl group; or
(a4) a ($C_2$-$C_6$) alkynyl group,

$R^2$ represents

(b1) a halogen atom;
(b2) a cyano group;
(b3) a nitro group;
(b4) a ($C_1$-$C_6$) alkyl group;
(b5) a ($C_1$-$C_6$) alkoxy group;
(b6) a ($C_2$-$C_6$) alkenyloxy group;
(b7) a ($C_2$-$C_6$) alkynyloxy group;
(b8) a halo ($C_1$-$C_6$) alkyl group;
(b9) a halo ($C_1$-$C_6$) alkoxy group;
(b10) a halo ($C_2$-$C_6$) alkenyloxy group;
(b11) a halo ($C_2$-$C_6$) alkynyloxy group;
(b12) a ($C_1$-$C_6$) alkylthio group;
(b13) a ($C_1$-$C_6$) alkylsulfinyl group;
(b14) a ($C_1$-$C_6$) alkylsulfonyl group;
(b15) a halo ($C_1$-$C_6$) alkylthio group;
(b16) a halo ($C_1$-$C_6$) alkylsulfinyl group; or
(b17) a halo ($C_1$-$C_6$) alkylsulfonyl group,

$G^1$, $G^2$, $G^3$ and $G^4$ may be the same or different and each represent C-$R^3$ or N (wherein
$R^3$s may be the same or different and represents

(c1) a hydrogen atom;
(c2) a halogen atom;
(c3) a hydroxyl group;
(c4) a formyl group;
(c5) a cyano group;
(c6) a ($C_1$-$C_6$) alkyl group;
(c7) a ($C_3$-$C_6$) cycloalkyl group;
(c8) a ($C_1$-$C_6$) alkoxy group;
(c9) a halo ($C_1$-$C_6$) alkyl group;

(c10) a halo ($C_3$-$C_6$) cycloalkyl group;
(c11) a halo ($C_1$-$C_6$) alkoxy group;
(c12) a ($C_1$-$C_6$) alkylthio group;
(c13) a ($C_1$-$C_6$) alkylsulfinyl group;
(c14) a ($C_1$-$C_6$) alkylsulfonyl group;
(c15) a halo ($C_1$-$C_6$) alkylthio group;
(c16) a halo ($C_1$-$C_6$) alkylsulfinyl group;
(c17) a halo ($C_1$-$C_6$) alkylsulfonyl group;
(c18) a ($C_3$-$C_6$) cycloalkyl ($C_1$-$C_6$) alkoxy group;
(c19) an $R^9$ ($R^{10}$) N group (wherein $R^9$ and $R^{10}$ may be the same or different and each represent a hydrogen atom; a ($C_1$-$C_6$) alkyl group; a ($C_3$-$C_6$) cycloalkyl ($C_1$-$C_6$) alkyl group; a halo ($C_1$-$C_6$) alkyl group; a ($C_1$-$C_6$) alkylcarbonyl group; a ($C_1$-$C_6$) alkoxycarbonyl group; or a phenylcarbonyl group);
(c20) a mono ($C_1$-$C_6$) alkylaminocarbonyl NH group;
(c21) a mono ($C_1$-$C_6$) alkylaminothiocarbonyl NH group;
(c22) a $C(R^9)=NOR^{10}$ group (wherein $R^9$ and $R^{10}$ are as defined above);
(c23) a carboxyl group;
(c24) a ($C_1$-$C_6$) alkoxycarbonyl group;
(c25) an $R^9$ ($R^{10}$) N carbonyl group (wherein $R^9$ and $R^{10}$ are as defined above);
(c26) an aryl group;
(c27) an aryl group having, on the ring, 1 to 5 substituting groups which may be the same or different and are selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c28) an aryl ($C_1$-$C_6$) alkyl group;
(c29) an aryl ($C_1$-$C_6$) alkyl group having, on the ring, 1 to 5 substituting groups which may be the same or different and are selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c30) an aryl ($C_1$-$C_6$) alkoxy group;
(c31) an aryl ($C_1$-$C_6$) alkoxy group having, on the ring, 1 to 5 substituting groups which may be the same or different and are selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c32) an aryl ($C_1$-$C_6$) alkyl NH group;
(c33) an aryl ($C_1$-$C_6$) alkyl NH group having, on the ring, 1 to 5 substituting groups which may be the same or different and are selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c34) a heterocyclic group; or
(c35) a heterocyclic group having, on the ring, 1 to 3 substituting groups which may be the same or different and are selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group),
$G^5$ represents C-$R^4$ or N

(wherein
$R^4$ represents

(d1) a hydrogen atom;
(d2) a halogen atom;
(d3) a ($C_1$-$C_6$) alkyl group;
(d4) a ($C_3$-$C_6$) cycloalkyl group;
(d5) a ($C_2$-$C_6$) alkenyl group;

(d6) a ($C_2$-$C_6$) alkynyl group;
(d7) a ($C_1$-$C_6$) alkoxy group;
(d8) a halo ($C_1$-$C_6$) alkyl group;
(d9) a halo ($C_3$-$C_6$) cycloalkyl group;
(d10) a halo ($C_2$-$C_6$) alkenyl group;
(d11) a halo ($C_2$-$C_6$) alkynyl group; or
(d12) a halo ($C_1$-$C_6$) alkoxy group),

$G^6$ represents N-$R^5$, 0 or S
(wherein
$R^5$ represents

(e1) a hydrogen atom;
(e2) a ($C_1$-$C_6$) alkyl group;
(e3) a ($C_3$-$C_6$) cycloalkyl group;
(e4) a ($C_2$-$C_6$) alkenyl group; or
(e5) a ($C_2$-$C_6$) alkynyl group),

$G^7$ and $G^8$ may be the same or different and each represent C-H or N, and
m represents an integer of 0 to 2}, or

a salt thereof.
[10] The condensed heterocyclic compound or the salt according to the above [9], wherein

$R^1$ represents (a1) a ($C_1$-$C_6$) alkyl group,
$R^2$ is

(b8) a halo ($C_1$-$C_6$) alkyl group;
(b15) a halo ($C_1$-$C_6$) alkylthio group; or
(b17) a halo ($C_1$-$C_6$) alkylsulfonyl group,

$G^1$, $G^2$, $G^3$ and $G^4$ each represent C-$R^3$
(wherein
$R^3$s may be the same or different and represents

(c1) a hydrogen atom;
(c2) a halogen atom;
(c4) a formyl group;
(c5) a cyano group;
(c6) a ($C_1$-$C_6$) alkyl group;
(c7) a ($C_3$-$C_6$) cycloalkyl group;
(c8) a ($C_1$-$C_6$) alkoxy group;
(c9) a halo ($C_1$-$C_6$) alkyl group;
(c12) a ($C_1$-$C_6$) alkylthio group;
(c15) a halo ($C_1$-$C_6$) alkylthio group;
(c19) an $R^9$($R^{10}$)N group (wherein $R^9$ and $R^{10}$ may be the same or different and each represent a hydrogen atom; a ($C_1$-$C_6$) alkyl group; a ($C_3$-$C_6$) cycloalkyl ($C_1$-$C_6$) alkyl group; a halo ($C_1$-$C_6$) alkyl group; a ($C_1$-$C_6$) alkylcarbonyl group; a ($C_1$-$C_6$) alkoxycarbonyl group; or a phenylcarbonyl group);
(c22) a C($R^9$)=NOR$^{10}$ group (wherein $R^9$ and $R^{10}$ are as defined above);
(c24) a ($C_1$-$C_6$) alkoxycarbonyl group; or
(c25) an $R^9$ ($R^{10}$) N carbonyl group (wherein $R^9$ and $R^{10}$ are as defined above)),

$G^5$ represents C-$R^4$ (wherein $R^4$ represents (d1) a hydrogen atom), and
$G^6$ represents N-$R^5$, O or S (wherein $R^5$ represents (e2) a ($C_1$-$C_6$) alkyl group) .

[11] The condensed heterocyclic compound or the salt according to the above [9] or [10], wherein

$R^2$ is

(b8) a halo ($C_1$-$C_6$) alkyl group or
(b15) a halo ($C_1$-$C_6$) alkylthio group,

$G^1$, $G^2$, $G^3$ and $G^4$ each represent C-$R^3$
(wherein
$R^3$s may be the same or different and represents

(c1) a hydrogen atom;
(c2) a halogen atom; or
(c9) a halo ($C_1$-$C_6$) alkyl group), and

$G^6$ represents N-$R^5$ or O (wherein $R^5$ represents (e2) a ($C_1$-$C_6$) alkyl group).

[12] An agricultural and horticultural insecticide comprising the condensed heterocyclic compound or the salt according to any of the above [9] to [11] as an active ingredient.
[13] A method for using an agricultural and horticultural insecticide, comprising treating plants or soil with an effective amount of the agricultural and horticultural insecticide according to the above [12].

ADVANTAGEOUS EFFECTS OF INVENTION

The compound of the present invention or a salt thereof is highly effective as an insecticide. The compound of the present invention or a salt thereof is effective not only against agricultural and horticultural pests but also against pests which live on pets such as dogs and cats and domestic animals such as cattle and sheep.

DESCRIPTION OF EMBODIMENTS

In the definitions of the general formula (1) representing the compound of the present invention, "halo" refers to a "halogen atom" and represents a chlorine atom, a bromine atom, an iodine atom or a fluorine atom.
The "($C_1$-$C_6$) alkyl group" refers to a straight-chain or branched-chain alkyl group of 1 to 6 carbon atoms, for example, a methyl group, an ethyl group, a n-propyl group, an isopropyl group, a n-butyl group, an isobutyl group, a sec-butyl group, a tert-butyl group, a n-pentyl group, an isopentyl group, a tert-pentyl group, a neopentyl group, a 2,3-dimethylpropyl group, a 1-ethylpropyl group, a 1-methylbutyl group, a 2-methylbutyl group, a n-hexyl group, an isohexyl group, a 2-hexyl group, a 3-hexyl group, a 2-methylpentyl group, a 3-methylpentyl group, a 1,1,2-trimethyl propyl group, a 3,3-dimethylbutyl group or the like.
The "($C_2$-$C_6$) alkenyl group" refers to a straight-chain or branched-chain alkenyl group of 2 to 6 carbon atoms, for example, a vinyl group, an allyl group, an isopropenyl group, a 1-butenyl group, a 2-butenyl group, a 2-methyl-2-propenyl group, a 1-methyl-2-propenyl group, a 2-methyl-1-propenyl group, a pentenyl group, a 1-hexenyl group, a 3,3-dimethyl-1-butenyl group or the like. The "($C_2$-$C_6$) alkynyl group" refers to a straight-chain or branched-chain alkynyl group of 2 to 6 carbon atoms, for example, an ethynyl group, a 1-propynyl group, a 2-propynyl group, a 1-butynyl group, a 2-butynyl group, a 3-butynyl group, a 3-methyl-1-propynyl group, a 2-methyl-3-propynyl group, a pentynyl group, a 1-hexynyl group, a 3-methyl-1-butynyl group, a 3,3-dimethyl-1-butynyl group or the like.
The "($C_3$-$C_6$) cycloalkyl group" refers to a cyclic alkyl group of 3 to 6 carbon atoms, for example, a cyclopropyl group, a cyclobutyl group, a cyclopentyl group, a cyclohexyl group or the like. The "($C_1$-$C_6$) alkoxy group" refers to a straight-chain or branched-chain alkoxy group of 1 to 6 carbon atoms, for example, a methoxy group, an ethoxy group, a n-propoxy group, an isopropoxy group, a n-butoxy group, a sec-butoxy group, a tert-butoxy group, a n-pentyloxy group, an isopentyloxy group, a tert-pentyloxy group, a neopentyloxy group, a 2,3-dimethylpropyloxy group, a 1-ethylpropyloxy group, a 1-methylbutyloxy group, a n-hexyloxy group, an isohexyloxy group, a 1,1,2-trimethylpropyloxy group or the like.
The "($C_2$-$C_6$) alkenyloxy group" refers to a straight-chain or branched-chain alkenyloxy group of 2 to 6 carbon atoms, for example, a propenyloxy group, a butenyloxy group, a pentenyloxy group, a hexenyloxy group or the like. The "($C_2$-$C_6$) alkynyloxy group" refers to a straight-chain or branched-chain alkynyloxy group of 2 to 6 carbon atoms, for example, a propynyloxy group, a butynyloxy group, a pentynyloxy group, a hexynyloxy group or the like.
The "($C_1$-$C_6$) alkylthio group" refers to a straight-chain or branched-chain alkylthio group of 1 to 6 carbon atoms, for example, a methylthio group, an ethylthio group, a n-propylthio group, an isopropylthio group, a n-butylthio group, a sec-butylthio group, a tert-butylthio group, a n-pentylthio group, an isopentylthio group, a tert-pentylthio group, a neopentylthio group, a 2,3-dimethylpropylthio group, a 1-ethylpropylthio group, a 1-methylbutylthio group, a n-hexylthio group, an isohexylthio group, a 1,1,2-trimethylpropylthio group or the like.
The "($C_1$-$C_6$) alkylsulfinyl group" refers to a straight-chain or branched-chain alkylsulfinyl group of 1 to 6 carbon atoms, for example, a methylsulfinyl group, an ethylsulfinyl group, a n-propylsulfinyl group, an isopropylsulfinyl group,

a n-butylsulfinyl group, a sec-butylsulfinyl group, a tert-butylsulfinyl group, a n-pentylsulfinyl group, an isopentylsulfinyl group, a tert-pentylsulfinyl group, a neopentylsulfinyl group, a 2,3-dimethylpropylsulfinyl group, a 1-ethylpropylsulfinyl group, a 1-methylbutylsulfinyl group, a n-hexylsulfinyl group, an isohexylsulfinyl group, a 1,1,2-trimethylpropylsulfinyl group or the like.

The "($C_1$-$C_6$) alkylsulfonyl group" refers to a straight-chain or branched-chain alkylsulfonyl group of 1 to 6 carbon atoms, for example, a methylsulfonyl group, an ethylsulfonyl group, a n-propylsulfonyl group, an isopropylsulfonyl group, a n-butylsulfonyl group, a sec-butylsulfonyl group, a tert-butylsulfonyl group, a n-pentylsulfonyl group, an isopentylsulfonyl group, a tert-pentylsulfonyl group, a neopentylsulfonyl group, a 2,3-dimethylpropylsulfonyl group, a 1-ethylpropylsulfonyl group, a 1-methylbutylsulfonyl group, a n-hexylsulfonyl group, an isohexylsulfonyl group, a 1,1,2-trimethylpropylsulfonyl group or the like.

The above-mentioned " ($C_1$-$C_6$) alkyl group", " ($C_2$-$C_6$) alkenyl group", " ($C_2$-$C_6$) alkynyl group", " ($C_1$-$C_6$) alkoxy group", " ($C_2$-$C_6$) alkenyloxy group", "($C_2$-$C_6$) alkynyloxy group", " ($C_1$-$C_6$) alkylthio group", "($C_1$-$C_6$) alkylsulfinyl group", "($C_1$-$C_6$) alkylsulfonyl group" and "($C_3$-$C_6$) cycloalkyl group" may be substituted with one or more halogen atoms at a substitutable position(s), and in the case where any of the above-listed groups is substituted with two or more halogen atoms, the halogen atoms may be the same or different.

The above-mentioned "groups substituted with one or more halogen atoms" are expressed as a "halo ($C_1$-$C_6$) alkyl group", a "halo ($C_2$-$C_6$) alkenyl group", a "halo ($C_2$-$C_6$) alkynyl group", a "halo ($C_1$-$C_6$) alkoxy group", a "halo ($C_2$-$C_6$) alkenyloxy group", a "halo ($C_2$-$C_6$) alkynyloxy group", a "halo ($C_1$-$C_6$) alkylthio group", a "halo ($C_1$-$C_6$) alkylsulfinyl group", a "halo ($C_1$-$C_6$) alkylsulfonyl group" and a "halo ($C_3$-$C_6$) cycloalkyl group".

The expressions "($C_1$-$C_6$)", "($C_2$-$C_6$)", "($C_3$-$C_6$)", etc. each refer to the range of the number of carbon atoms in each group. The same definition holds true for groups in which two or more of the above-mentioned groups are coupled together, and for example, the " ($C_1$-$C_6$) alkoxy ($C_1$-$C_6$) alkyl group" means that a straight-chain or branched-chain alkoxy group of 1 to 6 carbon atoms is bound to a straight-chain or branched-chain alkyl group of 1 to 6 carbon atoms.

The "aryl group" refers to an aromatic hydrocarbon group of 6 to 10 carbon atoms, for example, a phenyl group, a 1-naphthyl group, a 2-naphthyl group or the like.

The "heterocyclic group" and the "heterocyclic ring" refer to a 5- or 6-membered monocyclic aromatic heterocyclic group containing, as ring atoms, one or more carbon atoms and 1 to 4 heteroatoms selected from an oxygen atom, a sulfur atom and a nitrogen atom; or a 4- to 6-membered monocyclic non-aromatic heterocyclic group containing, as ring atoms, one or more carbon atoms and 1 to 4 heteroatoms selected from an oxygen atom, a sulfur atom and a nitrogen atom.

Examples of the "aromatic heterocyclic group" include monocyclic aromatic heterocyclic groups such as furyl, thienyl, pyridyl, pyrimidinyl, pyridazinyl, pyrazinyl, pyrrolyl, imidazolyl, pyrazolyl, thiazolyl, isothiazolyl, oxazolyl, isoxazolyl, oxadiazolyl, thiadiazolyl, triazolyl, tetrazolyl and triazinyl.

Examples of the "non-aromatic heterocyclic group" include monocyclic non-aromatic heterocyclic groups such as oxetanyl, thietanyl, azetidinyl, pyrrolidinyl, piperidinyl, morpholinyl, thiomorpholinyl, piperazinyl, hexamethyleneiminyl, oxazolidinyl, thiazolidinyl, imidazolidinyl, oxazolinyl, thiazolinyl, isoxazolinyl, imidazolinyl, dioxolyl, dioxolanyl, dihydrooxadiazolyl, 2-oxo-pyrrolidin-1-yl, 2-oxo-1,3-oxazolidin-5-yl, 5-oxo-1,2,4-oxadiazolin-3-yl, 1,3-dioxolan-2-yl, 1,3-dioxan-2-yl, 1,3-dioxepan-2-yl, pyranyl, tetrahydropyranyl, thiopyranyl, tetrahydrothiopyranyl, 1-oxide tetrahydrothiopyranyl, 1,1-dioxide tetrahydrothiopyranyl, tetrahydrofuranyl, dioxanyl, pyrazolidinyl, pyrazolinyl, tetrahydropyrimidinyl, dihydrotriazolyl and tetrahydrotriazolyl.

Examples of the salt of the compound represented by the general formula (1) of the present invention include inorganic acid salts, such as hydrochlorides, sulfates, nitrates and phosphates; organic acid salts, such as acetates, fumarates, maleates, oxalates, methanesulfonates, benzenesulfonates and p-toluenesulfonates; and salts with an inorganic or organic base such as a sodium ion, a potassium ion, a calcium ion and a trimethylammonium ion.

The compound represented by the general formula (1) of the present invention and a salt thereof can have one or more chiral centers in the structural formula, and can exist as two or more kinds of optical isomers or diastereomers. All the optical isomers and mixtures of the isomers at any ratio are also included in the present invention. Further, the compound represented by the general formula (1) of the present invention and a salt thereof can exist as two kinds of geometric isomers due to a carbon-carbon double bond in the structural formula. All the geometric isomers and mixtures of the isomers at any ratio are also included in the present invention.

Preferable embodiments of the compound represented by the general formula (1) of the present invention are described below.

$R^1$ is preferably (a1) a ($C_1$-$C_6$) alkyl group.
$R^2$ is preferably

(b8) a halo ($C_1$-$C_6$) alkyl group;
(b15) a halo ($C_1$-$C_6$) alkylthio group; or
(b17) a halo ($C_1$-$C_6$) alkylsulfonyl group,

and more preferably
(b8) a halo ($C_1$-$C_6$) alkyl group or
(b15) a halo ($C_1$-$C_6$) alkylthio group.

Each $R^3$ is independently

(c1) a hydrogen atom;
(c2) a halogen atom;
(c4) a formyl group;
(c5) a cyano group;
(c6) a ($C_1$-$C_6$) alkyl group;
(c7) a ($C_3$-$C_6$) cycloalkyl group;
(c8) a ($C_1$-$C_6$) alkoxy group;
(c9) a halo ($C_1$-$C_6$) alkyl group;
(c11) a halo ($C_1$-$C_6$) alkoxy group;
(c12) a ($C_1$-$C_6$) alkylthio group;
(c13) a ($C_1$-$C_6$) alkylsulfinyl group;
(c14) a ($C_1$-$C_6$) alkylsulfonyl group;
(c15) a halo ($C_1$-$C_6$) alkylthio group;
(c19) an $R^9(R^{10})N$ group (wherein $R^9$ and $R^{10}$ independently represent a hydrogen atom; a ($C_1$-$C_6$) alkyl group; a ($C_3$-$C_6$) cycloalkyl ($C_1$-$C_6$) alkyl group; a halo ($C_1$-$C_6$) alkyl group; a ($C_1$-$C_6$) alkylcarbonyl group; a ($C_1$-$C_6$) alkoxycarbonyl group; or a phenylcarbonyl group);
(c22) an $R^{10}ON=C(R^9)$ group (wherein $R^9$ and $R^{10}$ are as defined above);
(c24) a ($C_1$-$C_6$) alkoxycarbonyl group;
(c25) an $R^9$ ($R^{10}$) N carbonyl group (wherein $R^9$ and $R^{10}$ are as defined above);
(c27) an aryl group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group; or
(c36) a ($C_1$-$C_6$) alkoxycarbonyl NH group,

and more preferably

(c1) a hydrogen atom;
(c2) a halogen atom;
(c8) a ($C_1$-$C_6$) alkoxy group;
(c9) a halo ($C_1$-$C_6$) alkyl group; or
(c11) a halo ($C_1$-$C_6$) alkoxy group.

$R^4$ is preferably

(d1) a hydrogen atom;
(d2) a halogen atom;
(d3) a ($C_1$-$C_6$) alkyl group;
(d4) a ($C_3$-$C_6$) cycloalkyl group;
(d7) a ($C_1$-$C_6$) alkoxy group;
(d12) a halo ($C_1$-$C_6$) alkoxy group;
(d13) a cyano group;
(d14) an $R^9$ ($R^{10}$) N group (wherein $R^9$ and $R^{10}$ are as defined above) ;
(d15) a ($C_1$-$C_6$) alkylthio group;
(d16) a ($C_1$-$C_6$) alkylsulfinyl group;
(d17) a ($C_1$-$C_6$) alkylsulfonyl group;
(d18) a ($C_1$-$C_6$) alkylthio group;
(d19) a formyl group;
(d20) a ($C_1$-$C_6$) alkoxycarbonyl group;
(d21) an $R^9$ ($R^{10}$) N carbonyl group (wherein $R^9$ and $R^{10}$ are as defined above); or
(d22) an $R^{10}ON=C(R^9)$ group (wherein $R^9$ and $R^{10}$ are as defined above),

and more preferably

(d1) a hydrogen atom or
(d2) a halogen atom.

$G^6$ is preferably N-$R^5$ or O.
$R^5$ is preferably (e2) a ($C_1$-$C_6$) alkyl group.

The compounds of the present invention can be produced according to, for example, the production methods described below, which are non-limiting examples.

Production Method 1

[Chem. 3]

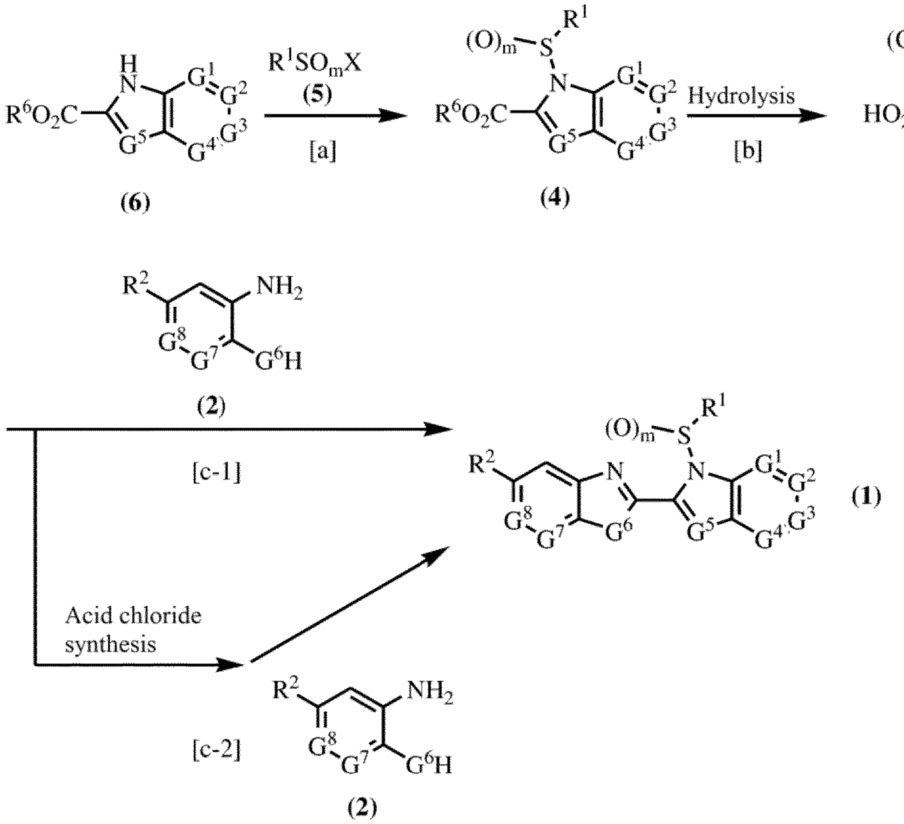


(In the formula, $R^1$, $R^2$, $R^6$, $G^1$, $G^2$, $G^3$, $G^4$, $G^5$, $G^6$, $G^7$, $G^8$ and m are as defined above, and X represents a halogen atom.)

The compound represented by the general formula (1) of the present invention can be produced through the steps [a], [b] and [c-1] or [c-2] described below.

Step [a]

A step of reacting the compound represented by the general formula (6) with the compound represented by the general formula (5), to produce the compound represented by the general formula (4) .

Step [b]

A step of hydrolyzing the compound represented by the general formula (4), to produce the compound represented by the general formula (3).

Step [c-1]

A step of reacting the compound represented by the general formula (3) with the compound represented by the general formula (2) in the presence of a condensing agent for amide condensation, to produce the compound represented by the general formula (1) .

Step [c-2]

A step of synthesizing an acid halide from the compound represented by the general formula (3) and reacting the acid halide with the compound represented by the general formula (2), to produce the compound represented by the general formula (1) .

Production Method at Step [a]

The compound represented by the general formula (4) can be produced by reacting the compound represented by the general formula (6) with the compound represented by the general formula (5) in the presence of a base and an inert solvent.

Examples of the base that can be used in this reaction include alkyllithiums such as sec-butyllithium and tert-butyllithium; organometallic compounds such as lithium hexamethyldisilazane and sodium hexamethyldisilazane; alkoxides such as sodium tert-butoxide and potassium tert-butoxide; and metal hydrides such as sodium hydride and potassium hydride. The amount of the base used is usually in the range of a 1- to 10-fold molar amount relative to the compound represented by the general formula (6).

The inert solvent used in this reaction may be any solvent that does not markedly inhibit the progress of the reaction, and examples include straight-chain or cyclic saturated hydrocarbons such as pentane, hexane and cyclohexane; aromatic hydrocarbons such as benzene, toluene and xylene; straight-chain or cyclic ethers such as diethyl ether, methyl tert-butyl ether, dioxane and tetrahydrofuran; and aprotic polar solvents such as N,N-dimethylformamide, N,N-dimethylacetamide, dimethyl sulfoxide and 1,3-dimethyl-2-imidazolidinone. One of these inert solvents may be used alone, and also two or more of them may be used as a mixture. The amount of the inert solvent used is usually selected as appropriate from the range of 0.1 to 100 L relative to 1 mol of the compound represented by the general formula (2) .

Since this reaction is an equimolar reaction of the compounds, they are basically used in equimolar amounts, but either of them may be used in an excess amount. The reaction temperature in this reaction is usually in the range of about -78°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

Production Method at Step [b]

The compound represented by the general formula (3) can be produced by hydrolyzing the compound represented by the general formula (4) in the presence of a base, an inert solvent and water.

Examples of the base that can be used in this reaction include hydroxides such as lithium hydroxide, sodium hydroxide, potassium hydroxide and calcium hydroxide; and alkoxides such as sodium methoxide, sodium ethoxide, sodium tert-butoxide and potassium tert-butoxide. The amount of the base used is usually in the range of a 1- to 10-fold molar amount relative to the compound represented by the general formula (4).

The inert solvent used in this reaction may be any solvent that does not markedly inhibit the reaction, and examples include alcohols such as methanol, ethanol, propanol, butanol and 2-propanol; straight-chain or cyclic ethers such as diethyl ether, tetrahydrofuran (THF) and dioxane; aprotic polar solvents such as N,N-dimethylformamide, N,N-dimethylacetamide, dimethyl sulfoxide and 1,3-dimethyl-2-imidazolidinone; and water. One of these inert solvents may be used alone, or two or more of them may be used as a mixture. The amount of the inert solvent used is usually selected as appropriate from the range of 0.1 to 100 L relative to 1 mol of the compound represented by the general formula (4).

The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

Production Method at Step [c-1]

The compound represented by the general formula (3) is reacted with the compound represented by the general formula (2) in the presence of a condensing agent, a base and an inert solvent. The resulting intermediate is optionally isolated, and is allowed to react under acid conditions, to yield the compound represented by the general formula (1).

Examples of the condensing agent used in this reaction include 1-ethyl-3-(3-dimethylaminopropyl)carbodiimide hydrochloride (EDC), diethyl phosphorocyanidate (DEPC), carbonyldiimidazole (CDI), 1,3-dicyclohexylcarbodiimide (DCC), chlorocarbonic esters and 2-chloro-1-methylpyridinium iodide. The amount of the condensing agent used is usually selected as appropriate from the range of a 1- to 2-fold molar amount relative to the compound represented by the general formula (3).

Examples of the base that can be used in this reaction include carbonates such as lithium carbonate, lithium hydrogen carbonate, sodium carbonate, sodium hydrogen carbonate, potassium carbonate, potassium hydrogen carbonate, calcium carbonate and magnesium carbonate; acetates such as lithium acetate, sodium acetate and potassium acetate; and organic bases such as pyridine, picoline, lutidine, triethylamine, tributylamine and diisopropylethylamine. The amount of the base used is usually selected as appropriate from the range of a 1- to 10-fold molar amount relative to the compound represented by the general formula (3).

The inert solvent used in this reaction may be any solvent that does not markedly inhibit the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; straight-chain or cyclic ethers such as diethyl ether, tetrahydrofuran (THF) and dioxane; nitriles such as acetonitrile and propionitrile; esters such as methyl acetate; and aprotic polar solvents such as N,N-dimethylformamide, N,N-dimethylacetamide, dimethyl sulfoxide and 1,3-dimethyl-2-imidazolidinone. One of these inert solvents may be used alone, or two or more of them may be used as a mixture . The amount of the inert solvent used is usually selected as appropriate from the range of 0.1 to 100 L relative to 1 mol of the compound represented by the general formula (3).

Since this reaction is an equimolar reaction of the compounds, they are basically used in equimolar amounts, but either of them may be used in an excess amount. The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours.

After the reaction is completed, the resulting intermediate is optionally isolated from the post-reaction mixture, and is allowed to react in the presence of an acid and an inert solvent, to yield the compound represented by the general formula (1).

Examples of the acid used in this reaction include inorganic acids such as hydrochloric acid, sulfuric acid and nitric acid; organic acids such as formic acid, acetic acid, propionic acid, trifluoroacetic acid and benzoic acid; sulfonic acids such as methanesulfonic acid and trifluoromethanesulfonic acid; and phosphoric acid. The amount of the acid used is usually selected as appropriate from the range of a 0.01- to 10-fold molar amount relative to the compound represented by the general formula (3).

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; straight-chain or cyclic ethers such as diethyl ether, methyl tert-butyl ether, dioxane and tetrahydrofuran; esters such as ethyl acetate; amides such as dimethylformamide and dimethylacetamide; ketones such as acetone and methyl ethyl ketone; and aprotic polar solvents such as dimethyl sulfoxide and 1,3-dimethyl-2-imidazolidinone. One of these inert solvents may be used alone, or two or more of them may be used as a mixture . The amount of the inert solvent used is usually selected as appropriate from the range of 0.1 to 100 L relative to 1 mol of the compound represented by the general formula (3).

The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

Production Method at Step [c-2]

The compound represented by the general formula (3) is reacted with a halogenating agent in the presence of an inert solvent to give an acid halide. The acid halide is reacted with the compound represented by the general formula (2) in the presence of an inert solvent and a base to give an intermediate. The obtained intermediate is optionally isolated,

and is allowed to react under acid conditions, to yield the compound represented by the general formula (1).

Examples of the halogenating agent used in this reaction include phosphorus pentachloride, thionyl chloride, phosphorus oxychloride and oxalyl chloride. The amount of the halogenating agent used is usually selected as appropriate from the range of a 1- to 1.5-fold molar amount relative to the compound represented by the general formula (3).

Examples of the base that can be used in this reaction include carbonates such as lithium carbonate, lithium hydrogen carbonate, sodium carbonate, sodium hydrogen carbonate, potassium carbonate, potassium hydrogen carbonate, calcium carbonate and magnesium carbonate; acetates such as lithium acetate, sodium acetate and potassium acetate; and organic bases such as pyridine, picoline, lutidine, triethylamine, tributylamine and diisopropylethylamine. The amount of the base used is usually selected as appropriate from the range of a 1- to 10-fold molar amount relative to the compound represented by the general formula (3).

The inert solvent used in this reaction may be any solvent that does not markedly inhibit the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; straight-chain or cyclic ethers such as diethyl ether, tetrahydrofuran (THF) and dioxane; nitriles such as acetonitrile and propionitrile; esters such as methyl acetate; and polar solvents such as N,N-dimethylformamide, N,N-dimethylacetamide, dimethyl sulfoxide and 1,3-dimethyl-2-imidazolidinone. One of these inert solvents may be used alone, or two or more of them may be used as a mixture . The amount of the inert solvent used is usually selected as appropriate from the range of 0.1 to 100 L relative to 1 mol of the compound represented by the general formula (3).

Since this reaction is an equimolar reaction of the compounds, they are basically used in equimolar amounts, but either of them may be used in an excess amount. The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours.

After the reaction is completed, the resulting intermediate is optionally isolated from the post-reaction mixture, and is allowed to react in the presence of an acid and an inert solvent, to yield the compound of interest.

Examples of the acid used in this reaction include inorganic acids such as hydrochloric acid, sulfuric acid and nitric acid; organic acids such as formic acid, acetic acid, propionic acid, trifluoroacetic acid and benzoic acid; sulfonic acids such as methanesulfonic acid and trifluoromethanesulfonic acid; and phosphoric acid. The amount of the acid used is usually selected as appropriate from the range of a 0.01- to 10-fold molar amount relative to the compound represented by the general formula (3).

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; straight-chain or cyclic ethers such as diethyl ether, methyl tert-butyl ether, dioxane and tetrahydrofuran; esters such as ethyl acetate; amides such as dimethylformamide and dimethylacetamide; ketones such as acetone and methyl ethyl ketone; and aprotic polar solvents such as dimethyl sulfoxide and 1,3-dimethyl-2-imidazolidinone. One of these inert solvents may be used alone, or two or more of them may be used as a mixture . The amount of the inert solvent used is usually selected as appropriate from the range of 0.1 to 100 L relative to 1 mol of the compound represented by the general formula (3).

The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

Production Method 2

[Chem. 4]

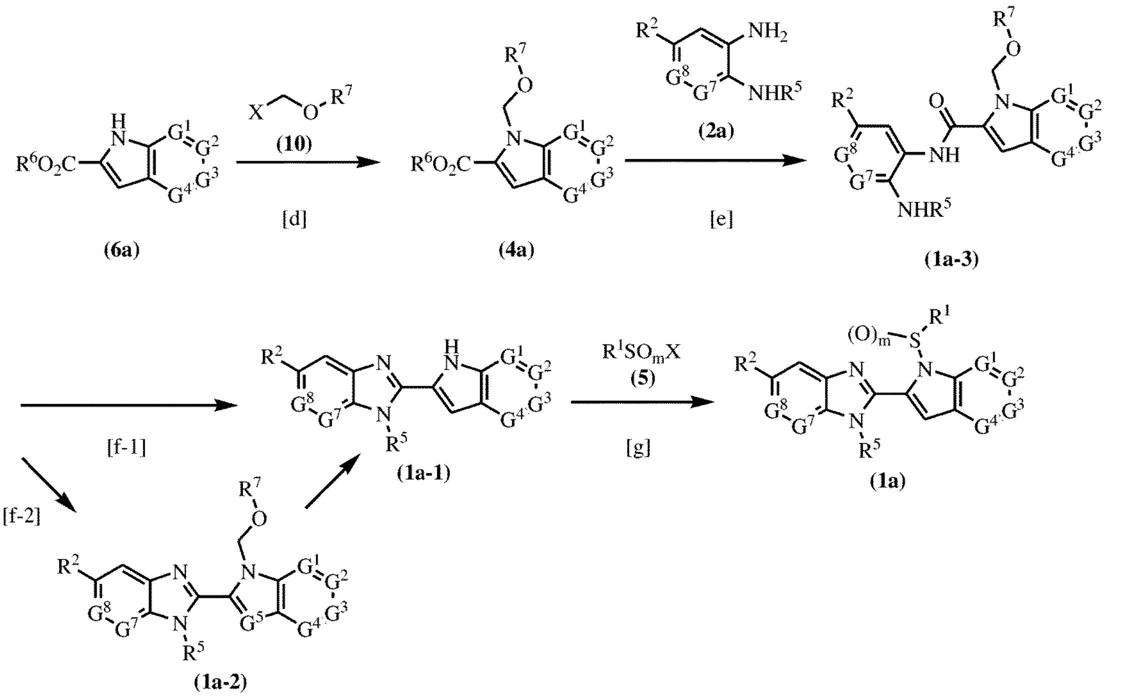


(In the formula, $R^1$, $R^2$, $R^5$, $R^6$, $G^1$, $G^2$, $G^3$, $G^4$, $G^7$, $G^8$, m and X are as defined above, and $R^7$ represents a ($C_1$-$C_6$) alkyl group or a phenyl ($C_1$-$C_6$) alkyl group.)

The compound represented by the general formula (1a) of the present invention can be produced through the steps [d], [e], [g] and [f-1] or [f-2] described below.

Step [d]

A step of reacting the compound represented by the general formula (6a) with the compound represented by the general formula (10), to produce the compound represented by the general formula (4a).

Step [e]

A step of reacting the compound represented by the general formula (4a) with the compound represented by the general formula (2a), to produce the compound represented by the general formula (1a-3).

Step [f-1]

A step of subjecting the compound represented by the general formula (la-3) to cyclization and concomitant deprotection of the protective group on the nitrogen atom, to produce the compound represented by the general formula (1a-1).

Step [f-2]

A step of subjecting the compound represented by the general formula (1a-3) to cyclization to produce the compound represented by the general formula (1a-2), followed by deprotection of the protective group on the nitrogen atom, to produce the compound represented by the general formula (la-1) .

Step [g]

A step of reacting the compound represented by the general formula (1a-1) with the compound represented by the general formula (5), to produce the compound represented by the general formula (1a).

Production Method at Step [d]

The compound represented by the general formula (4a) can be produced by reacting the compound represented by the general formula (6a) with the compound represented by the general formula (10) in the presence of a base and an inert solvent.
Examples of the base that can be used in this reaction include alkyllithiums such as methyllithium, n-butyllithium, sec-butyllithium and tert-butyllithium; organometallic compounds such as lithium hexamethyldisilazane and sodium hexamethyldisilazane; hydroxides such as lithium hydroxide, sodium hydroxide, potassium hydroxide and calcium hydroxide; carbonates such as lithium carbonate, lithium hydrogen carbonate, sodium carbonate, sodium hydrogen carbonate, potassium carbonate, potassium hydrogen carbonate, calcium carbonate and magnesium carbonate; acetates such as lithium acetate, sodium acetate and potassium acetate; alkoxides such as sodium methoxide, sodium ethoxide, sodium tert-butoxide and potassium tert-butoxide; and metal hydrides such as sodium hydride and potassium hydride. The amount of the base used is usually selected as appropriate from the range of a 1- to 5-fold molar amount relative to the compound represented by the general formula (6a).
The inert solvent used in this reaction may be any solvent that does not markedly inhibit the progress of the reaction, and examples include straight-chain or cyclic saturated hydrocarbons such as pentane, hexane and cyclohexane; aromatic hydrocarbons such as benzene, toluene and xylene; straight-chain or cyclic ethers such as diethyl ether, methyl tert-butyl ether, dioxane and tetrahydrofuran; and aprotic polar solvents such as N,N-dimethylformamide, N,N-dimethylacetamide, dimethyl sulfoxide and 1,3-dimethyl-2-imidazolidinone. One of these inert solvents may be used alone, or two or more of them may be used as a mixture . The amount of the inert solvent used is usually selected as appropriate from the range of 0.1 to 100 L relative to 1 mol of the compound represented by the general formula (6a).
Since this reaction is an equimolar reaction of the compounds, they are basically used in equimolar amounts, but either of them may be used in an excess amount. The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

Production Method at Step [e]

The compound represented by the general formula (1a-3) can be produced by reacting the compound represented by the general formula (4a) with the compound represented by the general formula (2a) in the presence of a base and an inert solvent.
Examples of the base that can be used in this reaction include alkyllithiums such as sec-butyllithium and tert-butyllithium; organometallic compounds such as lithium hexamethyldisilazane and sodium hexamethyldisilazane; alkoxides such as sodium tert-butoxide and potassium tert-butoxide; and metal hydrides such as sodium hydride and potassium hydride. The amount of the base used is usually selected as appropriate from the range of a 1- to 5-fold molar amount relative to the compound represented by the general formula (4a).
The inert solvent used in this reaction may be any solvent that does not markedly inhibit the progress of the reaction, and examples include straight-chain or cyclic saturated hydrocarbons such as pentane, hexane and cyclohexane; aromatic hydrocarbons such as benzene, toluene and xylene; straight-chain or cyclic ethers such as diethyl ether, methyl tert-butyl ether, dioxane and tetrahydrofuran; and aprotic polar solvents such as N,N-dimethylformamide, N,N-dimethylacetamide, dimethyl sulfoxide and 1,3-dimethyl-2-imidazolidinone. One of these inert solvents may be used alone, or two or more of them may be used as a mixture . The amount of the inert solvent used is usually selected as appropriate from the range of 0.1 to 100 L relative to 1 mol of the compound represented by the general formula (4a).
Since this reaction is an equimolar reaction of the compounds, they are basically used in equimolar amounts, but either of them may be used in an excess amount. The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

Production Method at Step [f-1]

The compound represented by the general formula (1a-1) can be produced by allowing the compound represented by the general formula (1a-3) to react in the presence of an acid and an inert solvent.

Examples of the acid that can be used in this reaction include inorganic acids such as hydrochloric acid, sulfuric acid and nitric acid; and organic acids such as methanesulfonic acid, p-toluenesulfonic acid, trifluoroacetic acid and trifluoromethanesulfonic acid. The amount of the acid used is usually selected as appropriate from the range of a 0.01- to 10-fold molar amount relative to the compound represented by the general formula (1a-3).

The inert solvent used in this reaction may be any solvent that does not markedly inhibit the progress of the reaction, and examples include alcohols such as methanol, ethanol, propanol, butanol and 2-propanol; straight-chain or cyclic saturated hydrocarbons such as pentane, hexane and cyclohexane; aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; straight-chain or cyclic ethers such as diethyl ether, methyl tert-butyl ether, dioxane, tetrahydrofuran and cyclopentyl methyl ether; esters such as ethyl acetate; nitriles such as acetonitrile and propionitrile; amides such as dimethylformamide and dimethylacetamide; ketones such as acetone and methyl ethyl ketone; aprotic polar solvents such as dimethyl sulfoxide and 1,3-dimethyl-2-imidazolidinone; and water. One of these inert solvents may be used alone, or two or more of them may be used as a mixture. The amount of the inert solvent used is usually selected as appropriate from the range of 0.1 to 100 L relative to 1 mol of the compound represented by the general formula (1a-3).

The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

Production Method at Step [f-2]

The compound represented by the general formula (1a-3) is allowed to react in the presence of an acid and an inert solvent to give the compound represented by the general formula (1a-2) . The compound represented by the general formula (1a-2) is allowed to react in the presence of an acid and an inert solvent, to yield the compound represented by the general formula (la-1) .

Examples of the acid that can be used in this reaction include inorganic acids such as hydrochloric acid, sulfuric acid, nitric acid and phosphoric acid; and organic acids such as acetic acid, methanesulfonic acid, p-toluenesulfonic acid, trifluoroacetic acid and trifluoromethanesulfonic acid. The amount of the acid used is usually selected as appropriate from the range of a 0.01- to 10-fold molar amount relative to the compound represented by the general formula (1a-3).

The inert solvent used in this reaction may be any solvent that does not markedly inhibit the progress of the reaction, and examples include alcohols such as methanol, ethanol, propanol, butanol and 2-propanol; straight-chain or cyclic saturated hydrocarbons such as pentane, hexane and cyclohexane; aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; straight-chain or cyclic ethers such as diethyl ether, methyl tert-butyl ether, dioxane, tetrahydrofuran and cyclopentyl methyl ether; esters such as ethyl acetate; nitriles such as acetonitrile and propionitrile; amides such as dimethylformamide and dimethylacetamide; ketones such as acetone and methyl ethyl ketone; aprotic polar solvents such as dimethyl sulfoxide and 1,3-dimethyl-2-imidazolidinone; and water. One of these inert solvents may be used alone, or two or more of them may be used as a mixture. The amount of the inert solvent used is usually selected as appropriate from the range of 0.1 to 100 L relative to 1 mol of the compound represented by the general formula (1a-3).

The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

Production Method of General Formula (1a) from General Formula (1a-1)

The compound represented by the general formula (1a) can be produced by subjecting the compound represented by the general formula (1a-1) to the reaction according to the production method at step [a] described in production method 1.

Production Method 3

[Chem. 5]

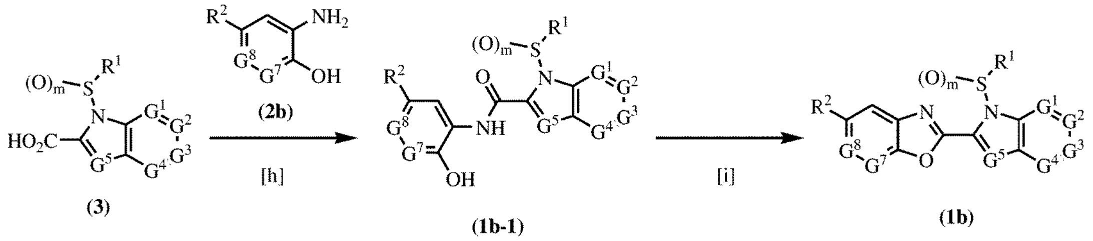


(3) (2b) (1b-1) (1b)

(In the formula, $R^1$, $R^2$, $G^1$, $G^2$, $G^3$, $G^4$, $G^5$, $G^7$, $G^8$ and m are as defined above.)

The compound represented by the general formula (1b) of the present invention can be produced through the steps [h] and [i] described below.

Step [h]

A step of reacting the compound represented by the general formula (3) with the compound represented by the general formula (2b), to produce the compound represented by the general formula (1b-1) .

Step [i]

A step of cyclizing the compound represented by the general formula (1b-1), to produce the compound represented by the general formula (1b).

Production Method at Step [h]

The compound represented by the general formula (1b-1) can be produced by reacting the compound represented by the general formula (3) with the compound represented by the general formula (2b) in the presence of a condensing agent, a base and an inert solvent.

Examples of the condensing agent used in this reaction include, but are not limited to, diethyl phosphorocyanidate (DEPC), carbonyldiimidazole (CDI), 1,3-dicyclohexylcarbodiimide (DCC), 1-ethyl-3-(3-dimethylaminopropyl)-carbodiimide (EDC), chlorocarbonic esters and 2-chloro-1-methylpyridinium iodide. The amount of the condensing agent used is usually selected as appropriate from the range of a 1- to 2-fold molar amount relative to the compound represented by the general formula (3) .

Examples of the base that can be used in this reaction include carbonates such as lithium carbonate, lithium hydrogen carbonate, sodium carbonate, sodium hydrogen carbonate, potassium carbonate, potassium hydrogen carbonate, calcium carbonate and magnesium carbonate; acetates such as lithium acetate, sodium acetate and potassium acetate; and organic bases such as pyridine, picoline, lutidine, triethylamine, tributylamine and diisopropylethylamine. The amount of the base used is usually selected as appropriate from the range of a 1- to 10-fold molar amount relative to the compound represented by the general formula (3).

The inert solvent used in this reaction may be any solvent that does not markedly inhibit the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; straight-chain or cyclic ethers such as diethyl ether, tetrahydrofuran (THF) and dioxane; nitriles such as acetonitrile and propionitrile; esters such as methyl acetate; and aprotic polar solvents such as N,N-dimethylformamide, N,N-dimethylacetamide, dimethyl sulfoxide and 1,3-dimethyl-2-imidazolidinone. One of these inert solvents may be used alone, or two or more of them may be used as a mixture . The amount of the inert solvent used is usually selected as appropriate from the range of 0.1 to 100 L relative to 1 mol of the compound represented by the general formula (3).

Since this reaction is an equimolar reaction of the compounds, they are basically used in equimolar amounts, but either of them may be used in an excess amount. The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

Production Method of General Formula (1b) from General Formula (1b-1)

The compound represented by the general formula (1b) can be produced by allowing the compound represented by the general formula (lb-1) to react in the presence of an azodicarboxylic acid ester, triphenylphosphine and an inert solvent.

Examples of the azodicarboxylic acid ester that can be used in this reaction include, but are not limited to, diethyl azodicarboxylate (DEAD), diisopropyl azodicarboxylate and bis(2-methoxyethyl) azodicarboxylate. The amount of the azodicarboxylic acid ester used is usually selected as appropriate from the range of a 1- to 5-fold molar amount relative to the compound represented by the general formula (3) .

The inert solvent used in this reaction may be any solvent that does not markedly inhibit the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; straight-chain or cyclic ethers such as diethyl ether, tetrahydrofuran (THF) and dioxane; nitriles such as acetonitrile and propionitrile; esters such as methyl acetate; and polar solvents such as N,N-dimethylformamide, N,N-dimethylacetamide, dimethyl sulfoxide and 1,3-dimethyl-2-imidazolidinone. One of these inert solvents may be used alone, or two or more of them may be used as a mixture . The amount of the inert solvent used is usually selected as appropriate from the range of 0.1 to 100 L relative to 1 mol of the compound represented by the general formula (1b-1).

The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

Production Method (1) of Intermediate

[Chem. 6]

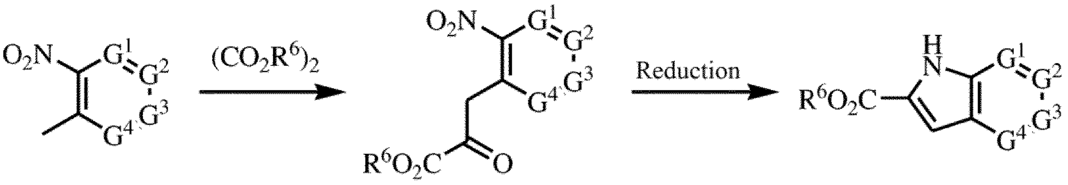


(7) (6)

(In the formula, $G^1$, $G^2$, $G^3$ and $G^4$ are as defined above, and $R^6$ represents a $(C_1\text{-}C_6)$ alkyl group.)

Production Method of General Formula (6) from General Formula (7)

The compound represented by the general formula (6) can be produced by subjecting the compound represented by the general formula (7) to the reaction according to the method described in the literature (J. O. C., 2001, 66, 3474.).

Production Method (2) of Intermediate

[Chem. 7]

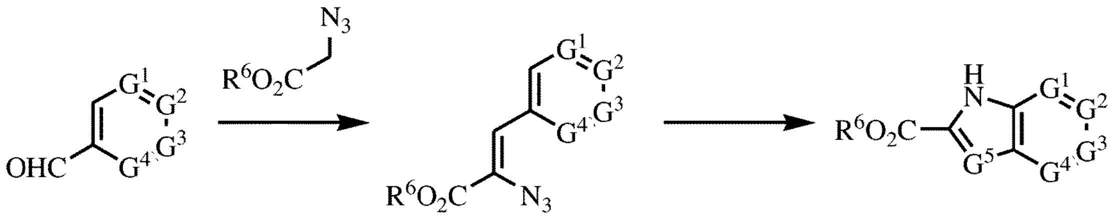

**(8)** **(6)**

(In the formula, $G^1$, $G^2$, $G^3$, $G^4$ and $R^6$ are as defined above.) Production Method of General Formula (6) from General Formula (8)

The compound represented by the general formula (6) can be produced by subjecting the compound represented by the general formula (8) to the reaction according to the method described in the literature (J. Med. Chem., 2004, 47, 6270.).

Production Method (3) of Intermediate

[Chem. 8]

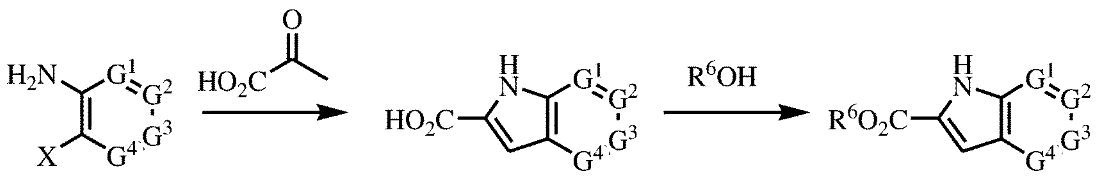

**(9)** **(6)**

Production Method of General Formula (6) from General Formula (9)

The compound represented by the general formula (6) can be produced by subjecting the compound represented by the general formula (9) to the reaction according to the method described in the literature (WO 2014/073627).

Representative examples of the compounds represented by the general formula (1) of the present invention are shown in Tables 1 to 10 below, but the present invention is not limited thereto.

In the tables below, "Me" represents a methyl group, "Et" represents an ethyl group, "Pr" represents a propyl group, "Ac" represents an acetyl group, "Ph" represents a phenyl group, and "c-" represents cyclo. Shown in the column of "Physical property" is a melting point (°C), a refractive index $n_D$ (the figure in parentheses is the measurement temperature (°C)) or "NMR". NMR data are shown in Table 11.

[Chem. 9]

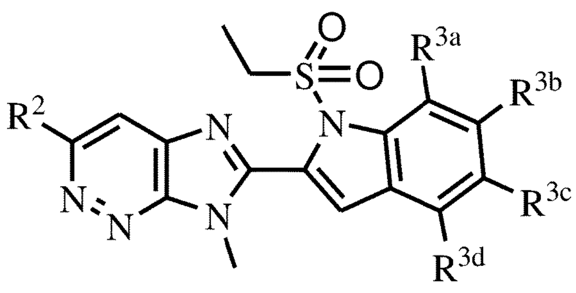

**(1-a)**

[Table 1]

Table 1

| Compound No. | $R^{3a}$ | $R^{3b}$ | $R^{3c}$ | $R^{3d}$ | $R^2$ | Physical property |
|---|---|---|---|---|---|---|
| 1-1 | H | H | H | H | $CF_3$ | 158-159 |
| 1-2 | Cl | H | H | H | $CF_3$ | |
| 1-3 | H | Cl | H | H | $CF_3$ | |
| 1-4 | H | H | Cl | H | $CF_3$ | |
| 1-5 | H | H | H | Cl | $CF_3$ | |
| 1-6 | Me | H | H | H | $CF_3$ | |
| 1-7 | H | Me | H | H | $CF_3$ | |
| 1-8 | H | H | Me | H | $CF_3$ | |
| 1-9 | H | H | H | Me | $CF_3$ | |
| 1-10 | H | $CF_3$ | H | H | $CF_3$ | |
| 1-11 | H | H | $CF_3$ | H | $CF_3$ | |
| 1-12 | H | CN | H | H | $CF_3$ | |
| 1-13 | H | H | CN | H | $CF_3$ | |
| 1-14 | H | OMe | H | H | $CF_3$ | |
| 1-15 | H | H | OMe | H | $CF_3$ | |
| 1-16 | H | $NH_2$ | H | H | $CF_3$ | |
| 1-17 | H | H | $NH_2$ | H | $CF_3$ | |
| 1-18 | H | $NMe_2$ | H | H | $CF_3$ | |
| 1-19 | H | H | $NMe_2$ | H | $CF_3$ | |
| 1-20 | H | NHAc | H | H | $CF_3$ | |
| 1-21 | H | H | NHAc | H | $CF_3$ | |
| 1-22 | H | SMe | H | H | $CF_3$ | |
| 1-23 | H | H | SMe | H | $CF_3$ | |
| 1-24 | H | $SCF_3$ | H | H | $CF_3$ | |
| 1-25 | H | H | $SCF_3$ | H | $CF_3$ | |

[Table 2]

Table 1 (Continued)

| Compound No. | $R^{3a}$ | $R^{3b}$ | $R^{3c}$ | $R^{3d}$ | $R^2$ | Physical property |
|---|---|---|---|---|---|---|
| 1-26 | H | CHO | H | H | $CF_3$ | |
| 1-27 | H | $CO_2Et$ | H | H | $CF_3$ | |
| 1-28 | H | CONHMe | H | H | $CF_3$ | |
| 1-29 | H | CH=NOEt | H | H | $CF_3$ | |
| 1-30 | H | c-Pr | H | H | $CF_3$ | |

(continued)

| Compound No. | $R^{3a}$ | $R^{3b}$ | $R^{3c}$ | $R^{3d}$ | $R^2$ | Physical property |
|---|---|---|---|---|---|---|
| 1-31 | H | Br | H | H | $CF_3$ | |
| 1-32 | H | I | H | H | $CF_3$ | |
| 1-33 | H | H | H | H | $CF_2CF_3$ | 145-146 |
| 1-34 | Cl | H | H | H | $CF_2CF_3$ | |
| 1-35 | H | Cl | H | H | $CF_2CF_3$ | 92-94 |
| 1-36 | H | H | Cl | H | $CF_2CF_3$ | NMR |
| 1-37 | H | H | H | Cl | $CF_2CF_3$ | |
| 1-38 | Me | H | H | H | $CF_2CF_3$ | |
| 1-39 | H | Me | H | H | $CF_2CF_3$ | |
| 1-40 | H | H | Me | H | $CF_2CF_3$ | |
| 1-41 | H | H | H | Me | $CF_2CF_3$ | |
| 1-42 | H | $CF_3$ | H | H | $CF_2CF_3$ | |
| 1-43 | H | H | $CF_3$ | H | $CF_2CF_3$ | 201-202 |
| 1-44 | H | CN | H | H | $CF_2CF_3$ | |
| 1-45 | H | H | CN | H | $CF_2CF_3$ | |
| 1-46 | H | OMe | H | H | $CF_2CF_3$ | |
| 1-47 | H | H | OMe | H | $CF_2CF_3$ | |
| 1-48 | H | $NH_2$ | H | H | $CF_2CF_3$ | |
| 1-49 | H | H | $NH_2$ | H | $CF_2CF_3$ | |
| 1-50 | H | $NMe_2$ | H | H | $CF_2CF_3$ | |

[Table 3]

Table 1 (Continued)

| Compound No. | $R^{3a}$ | $R^{3b}$ | $R^{3c}$ | $R^{3d}$ | $R^2$ | Physical property |
|---|---|---|---|---|---|---|
| 1-51 | H | H | $NMe_2$ | H | $CF_2CF_3$ | |
| 1-52 | H | NHAc | H | H | $CF_2CF_3$ | |
| 1-53 | H | H | NHAc | H | $CF_2CF_3$ | |
| 1-54 | H | SMe | H | H | $CF_2CF_3$ | |
| 1-55 | H | H | SMe | H | $CF_2CF_3$ | |
| 1-56 | H | $SCF_3$ | H | H | $CF_2CF_3$ | |
| 1-57 | H | H | $SCF_3$ | H | $CF_2CF_3$ | |
| 1-58 | H | CHO | H | H | $CF_2CF_3$ | |
| 1-59 | H | $CO_2Et$ | H | H | $CF_2CF_3$ | |
| 1-60 | H | CONHMe | H | H | $CF_2CF_3$ | |
| 1-61 | H | CH=NOEt | H | H | $CF_2CF_3$ | |
| 1-62 | H | c-Pr | H | H | $CF_2CF_3$ | |

(continued)

| Compound No. | $R^{3a}$ | $R^{3b}$ | $R^{3c}$ | $R^{3d}$ | $R^2$ | Physical property |
|---|---|---|---|---|---|---|
| 1-63 | H | Br | H | H | $CF_2CF_3$ | 106-110 |
| 1-64 | H | I | H | H | $CF_2CF_3$ | |
| 1-65 | H | F | H | H | $CF_2CF_3$ | 82-86 |
| 1-66 | H | Cl | Br | H | $CF_2CF_3$ | 261-263 |

[Chem. 10]

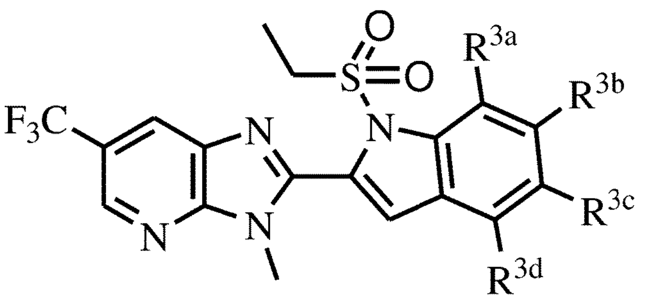

(1-b)

[Table 4]

Table 2

| Compound No. | $R^{3a}$ | $R^{3b}$ | $R^{3c}$ | $R^{3d}$ | Physical property |
|---|---|---|---|---|---|
| 2-1 | H | H | H | H | 131-132 |
| 2-2 | Cl | H | H | H | |
| 2-3 | H | Cl | H | H | 170-172 |
| 2-4 | H | H | Cl | H | |
| 2-5 | H | H | H | Cl | 154-156 |
| 2-6 | Me | H | H | H | |
| 2-7 | H | Me | H | H | 104-107 |
| 2-8 | H | H | Me | H | |
| 2-9 | H | H | H | Me | |
| 2-10 | H | $CF_3$ | H | H | 124-128 |
| 2-11 | H | H | $CF_3$ | H | 134-135 |
| 2-12 | H | CN | H | H | |
| 2-13 | H | H | CN | H | |
| 2-14 | H | OMe | H | H | 171-173 |
| 2-15 | H | H | OMe | H | 208-212 |
| 2-16 | H | $NH_2$ | H | H | |
| 2-17 | H | H | $NH_2$ | H | |
| 2-18 | H | $NMe_2$ | H | H | |
| 2-19 | H | H | $NMe_2$ | H | |

(continued)

| Compound No. | $R^{3a}$ | $R^{3b}$ | $R^{3c}$ | $R^{3d}$ | Physical property |
|---|---|---|---|---|---|
| 2-20 | H | NHAc | H | H | |
| 2-21 | H | H | NHAc | H | |
| 2-22 | H | SMe | H | H | |
| 2-23 | H | H | SMe | H | |
| 2-24 | H | $SCF_3$ | H | H | |
| 2-25 | H | H | $SCF_3$ | H | |

[Table 5]

Table 2 (Continued)

| Compound No. | $R^{3a}$ | $R^{3b}$ | $R^{3c}$ | $R^{3d}$ | Physical property |
|---|---|---|---|---|---|
| 2-26 | H | CHO | H | H | |
| 2-27 | H | $CO_2Et$ | H | H | |
| 2-28 | H | CONHMe | H | H | |
| 2-29 | H | CH=NOEt | H | H | |
| 2-30 | H | c-Pr | H | H | 154-156 |
| 2-31 | H | Br | H | H | 175-177 |
| 2-32 | H | I | H | H | |
| 2-33 | H | H | H | $CF_3$ | |
| 2-34 | H | H | H | CN | |
| 2-35 | OMe | H | H | H | |
| 2-36 | H | H | H | OMe | |
| 2-37 | H | $OCH_2CF_3$ | H | H | |
| 2-38 | H | H | $OCH_2CF_3$ | H | |
| 2-39 | H | H | H | $OCH_2CF_3$ | |
| 2-40 | H | H | H | $NH_2$ | |
| 2-41 | H | H | H | $NMe_2$ | |
| 2-42 | H | H | H | NHAc | |
| 2-43 | H | H | H | SMe | |
| 2-44 | H | SOMe | H | H | |
| 2-45 | H | H | SOMe | H | |
| 2-46 | H | H | H | SOMe | |
| 2-47 | H | $SO_2Me$ | H | H | |
| 2-48 | H | H | $SO_2Me$ | H | |
| 2-49 | H | H | H | $SO_2Me$ | |
| 2-50 | H | H | H | $SCF_3$ | |

[Table 6]

Table 2 (Continued)

| Compound No. | $R^{3a}$ | $R^{3b}$ | $R^{3c}$ | $R^{3d}$ | Physical property |
|---|---|---|---|---|---|
| 2-51 | H | H | CHO | H | |
| 2-52 | H | H | H | CHO | |
| 2-53 | H | H | $CO_2Et$ | H | |
| 2-54 | H | H | H | $CO_2Et$ | |
| 2-55 | H | H | CONHMe | H | |
| 2-56 | H | H | H | CONHMe | |
| 2-57 | H | H | C=NOEt | H | |
| 2-58 | H | H | H | C=NOEt | |
| 2-59 | H | $C=NOCH_2CF_3$ | H | H | |
| 2-60 | H | H | $C=NOCH_2CF_3$ | H | |
| 2-61 | H | H | H | $C=NOCH_2CF_3$ | |
| 2-62 | H | H | c-Pr | H | 155-157 |
| 2-63 | H | H | H | c-Pr | |
| 2-64 | H | H | Br | H | 101-104 |
| 2-65 | H | H | $OCF_3$ | H | 113-115 |
| 2-66 | H | OEt | H | H | 127-128 |
| 2-67 | H | Cl | I | H | 204-205 |

[Chem. 11]

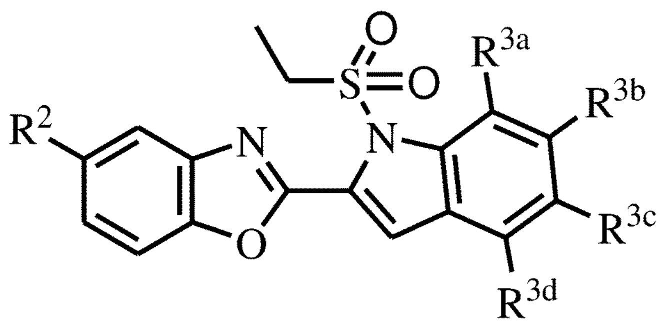

**(1-c)**

[Table 7]

Table 3

| Compound No. | $R^{3a}$ | $R^{3b}$ | $R^{3c}$ | $R^{3d}$ | $R^2$ | Physical property |
|---|---|---|---|---|---|---|
| 3-1 | H | H | H | H | $SCF_3$ | 118-119 |
| 3-2 | Cl | H | H | H | $SCF_3$ | |

(continued)

| Compound No. | $R^{3a}$ | $R^{3b}$ | $R^{3c}$ | $R^{3d}$ | $R^2$ | Physical property |
|---|---|---|---|---|---|---|
| 3-3 | H | Cl | H | H | $SCF_3$ | |
| 3-4 | H | H | Cl | H | $SCF_3$ | 126-130 |
| 3-5 | H | H | H | Cl | $SCF_3$ | |
| 3-6 | Me | H | H | H | $SCF_3$ | |
| 3-7 | H | Me | H | H | $SCF_3$ | |
| 3-8 | H | H | Me | H | $SCF_3$ | |
| 3-9 | H | H | H | Me | $SCF_3$ | |
| 3-10 | H | $CF_3$ | H | H | $SCF_3$ | |
| 3-11 | H | H | $CF_3$ | H | $SCF_3$ | 155-157 |
| 3-12 | H | CN | H | H | $SCF_3$ | |
| 3-13 | H | H | CN | H | $SCF_3$ | |
| 3-14 | H | OMe | H | H | $SCF_3$ | |
| 3-15 | H | H | OMe | H | $SCF_3$ | |
| 3-16 | H | $NH_2$ | H | H | $SCF_3$ | |
| 3-17 | H | H | $NH_2$ | H | $SCF_3$ | |
| 3-18 | H | $NMe_2$ | H | H | $SCF_3$ | |
| 3-19 | H | H | $NMe_2$ | H | $SCF_3$ | |
| 3-20 | H | NHAc | H | H | $SCF_3$ | |
| 3-21 | H | H | NHAc | H | $SCF_3$ | |
| 3-22 | H | SMe | H | H | $SCF_3$ | |
| 3-23 | H | H | SMe | H | $SCF_3$ | |
| 3-24 | H | $SCF_3$ | H | H | $SCF_3$ | |
| 3-25 | H | H | $SCF_3$ | H | $SCF_3$ | |

[Table 8]

Table 3 (Continued)

| Compound No. | $R^{3a}$ | $R^{3b}$ | $R^{3c}$ | $R^{3d}$ | $R^2$ | Physical property |
|---|---|---|---|---|---|---|
| 3-26 | H | CHO | H | H | $SCF_3$ | |
| 3-27 | H | $CO_2Et$ | H | H | $SCF_3$ | |
| 3-28 | H | CONHMe | H | H | $SCF_3$ | |
| 3-29 | H | CH=NOEt | H | H | $SCF_3$ | |
| 3-30 | H | c-Pr | H | H | $SCF_3$ | |
| 3-31 | H | Br | H | H | $SCF_3$ | |
| 3-32 | H | I | H | H | $SCF_3$ | |
| 3-33 | H | H | H | H | $SO_2CF_3$ | |
| 3-34 | Cl | H | H | H | $SO_2CF_3$ | |

(continued)

| Compound No. | $R^{3a}$ | $R^{3b}$ | $R^{3c}$ | $R^{3d}$ | $R^2$ | Physical property |
|---|---|---|---|---|---|---|
| 3-35 | H | Cl | H | H | $SO_2CF_3$ | |
| 3-36 | H | H | Cl | H | $SO_2CF_3$ | |
| 3-37 | H | H | H | Cl | $SO_2CF_3$ | |
| 3-38 | Me | H | H | H | $SO_2CF_3$ | |
| 3-39 | H | Me | H | H | $SO_2CF_3$ | |
| 3-40 | H | H | Me | H | $SO_2CF_3$ | |
| 3-41 | H | H | H | Me | $SO_2CF_3$ | |
| 3-42 | H | $CF_3$ | H | H | $SO_2CF_3$ | |
| 3-43 | H | H | $CF_3$ | H | $SO_2CF_3$ | |
| 3-44 | H | CN | H | H | $SO_2CF_3$ | |
| 3-45 | H | H | CN | H | $SO_2CF_3$ | |
| 3-46 | H | OMe | H | H | $SO_2CF_3$ | |
| 3-47 | H | H | OMe | H | $SO_2CF_3$ | |
| 3-48 | H | $NH_2$ | H | H | $SO_2CF_3$ | |
| 3-49 | H | H | $NH_2$ | H | $SO_2CF_3$ | |
| 3-50 | H | $NMe_2$ | H | H | $SO_2CF_3$ | |

[Table 9]

Table 3 (Continued)

| Compound No. | $R^{3a}$ | $R^{3b}$ | $R^{3c}$ | $R^{3d}$ | $R^2$ | Physical property |
|---|---|---|---|---|---|---|
| 3-51 | H | H | $NMe_2$ | H | $SO_2CF_3$ | |
| 3-52 | H | NHAc | H | H | $SO_2CF_3$ | |
| 3-53 | H | H | NHAc | H | $SO_2CF_3$ | |
| 3-54 | H | SMe | H | H | $SO_2CF_3$ | |
| 3-55 | H | H | SMe | H | $SO_2CF_3$ | |
| 3-56 | H | $SCF_3$ | H | H | $SO_2CF_3$ | |
| 3-57 | H | H | $SCF_3$ | H | $SO_2CF_3$ | |
| 3-58 | H | CHO | H | H | $SO_2CF_3$ | |
| 3-59 | H | $CO_2Et$ | H | H | $SO_2CF_3$ | |
| 3-60 | H | CONHMe | H | H | $SO_2CF_3$ | |
| 3-61 | H | C=NOEt | H | H | $SO_2CF_3$ | |
| 3-62 | H | c-Pr | H | H | $SO_2CF_3$ | |
| 3-63 | H | Br | H | H | $SO_2CF_3$ | |
| 3-64 | H | I | H | H | $SO_2CF_3$ | |

[Chem. 12]

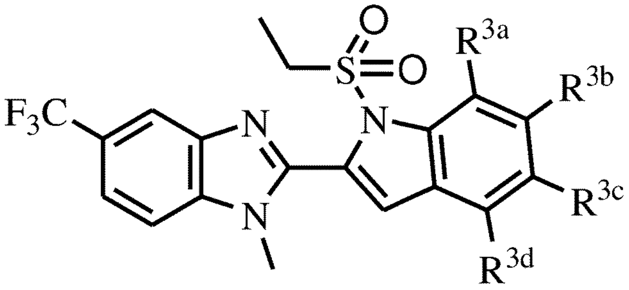

(1-d)

[Table 10]

Table 4

| Compound No. | $R^{3a}$ | $R^{3b}$ | $R^{3c}$ | $R^{3d}$ | Physical property |
|---|---|---|---|---|---|
| 4-1 | H | H | H | H | 135-137 |
| 4-2 | Cl | H | H | H | |
| 4-3 | H | Cl | H | H | |
| 4-4 | H | H | Cl | H | |
| 4-5 | H | H | H | Cl | |
| 4-6 | Me | H | H | H | |
| 4-7 | H | Me | H | H | |
| 4-8 | H | H | Me | H | |
| 4-9 | H | H | H | Me | |
| 4-10 | H | $CF_3$ | H | H | |
| 4-11 | H | H | $CF_3$ | H | |
| 4-12 | H | CN | H | H | |
| 4-13 | H | H | CN | H | |
| 4-14 | H | OMe | H | H | |
| 4-15 | H | H | OMe | H | |
| 4-16 | H | $NH_2$ | H | H | |
| 4-17 | H | H | $NH_2$ | H | |
| 4-18 | H | $NMe_2$ | H | H | |
| 4-19 | H | H | $NMe_2$ | H | |
| 4-20 | H | NHAc | H | H | |
| 4-21 | H | H | NHAc | H | |
| 4-22 | H | SMe | H | H | |
| 4-23 | H | H | SMe | H | |
| 4-24 | H | $SCF_3$ | H | H | |
| 4-25 | H | H | $SCF_3$ | H | |

[Table 11]

Table 4 (Continued)

| Compound No. | $R^{3a}$ | $R^{3b}$ | $R^{3c}$ | $R^{3d}$ | Physical property |
|---|---|---|---|---|---|
| 4-26 | H | CHO | H | H | |
| 4-27 | H | $CO_2Et$ | H | H | |
| 4-28 | H | CONHMe | H | H | |
| 4-29 | H | CH=NOEt | H | H | |
| 4-30 | H | c-Pr | H | H | 106-108 |
| 4-31 | H | Br | H | H | 202-203 |
| 4-32 | H | I | H | H | |
| 4-33 | H | $NHCO_2Et$ | H | H | |
| 4-34 | H | 4-F-Ph | H | H | 114-116 |

[Chem. 13]

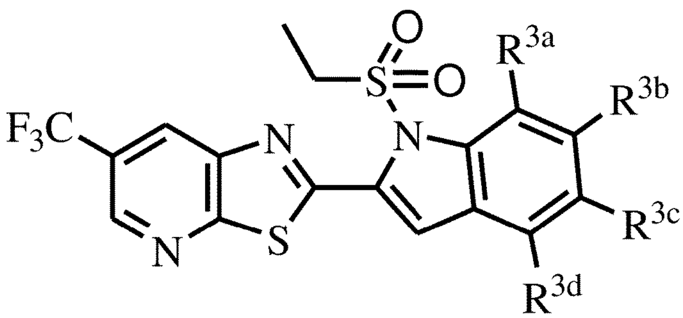

(1-e)

[Table 12]

Table 5

| Compound No. | $R^{3a}$ | $R^{3b}$ | $R^{3c}$ | $R^{3d}$ | Physical property |
|---|---|---|---|---|---|
| 5-1 | H | H | H | H | |
| 5-2 | Cl | H | H | H | |
| 5-3 | H | Cl | H | H | |
| 5-4 | H | H | Cl | H | |
| 5-5 | H | H | H | Cl | |
| 5-6 | Me | H | H | H | |
| 5-7 | H | Me | H | H | |
| 5-8 | H | H | Me | H | |
| 5-9 | H | H | H | Me | |
| 5-10 | H | $CF_3$ | H | H | |
| 5-11 | H | H | $CF_3$ | H | |

(continued)

| Compound No. | $R^{3a}$ | $R^{3b}$ | $R^{3c}$ | $R^{3d}$ | Physical property |
|---|---|---|---|---|---|
| 5-12 | H | CN | H | H | |
| 5-13 | H | H | CN | H | |
| 5-14 | H | OMe | H | H | |
| 5-15 | H | H | OMe | H | |
| 5-16 | H | $NH_2$ | H | H | |
| 5-17 | H | H | $NH_2$ | H | |
| 5-18 | H | $NMe_2$ | H | H | |
| 5-19 | H | H | $NMe_2$ | H | |
| 5-20 | H | NHAc | H | H | |
| 5-21 | H | H | NHAc | H | |
| 5-22 | H | SMe | H | H | |
| 5-23 | H | H | SMe | H | |
| 5-24 | H | $SCF_3$ | H | H | |
| 5-25 | H | H | $SCF_3$ | H | |

[Table 13]

Table 5 (Continued)

| Compound No. | $R^{3a}$ | $R^{3b}$ | $R^{3c}$ | $R^{3d}$ | Physical property |
|---|---|---|---|---|---|
| 5-26 | H | CHO | H | H | |
| 5-27 | H | $CO_2Et$ | H | H | |
| 5-28 | H | CONHMe | H | H | |
| 5-29 | H | CH=NOEt | H | H | |
| 5-30 | H | c-Pr | H | H | |
| 5-31 | H | Br | H | H | |
| 5-32 | H | I | H | H | |

[Chem. 14]

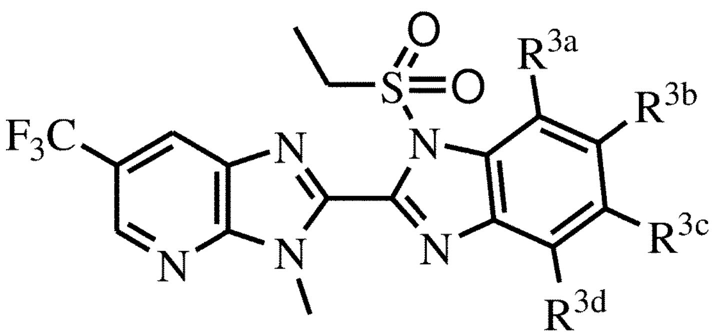

[Table 14]

Table 6

| Compound No. | $R^{3a}$ | $R^{3b}$ | $R^{3c}$ | $R^{3d}$ | Physical property |
|---|---|---|---|---|---|
| 6-1 | H | H | H | H | NMR |
| 6-2 | H | Br | H | H | 203-206 |
| 6-3 | H | H | Br | H | 184-186 |
| 6-4 | H | Br | Br | H | |
| 6-5 | H | $CF_3$ | H | H | 172-173 |
| 6-6 | H | H | $CF_3$ | H | 147-148 |

[Chem. 15]

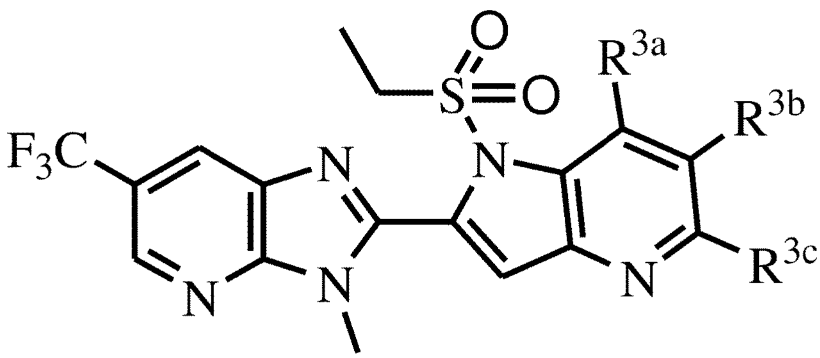

[Table 15]

Table 7

| Compound No. | $R^{3a}$ | $R^{3b}$ | $R^{3c}$ | Physical property |
|---|---|---|---|---|
| 7-1 | H | $CF_3$ | H | 159-163 |

[Chem. 16]

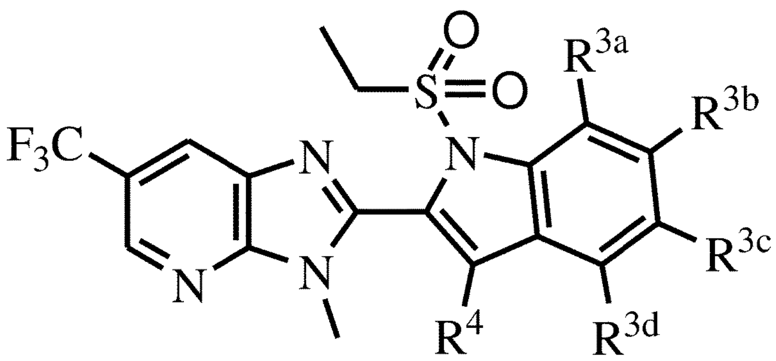

[Table 16]

Table 8

| Compound No. | $R^{3a}$ | $R^{3b}$ | $R^{3c}$ | $R^{3d}$ | $R^4$ | Physical property |
|---|---|---|---|---|---|---|
| 8-1 | H | H | H | H | Me | |
| 8-2 | H | H | H | H | CN | |
| 8-3 | H | H | H | H | OMe | |
| 8-4 | H | H | H | H | $OCH_2CF_3$ | |
| 8-5 | H | H | H | H | $NH_2$ | |
| 8-6 | H | H | H | H | $NMe_2$ | |
| 8-7 | H | H | H | H | NHAc | |
| 8-8 | H | H | H | H | SMe | |
| 8-9 | H | H | H | H | SOMe | |
| 8-10 | H | H | H | H | $SO_2Me$ | |
| 8-11 | H | H | H | H | $SCF_3$ | |
| 8-12 | H | H | H | H | CHO | |
| 8-13 | H | H | H | H | $CO_2Et$ | |
| 8-14 | H | H | H | H | CONHMe | |
| 8-15 | H | H | H | H | C=NOEt | |
| 8-16 | H | H | H | H | $C=NOCH_2CF_3$ | |
| 8-17 | H | H | H | H | c-Pr | |
| 8-18 | H | H | Br | H | Cl | NMR |
| 8-19 | H | Cl | Br | H | Br | 214-215 |
| 8-20 | H | H | H | Cl | Cl | 101-103 |
| 8-21 | H | Cl | OMe | H | Cl | 190-193 |
| 8-22 | H | OMe | Cl | H | Cl | NMR |

[Chem. 17]

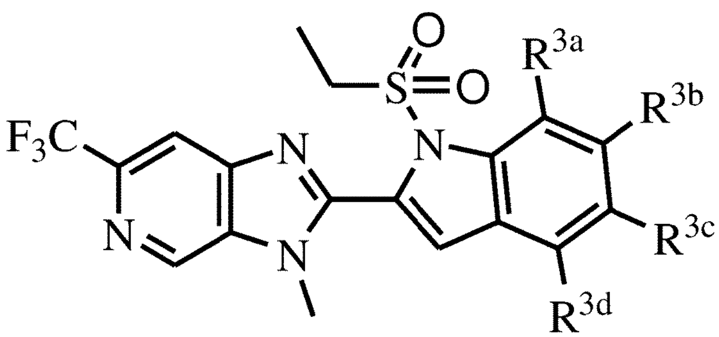

[Table 17]

Table 9

| Compound No. | $R^{3a}$ | $R^{3b}$ | $R^{3c}$ | $R^{3d}$ | Physical property |
|---|---|---|---|---|---|
| 9-1 | H | H | H | H | |
| 9-2 | H | H | Cl | H | |
| 9-3 | H | $CF_3$ | H | H | |
| 9-4 | H | H | $CF_3$ | H | |
| 9-5 | H | H | H | $CF_3$ | |
| 9-6 | H | Br | H | H | |

[Chem. 18]

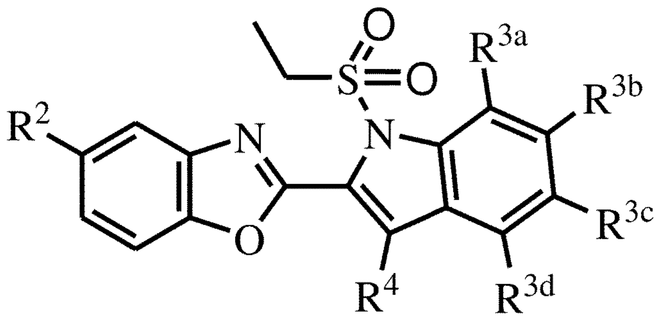

[Table 18]

Table 10

| Compound No. | $R^{3a}$ | $R^{3b}$ | $R^{3c}$ | $R^{3d}$ | $R^4$ | Physical property |
|---|---|---|---|---|---|---|
| 10-1 | H | H | H | H | Me | |
| 10-2 | H | H | H | H | CN | |
| 10-3 | H | H | H | H | OMe | |
| 10-4 | H | H | H | H | $OCH_2CF_3$ | |
| 10-5 | H | H | H | H | $NH_2$ | |
| 10-6 | H | H | H | H | $NMe_2$ | |
| 10-7 | H | H | H | H | NHAc | |
| 10-8 | H | H | H | H | SMe | |
| 10-9 | H | H | H | H | SOMe | |
| 10-10 | H | H | H | H | $SO_2Me$ | |
| 10-11 | H | H | H | H | $SCF_3$ | |
| 10-12 | H | H | H | H | CHO | |
| 10-13 | H | H | H | H | $CO_2Et$ | |
| 10-14 | H | H | H | H | CONHMe | |
| 10-15 | H | H | H | H | C=NOEt | |
| 10-16 | H | H | H | H | $C=NOCH_2CF_3$ | |

(continued)

| Compound No. | $R^{3a}$ | $R^{3b}$ | $R^{3c}$ | $R^{3d}$ | $R^4$ | Physical property |
|---|---|---|---|---|---|---|
| 10-17 | H | H | H | H | c-Pr | |
| 10-18 | H | H | Br | H | Cl | |
| 10-19 | H | Cl | Br | H | Br | |
| 10-20 | H | H | Cl | H | Cl | 112-114 |

[Table 19]

| Compound No. | $^1$H-NMR data ($CDCl_3$) |
|---|---|
| 1-36 | δ 8.20 (s, 1H), 8.08 (d, 1H), 7.74 (d, 1H), 7.50 (dd, 1H), 7.12 ( d, 1H), 4.33 (s, 1H), 4.11 (s, 1H), 3.56 (s, 3H), 1.27 (t, 3H) |
| 6-1 | δ 8.80 (s, 1H), 8.37 (s, 1H), 8.07 (d, 1H), 7.92 (d, 1H), 7.56-7.4 8 (m, 2H), 4.30 (q, 2H), 4.16 (s, 3H), 1.59 (t, 3H) |
| 8-18 | δ 8.80 (d, 1H), 8.36 (d, 1H), 8.03 (dd, 1H), 7.90 (d, 1H), 7.64 ( dd, 1H), 3.89 (s, 3H), 3.90-3.80 (m, 1H), 3.58-3.47 (m, 1H), 1.36 (t, 3H) |
| 8-22 | δ 8.79 (d, 1H), 8.35 (d, 1H), 7.74 (s, 1H), 7.71 (s, 1H), 4.03 (s, 3H), 3.88 (s, 3H), 3.86-3.76 (m, 1H), 3.57-3.48 (m, 1H), 1.35 (t, |
| | 3H) |

The agricultural and horticultural insecticide comprising the compound represented by the general formula (1) of the present invention or a salt thereof as an active ingredient is suitable for controlling a variety of pests which may damage paddy rice, fruit trees, vegetables, other crops and ornamental flowering plants. The target pests are, for example, agricultural and forest pests, horticultural pests, stored grain pests, sanitary pests, nematodes, etc.

Specific examples of the pests, nematodes, etc. include the following:

the species of the order Lepidoptera such as *Parasa consocia, Anomis mesogona, Papilio xuthus, Matsumuraeses azukivora, Ostrinia scapulalis, Spodoptera exempta, Hyphantria cunea, Ostrinia furnacalis, Pseudaletia separata, Tinea translucens, Bactra furfurana, Parnara guttata, Marasmia exigua, Parnara guttata, Sesamia inferens, Brachmia triannulella, Monema flavescens, Trichoplusia ni, Pleuroptya ruralis, Cystidia couaggaria, Lampides boeticus, Cephonodes hylas, Helicoverpa armigera, Phalerodonta manleyi, Eumeta japonica, Pieris brassicae, Malacosoma neustria testacea, Stathmopoda masinissa, Cuphodes diospyrosella, Archips xylosteanus, Agrotis segetum, Tetramoera schistaceana, Papilio machaon hippocrates, Endoclyta sinensis, Lyonetia prunifoliella, Phyllonorycter ringoneella, Cydia kurokoi, Eucoenogenes aestuosa, Lobesia botrana, Latoia sinica, Euzophera batangensis, Phalonidia mesotypa, Spilosoma imparilis, Glyphodes pyloalis, Olethreutes mori, Tineola bisselliella, Endoclyta excrescens, Nemapogon granellus, Synanthedon hector, Cydia pomonella, Plutella xylostella, Cnaphalocrocis medinalis, Sesamia calamistis, Scirpophaga incertulas, Pediasia teterrellus, Phthorimaea operculella, Stauropus fagi persimilis, Etiella zinckenella, Spodoptera exigua, Palpifer sexnotata, Spodoptera mauritia, Scirpophaga innotata, Xestia c-nigrum, Spodoptera depravata, Ephestia kuehniella, Angerona prunaria, Clostera anastomosis, Pseudoplusia includens, Matsumuraeses falcana, Helicoverpa assulta, Autographa nigrisigna, Agrotis ipsilon, Euproctis pseudoconspersa, Adoxophyes orana, Caloptilia theivora, Homona magnanima, Ephestia elutella, Eumeta minuscula, Clostera anachoreta, Heliothis maritima, Sparganothis pilleriana, Busseola fusca, Euproctis subflava, Biston robustum, Heliothis zea, Aedia leucomelas, Narosoideus flavidorsalis, Viminia rumicis, Bucculatrix pyrivorella, Grapholita molesta, Spulerina astaurota, Ectomyelois pyrivorella, Chilo suppressalis, Acrolepiopsis sapporensis, Plodia interpunctella, Hellula undalis, Sitotroga cerealella, Spodoptera litura,* a species of the family Tortricidae (*Eucosma aporema*), *Acleris comariana, Scopelodes contractus, Orgyia thyellina, Spodoptera frugiperda, Ostrinia zaguliaevi, Naranga aenescens, Andraca bipunctata, Paranthrene regalis, Acosmeryx castanea, Phyllocnistis toparcha, Endopiza viteana, Eupoecillia ambiguella, Anticarsia gemmatalis, Cnephasia cinereipalpana, Lymantria dispar, Dendrolimus spectabilis, Leguminivora glycinivorella, Maruca testulalis, Matsumuraeses phaseoli, Caloptilia soyella, Phyllocnistis citrella, Omiodes indicata, Archips fuscocupreanus, Acanthoplusia agnata, Bambalina* sp., *Carposina niponensis, Conogethes punctiferalis, Synanthedon* sp., *Lyonetia clerkella, Papilio helenus, Colias erate*

*poliographus, Phalera flavescens,* the species of the family Pieridae such as *Pieris rapae crucivora* and *Pieris rapae, Euproctis similis, Acrolepiopsis suzukiella, Ostrinia nubilalis, Mamestra brassicae, Ascotis selenaria, Phtheochroides clandestina, Hoshinoa adumbratana, Odonestis pruni japonensis, Triaena intermedia, Adoxophyes orana fasciata, Grapholita inopinata, Spilonota ocellana, Spilonota lechriaspis, Illiberis pruni, Argyresthia conjugella, Caloptilia zachrysa, Archips breviplicanus, Anomis flava, Pectinophora gossypiella, Notarcha derogata, Diaphania indica, Heliothis virescens* and *Earias cupreoviridis;*

the species of the order Hemiptera such as *Nezara antennata, Stenotus rubrovittatus, Graphosoma rubrolineatum, Trigonotylus coelestialium, Aeschynteles maculatus, Creontiades pallidifer, Dysdercus cingulatus, Chrysomphalus ficus, Aonidiella aurantii, Graptopsaltria nigrofuscata, Blissus leucopterus, Icerya purchasi, Piezodorus hybneri, Lagynotomus elongatus, Thaia subrufa, Scotinophara lurida, Sitobion ibarae, Stariodes iwasakii, Aspidiotus destructor, Taylorilygus pallidulus, Myzus mumecola, Pseudaulacaspis prunicola, Acyrthosiphon pisum, Anacanthocoris striicornis, Ectometopterus micantulus, Eysarcoris lewisi, Molipteryx fuliginosa, Cicadella viridis, Rhopalosophum rufiabdominalis, Saissetia oleae, Trialeurodes vaporariorum, Aguriahana quercus, Lygus* spp., *Euceraphis punctipennis, Andaspis kashicola, Coccus pseudomagnoliarum, Cavelerius saccharivorus, Galeatus spinifrons, Macrosiphoniella sanborni, Aonidiella citrina, Halyomorpha mista, Stephanitis fasciicarina, Trioza camphorae, Leptocorisa chinensis, Trioza quercicola, Uhlerites latius, Erythroneura comes, Paromius exiguus, Duplaspidiotus claviger, Nephotettix nigropictus, Halticiellus insularis, Perkinsiella saccharicida, Psylla malivorella, Anomomeura mori, Pseudococcus longispinis, Pseudaulacaspis pentagona, Pulvinaria kuwacola, Apolygus lucorum, Togo hemipterus, Toxoptera aurantii, Saccharicoccus sacchari, Geoica lucifuga, Numata muiri, Comstockaspis perniciosa, Unaspis citri, Aulacorthum solani, Eysarcoris ventralis, Bemisia argentifolii, Cicadella spectra, Aspidiotus hederae, Liorhyssus hyalinus, Calophya nigridorsalis, Sogatella furcifera, Megoura crassicauda, Brevicoryne brassicae, Aphis glycines, Leptocorisa oratorius, Nephotettix virescens, Uroeucon formosanum, Cyrtopeltis tennuis, Bemisia tabaci, Lecanium persicae, Parlatoria theae, Pseudaonidia paeoniae, Empoasca onukii, Plautia stali, Dysaphis tulipae, Macrosiphum euphorbiae, Stephanitis pyrioides, Ceroplastes ceriferus, Parlatoria camelliae, Apolygus spinolai, Nephotettix cincticeps, Glaucias subpunctatus, Orthotylus flavosparsus, Rhopalosiphum maidis, Peregrinus maidis, Eysarcoris parvus, Cimex lectularius, Psylla abieti, Nilaparvata lugens, Psylla tobirae, Eurydema rugosum, Schizaphis piricola, Psylla pyricola, Parlatoreopsis pyri, Stephanitis nashi, Dysmicoccus wistariae, Lepholeucaspis japonica, Sappaphis piri, Lipaphis erysimi, Neotoxoptera formosana, Rhopalosophum nymphaeae, Edwardsiana rosae, Pinnaspis aspidistrae, Psylla alni, Speusotettix subfusculus, Alnetoidia alneti, Sogatella panicicola, Adelphocoris lineolatus, Dysdercus poecilus, Parlatoria ziziphi, Uhlerites debile, Laodelphax striatellus, Eurydema pulchrum, Cletus trigonus, Clovia punctata, Empoasca* spp., *Coccus hesperidum, Pachybrachius luridus, Planococcus kraunhiae, Stenotus binotatus, Arboridia apicalis, Macrosteles fascifrons, Dolycoris baccarum, Adelphocoris triannulatus, Viteus vitifolii, Acanthocoris sordidus, Leptocorisa acuta, Macropes obnubilus, Cletus punctiger, Riptortus clavatus, Paratrioza cockerelli, Aphrophora costalis, Lygus disponsi, Lygus saundersi, Crisicoccus pini, Empoasca abietis, Crisicoccus matsumotoi, Aphis craccivora, Megacopta punctatissimum, Eysarcoris guttiger, Lepidosaphes beckii, Diaphorina citri, Toxoptera citricidus, Planococcus citri, Dialeurodes citri, Aleurocanthus spiniferus, Pseudococcus citriculus, Zyginella citri, Pulvinaria citricola, Coccus discrepans, Pseudaonidia duplex, Pulvinaria aurantii, Lecanium corni, Nezara viridula, Stenodema calcaratum, Rhopalosiphum padi, Sitobion akebiae, Schizaphis graminum, Sorhoanus tritici, Brachycaudus helichrysi, Carpocoris purpureipennis, Myzus persicae, Hyalopterus pruni, Aphis farinose yanagicola, Metasalis populi, Unaspis yanonensis, Mesohomotoma camphorae, Aphis spiraecola, Aphis pomi, Lepidosaphes ulmi, Psylla mali, Heterocordylus flavipes, Myzus malisuctus, Aphidonuguis mali, Orientus ishidai, Ovatus malicolens, Eriosoma lanigerum, Ceroplastes rubens* and *Aphis gossypii;*

the species of the order Coleoptera such as *Xystrocera globosa, Paederus fuscipes, Eucetonia roelofsi, Callosobruchus chinensis, Cylas formicarius, Hypera postica, Echinocnemus squameus, Oulema oryzae, Donacia provosti, Lissorhoptrus oryzophilus, Colasposoma dauricum, Euscepes postfasciatus, Epilachna varivestis, Acanthoscelides obtectus, Diabrotica virgifera virgifera, Involvulus cupreus, Aulacophora femoralis, Bruchus pisorum, Epilachna vigintioctomaculata, Carpophilus dimidiatus, Cassida nebulosa, Luperomorpha tunebrosa, Phyllotreta striolata, Psacothea hilaris, Aeolesthes chrysothrix, Curculio sikkimensis, Carpophilus hemipterus, Oxycetonia jucunda, Diabrotica* spp., *Mimela splendens, Sitophilus zeamais, Tribolium castaneum, Sitophilus oryzae, Palorus subdepressus, Melolontha japonica, Anoplophora malasiaca, Neatus picipes, Leptinotarsa decemlineata, Diabrotica undecimpunctata howardi, Sphenophorus venatus, Crioceris quatuordecimpunctata, Conotrachelus nenuphar, Ceuthorhynchidius albosuturalis, Phaedon brassicae, Lasioderma serricorne, Sitona japonicus, Adoretus tenuimaculatus, Tenebrio molitor, Basilepta balyi, Hypera nigrirostris, Chaetocnema concinna, Anomala cuprea, Heptophylla picea, Epilachna vigintioctopunctata, Diabrotica longicornis, Eucetonia pilifera, Agriotes* spp., *Attagenus unicolor japonicus, Pagria signata, Anomala rufocuprea, Palorus ratzeburgii, Alphitobius laevigatus, Anthrenus verbasci, Lyctus brunneus, Tribolium confusum, Medythia nigrobilineata, Xylotrechus pyrrhoderus, Epitrix cucumeris, Tomicus piniperda, Monochamus alternatus, Popillia japonica, Epicauta gorhami, Sitophilus zeamais, Rhynchites heros, Listroderes costirostris, Callosobruchus maculatus, Phyllobius armatus, Anthonomus pomorum, Linaeidea aenea* and *An-*

*thonomus grandis;*

the species of the order Diptera such as *Culex pipiens pallens, Pegomya hyoscyami, Liriomyza huidobrensis, Musca domestica, Chlorops oryzae, Hydrellia sasakii, Agromyza oryzae, Hydrellia griseola, Hydrellia griseola, Ophiomyia phaseoli, Dacus cucurbitae, Drosophila suzukii, Rhacochlaena japonica, Muscina stabulans,* the species of the family Phoridae such as *Megaselia spiracularis, Clogmia albipunctata, Tipula aino, Phormia regina, Culex tritaeniorhynchus, Anopheles sinensis, Hylemya brassicae, Asphondylia* sp., *Delia platura, Delia antiqua, Rhagoletis cerasi, Culex pipiens molestus* Forskal, *Ceratitis capitata, Bradysia agrestis, Pegomya cunicularia, Liriomyza sativae, Liriomyza bryoniae, Chromatomyia horticola, Liriomyza chinensis, Culex quinquefasciatus, Aedes aegypti, Aedes albopictus, Liriomyza trifolii, Liriomyza sativae, Dacus dorsalis, Dacus tsuneonis, Sitodiplosis mosellana, Meromuza nigriventris, Anastrepha ludens* and *Rhagoletis pomonella;*

the species of the order Hymenoptera such as *Pristomyrmex pungens,* the species of the family Bethylidae, *Monomorium pharaonis, Pheidole noda, Athalia rosae, Dryocosmus kuriphilus, Formica fusca japonica,* the species of the subfamily Vespinae, *Athalia infumata infumata, Arge pagana, Athalia japonica, Acromyrmex* spp., *Solenopsis* spp., *Arge mali* and *Ochetellus glaber;*

the species of the order Orthoptera such as *Homorocoryphus lineosus, Gryllotalpa* sp., *Oxya hyla intricata, Oxya yezoensis, Locusta migratoria, Oxya japonica, Homorocoryphus jezoensis* and *Teleogryllus emma;*

the species of the order Thysanoptera such as *Selenothrips rubrocinctus, Stenchaetothrips biformis, Haplothrips aculeatus, Ponticulothrips diospyrosi, Thrips flavus, Anaphothrips obscurus, Liothrips floridensis, Thrips simplex, Thrips nigropilosus, Heliothrips haemorrhoidalis, Pseudodendrothrips mori, Microcephalothrips abdominalis, Leeuwenia pasanii, Litotetothrips pasaniae, Scirtothrips citri, Haplothrips chinensis, Mycterothrips glycines, Thrips setosus, Scirtothrips dorsalis, Dendrothrips minowai, Haplothrips niger, Thrips tabaci, Thrips alliorum, Thrips hawaiiensis, Haplothrips kurdjumovi, Chirothrips manicatus, Frankliniella intonsa, Thrips coloratus, Franklinella occidentalis, Thrips palmi, Frankliniella lilivora* and *Liothrips vaneeckei;*

the species of the order Acari such as *Leptotrombidium akamushi, Tetranychus ludeni, Dermacentor variabilis, Tetranychus truncatus, Ornithonyssus bacoti, Demodex canis, Tetranychus viennensis, Tetranychus kanzawai,* the species of the family Ixodidae such as *Rhipicephalus sanguineus, Cheyletus malaccensis, Tyrophagus putrescentiae, Dermatophagoides farinae, Latrodectus hasseltii, Dermacentor taiwanicus, Acaphylla theavagrans, Polyphagotarsonemus latus, Aculops lycopersici, Ornithonyssus sylvairum, Tetranychus urticae, Eriophyes chibaensis, Sarcoptes scabiei, Haemaphysalis longicornis, Ixodes scapularis, Tyrophagus similis, Cheyletus eruditus, Panonychus citri, Cheyletus moorei, Brevipalpus phoenicis, Octodectes cynotis, Dermatophagoides ptrenyssnus, Haemaphysalis flava, Ixodes ovatus, Phyllocoptruta citri, Aculus schlechtendali, Panonychus ulmi, Amblyomma americanum, Dermanyssus gallinae, Rhyzoglyphus robini* and *Sancassania* sp. ;

the species of the order Isoptera such as *Reticulitermes miyatakei, Incisitermes minor, Coptotermes formosanus, Hodotermopsis japonica, Reticulitermes* sp., *Reticulitermes flaviceps amamianus, Glyptotermes kushimensis, Coptotermes guangzhoensis, Neotermes koshunensis, Glyptotermes kodamai, Glyptotermes satsumensis, Cryptotermes domesticus, Odontotermes formosanus, Glyptotermes nakajimai, Pericapritermes nitobei* and *Reticulitermes speratus;*

the species of the order Blattodea such as *Periplaneta fuliginosa, Blattella germanica, Blatta orientalis, Periplaneta brunnea, Blattella lituricollis, Periplaneta japonica* and *Periplaneta americana;*

the species of the order Siphonaptera such as *Pulex irritans, Ctenocephalides felis* and *Ceratophyllus gallinae;*

the species of the phylum Nematoda such as *Nothotylenchus acris, Aphelenchoides besseyi, Pratylenchus penetrans, Meloidogyne hapla, Meloidogyne incognita, Globodera rostochiensis, Meloidogyne javanica, Heterodera glycines, Pratylenchus coffeae, Pratylenchus neglectus* and *Tylenchus semipenetrans;* and

the species of the phylum Mollusca such as *Pomacea canaliculata, Achatina fulica, Meghimatium bilineatum, Lehmannina valentiana, Limax flavus* and *Acusta despecta sieboldiana.*

In addition, the agricultural and horticultural insecticide of the present invention has a strong insecticidal effect on *Tuta absoluta* as well.

Further, mites and ticks parasitic on animals are also included in the target pests, and the examples include the species of the family Ixodidae such as *Boophilus microplus, Rhipicephalus sanguineus, Haemaphysalis longicornis, Haemaphysalis flava, Haemaphysalis campanulata, Haemaphysalis concinna, Haemaphysalis japonica, Haemaphysalis kitaokai, Haemaphysalis ias, Ixodes ovatus, Ixodes nipponensis, Ixodes persulcatus, Amblyomma testudinarium, Haemaphysalis megaspinosa, Dermacentor reticulatus* and *Dermacentor taiwanesis; Dermanyssus gallinae;* the species of the genus *Ornithonyssus* such as *Ornithonyssus sylviarum* and *Ornithonyssus bursa;* the species of the family Trombiculidae such as *Eutrombicula wichmanni, Leptotrombidium akamushi, Leptotrombidium pallidum, Leptotrombidium fuji, Leptotrombidium tosa, Neotrombicula autumnalis, Eutrombicula alfreddugesi* and *Helenicula miyagawai;* the species of the family Cheyletidae such as *Cheyletiella yasguri, Cheyletiella parasitivorax* and *Cheyletiella blakei;* the species of the superfamily Sarcoptoidea such as *Psoroptes cuniculi, Chorioptes bovis, Otodectes cynotis, Sarcoptes scabiei* and

*Notoedres cati;* and the species of the family Demodicidae such as *Demodex canis.*

Other target pests include fleas including ectoparasitic wingless insects belonging to the order Siphonaptera, more specifically, the species belonging to the families Pulicidae and Ceratophyllidae. Examples of the species belonging to the family Pulicidae include *Ctenocephalides canis, Ctenocephalides felis, Pulex irritans, Echidnophaga gallinacea, Xenopsylla cheopis, Leptopsylla segnis, Nosopsyllus fasciatus* and *Monopsyllus anisus.*

Other target pests include ectoparasites, for example, the species of the suborder Anoplura such as *Haematopinus eurysternus, Haematopinus asini, Dalmalinia ovis, Linognathus vituli, Haematopinus suis, Phthirus pubis* and *Pediculus capitis;* the species of the suborder Mallophaga such as *Trichodectes canis;* and hematophagous Dipteran insect pests such as *Tabanus trigonus, Culicoides schultzei* and *Simulium ornatum.* In addition, examples of endoparasites include nematodes such as lungworms, whipworms, nodular worms, endogastric parasitic worms, ascarides and filarial worms; cestodes such as *Spirometra erinacei, Diphyllobothrium latum, Dipylidium caninum, Multiceps multiceps, Echinococcus granulosus* and *Echinococcus multilocularis;* trematodes such as *Schistosoma japonicum* and *Fasciola hepatica;* and protozoa such as coccidia, *Plasmodium,* intestinal *Sarcocystis, Toxoplasma* and *Cryptosporidium.*

The agricultural and horticultural insecticide comprising the compound represented by the general formula (1) of the present invention or a salt thereof as an active ingredient has a remarkable control effect on the above-described pests which damage lowland crops, field crops, fruit trees, vegetables, other crops, ornamental flowering plants, etc. The desired effect can be obtained when the agricultural and horticultural insecticide is applied to nursery facilities for seedlings, paddy fields, fields, fruit trees, vegetables, other crops, ornamental flowering plants, etc. and their seeds, paddy water, foliage, cultivation media such as soil, or the like around the expected time of pest infestation, i.e., before the infestation or upon the confirmation of the infestation. In particularly preferable embodiments, the application of the agricultural and horticultural insecticide utilizes so-called penetration and translocation. That is, nursery soil, soil in transplanting holes, plant foot, irrigation water, cultivation water in hydroponics, or the like is treated with the agricultural and horticultural insecticide to allow crops, ornamental flowering plants, etc. to absorb the compound of the present invention through the roots via soil or otherwise.

Examples of useful plants to which the agricultural and horticultural insecticide of the present invention can be applied include, but are not particularly limited to, cereals (e.g., rice, barley, wheat, rye, oats, corn, etc.), legumes (e.g., soybeans, azuki beans, broad beans, green peas, kidney beans, peanuts, etc.), fruit trees and fruits (e.g., apples, citrus fruits, pears, grapes, peaches, plums, cherries, walnuts, chestnuts, almonds, bananas, etc.), leaf and fruit vegetables (e.g., cabbages, tomatoes, spinach, broccoli, lettuce, onions, green onions (chives and Welsh onions), green peppers, eggplants, strawberries, pepper crops, okra, Chinese chives, etc.), root vegetables (e.g., carrots, potatoes, sweet potatoes, taros, Japanese radishes, turnips, lotus roots, burdock roots, garlic, Chinese scallions, etc.), crops for processing (e.g., cotton, hemp, beet, hops, sugarcane, sugar beet, olives, rubber, coffee, tobacco, tea, etc.), gourds (e.g., Japanese pumpkins, cucumbers, watermelons, oriental sweet melons, melons, etc.), pasture grass (e.g., orchardgrass, sorghum, timothy, clover, alfalfa, etc.), lawn grass (e.g., Korean lawn grass, bent grass, etc.), spice and aromatic crops and ornamental crops (e.g., lavender, rosemary, thyme, parsley, pepper, ginger, etc.), ornamental flowering plants (e.g., chrysanthemum, rose, carnation, orchid, tulip, lily, etc.), garden trees (e.g., ginkgo trees, cherry trees, Japanese aucuba, etc.) and forest trees (e.g., *Abies sachalinensis, Picea jezoensis,* pine, yellow cedar, Japanese cedar, hinoki cypress, eucalyptus, etc.).

The above-mentioned "plants" also include plants provided with herbicide tolerance by a classical breeding technique or a gene recombination technique. Examples of such herbicide tolerance include tolerance to HPPD inhibitors, such as isoxaflutole; ALS inhibitors, such as imazethapyr and thifensulfuron-methyl; EPSP synthase inhibitors, such as glyphosate; glutamine synthetase inhibitors, such as glufosinate; acetyl-CoA carboxylase inhibitors, such as sethoxydim; or other herbicides, such as bromoxynil, dicamba and 2,4-D.

Examples of the plants provided with herbicide tolerance by a classical breeding technique include varieties of rapeseed, wheat, sunflower and rice tolerant to the imidazolinone family of ALS-inhibiting herbicides such as imazethapyr, and such plants are sold under the trade name of Clearfield (registered trademark) . Also included is a variety of soybean provided with tolerance to the sulfonyl urea family of ALS-inhibiting herbicides such as thifensulfuron-methyl by a classical breeding technique, and this is sold under the trade name of STS soybean. Also included are plants provided with tolerance to acetyl-CoA carboxylase inhibitors such as trione oxime herbicides and aryloxy phenoxy propionic acid herbicides by a classical breeding technique, for example, SR corn and the like.

Plants provided with tolerance to acetyl-CoA carboxylase inhibitors are described in Proc. Natl. Acad. Sci. USA, 87, 7175-7179 (1990), and the like. Further, acetyl-CoA carboxylase mutants resistant to acetyl-CoA carboxylase inhibitors are reported in Weed Science, 53, 728-746 (2005), and the like, and by introducing the gene of such an acetyl-CoA carboxylase mutant into plants by a gene recombination technique, or introducing a resistance-conferring mutation into acetyl-CoA carboxylase of plants, plants tolerant to acetyl-CoA carboxylase inhibitors can be engineered. Alternatively, by introducing a nucleic acid causing base substitution mutation into plant cells (a typical example of this technique is chimeraplasty technique (Gura T. 1999. Repairing the Genome's Spelling Mistakes. Science 285: 316-318.)) to allow site-specific substitution mutation in the amino acids encoded by an acetyl-CoA carboxylase gene, an ALS gene or the

like of plants, plants tolerant to acetyl-CoA carboxylase inhibitors, ALS inhibitors or the like can be engineered. The agricultural and horticultural insecticide of the present invention can be applied to these plants as well.

Further, exemplary toxins expressed in genetically modified plants include insecticidal proteins of *Bacillus cereus* or *Bacillus popilliae; Bacillus thuringiensis* δ-endotoxins, such as Cry1Ab, Cry1Ac, Cry1F, Cry1Fa2, Cry2Ab, Cry3A, Cry3Bb1 and Cry9C, and other insecticidal proteins, such as VIP1, VIP2, VIP3 and VIP3A; nematode insecticidal proteins; toxins produced by animals, such as scorpion toxins, spider toxins, bee toxins and insect-specific neurotoxins; toxins of filamentous fungi; plant lectins; agglutinin; protease inhibitors, such as trypsin inhibitors, serine protease inhibitors, patatin, cystatin and papain inhibitors; ribosome inactivating proteins (RIP), such as ricin, maize RIP, abrin, luffin, saporin and bryodin; steroid metabolizing enzymes, such as 3-hydroxy steroid oxidase, ecdysteroid-UDP-glucosyltransferase and cholesterol oxidase; ecdysone inhibitors; HMG-CoA reductase; ion channel inhibitors, such as sodium channel inhibitors and calcium channel inhibitors; juvenile hormone esterase; diuretic hormone receptors; stilbene synthase; bibenzyl synthase; chitinase; and glucanase.

Also included are hybrid toxins, partially deficient toxins and modified toxins derived from the following: δ-endotoxin proteins such as Cry1Ab, Cry1Ac, Cry1F, Cry1Fa2, Cry2Ab, Cry3A, Cry3Bb1, Cry9C, Cry34Ab and Cry35Ab, and other insecticidal proteins such as VIP1, VIP2, VIP3 and VIP3A. The hybrid toxin can be produced by combining some domains of these proteins differently from the original combination in nature with the use of a recombination technique. As the partially deficient toxin, a Cry1Ab toxin in which a part of the amino acid sequence is deleted is known. In the modified toxin, one or more amino acids of a naturally occurring toxin are substituted.

Examples of the foregoing toxins and genetically modified plants capable of synthesizing these toxins are described in EP-A-0 374 753, WO 93/07278, WO 95/34656, EP-A-0 427 529, EP-A-451 878, WO 03/052073, etc.

Due to the toxins contained in such genetically modified plants, the plants exhibit resistance to pests, in particular, Coleopteran insect pests, Hemipteran insect pests, Dipteran insect pests, Lepidopteran insect pests and nematodes. The above-described technologies and the agricultural and horticultural insecticide of the present invention can be used in combination or used systematically.

In order to control target pests, the agricultural and horticultural insecticide of the present invention, with or without appropriate dilution or suspension in water etc., is applied to plants potentially infested with the target insect pests or nematodes in an amount effective for the control of the insect pests or nematodes. For example, in order to control insect pests and nematodes that may damage crop plants such as fruit trees, cereals and vegetables, foliar application and seed treatment such as dipping, dust coating and calcium peroxide coating can be performed. Further, treatment of soil or the like may also be performed to allow plants to absorb agrochemicals through their roots. Examples of such treatment include whole soil incorporation, planting row treatment, bed soil incorporation, plug seedling treatment, planting hole treatment, plant foot treatment, top-dressing, treatment of nursery boxes for paddy rice, and submerged application. In addition, application to culture media in hydroponics, smoking treatment, trunk injection and the like can also be performed.

Further, the agricultural and horticultural insecticide of the present invention, with or without appropriate dilution or suspension in water etc., can be applied to sites potentially infested with pests in an amount effective for the control of the pests. For example, it can be directly applied to stored grain pests, house pests, sanitary pests, forest pests, etc., and also be used for coating of residential building materials, for smoking treatment, or as a bait formulation.

Exemplary methods of seed treatment include dipping of seeds in a diluted or undiluted fluid of a liquid or solid formulation for the permeation of agrochemicals into the seeds; mixing or dust coating of seeds with a solid or liquid formulation for the adherence of the formulation onto the surfaces of the seeds; coating of seeds with a mixture of a solid or liquid formulation and an adhesive carrier such as resins and polymers; and application of a solid or liquid formulation to the vicinity of seeds at the same time as seeding.

The term "seed" in the above-mentioned seed treatment refers to a plant body which is in the early stages of cultivation and used for plant propagation. The examples include, in addition to a so-called seed, a plant body for vegetative propagation, such as a bulb, a tuber, a seed potato, a bulbil, a propagule, a discoid stem and a stem used for cuttage.

The term "soil" or "cultivation medium" in the method of the present invention for using an insecticide refers to a support medium for crop cultivation, in particular a support medium which allows crop plants to spread their roots therein, and the materials are not particularly limited as long as they allow plants to grow. Examples of the support medium include what is called soils, seedling mats and water, and specific examples of the materials include sand, pumice, vermiculite, diatomite, agar, gelatinous substances, high-molecular-weight substances, rock wool, glass wool, wood chip and bark.

Exemplary methods of the application to crop foliage or to stored grain pests, house pests, sanitary pests, forest pests, etc. include application of a liquid formulation, such as an emulsifiable concentrate and a flowable, or a solid formulation, such as a wettable powder and a water-dispersible granule, after appropriate dilution in water; dust application; and smoking.

Exemplary methods of soil application include application of a water-diluted or undiluted liquid formulation to

the foot of plants, nursery beds for seedlings, or the like; application of a granule to the foot of plants, nursery beds for seedlings, or the like; application of a dust, a wettable powder, a water-dispersible granule, a granule or the like onto soil and subsequent incorporation of the formulation into the whole soil before seeding or transplanting; and application of a dust, a wettable powder, a water-dispersible granule, a granule or the like to planting holes, planting rows or the like before seeding or planting.

To nursery boxes for paddy rice, for example, a dust, a water-dispersible granule, a granule or the like can be applied, although the suitable formulation may vary depending on the application timing, in other words, depending on the cultivation stage such as seeding time, greening period and planting time. A formulation such as a dust, a water-dispersible granule and a granule may be mixed with nursery soil. For example, such a formulation is incorporated into bed soil, covering soil or the whole soil. Simply, nursery soil and such a formulation may be alternately layered.

In the application to paddy fields, a solid formulation, such as a jumbo, a pack, a granule and a water-dispersible granule, or a liquid formulation, such as a flowable and an emulsifiable concentrate, is applied usually to flooded paddy fields. In a rice planting period, a suitable formulation, as it is or after mixed with a fertilizer, may be applied onto soil or injected into soil. In addition, an emulsifiable concentrate, a flowable or the like may be applied to the source of water supply for paddy fields, such as a water inlet and an irrigation device. In this case, treatment can be accomplished with the supply of water and thus achieved in a labor-saving manner.

In the case of field crops, their seeds, cultivation media in the vicinity of their plants, or the like may be treated in the period of seeding to seedling culture. In the case of plants of which the seeds are directly sown in the field, in addition to direct seed treatment, plant foot treatment during cultivation is preferable. Specifically, the treatment can be performed by, for example, applying a granule onto soil, or drenching soil with a formulation in a water-diluted or undiluted liquid form. Another preferable treatment is incorporation of a granule into cultivation media before seeding.

In the case of culture plants to be transplanted, preferable examples of the treatment in the period of seeding to seedling culture include, in addition to direct seed treatment, drench treatment of nursery beds for seedlings with a formulation in a liquid form; and granule application to nursery beds for seedlings. Also included are treatment of planting holes with a granule; and incorporation of a granule into cultivation media in the vicinity of planting points at the time of fix planting.

The agricultural and horticultural insecticide of the present invention is commonly used as a formulation convenient for application, which is prepared in the usual method for preparing agrochemical formulations.

That is, the condensed heterocyclic compound represented by the general formula (1) of the present invention or a salt thereof and an appropriate inactive carrier, and if needed an adjuvant, are blended in an appropriate ratio, and through the step of dissolution, separation, suspension, mixing, impregnation, adsorption and/or adhesion, are formulated into an appropriate form for application, such as a suspension concentrate, an emulsifiable concentrate, a soluble concentrate, a wettable powder, a water-dispersible granule, a granule, a dust, a tablet and a pack.

The composition (agricultural and horticultural insecticide or animal parasite control agent) of the present invention can optionally contain an additive usually used for agrochemical formulations or animal parasite control agents in addition to the active ingredient. Examples of the additive include carriers such as solid or liquid carriers, surfactants, dispersants, wetting agents, binders, tackifiers, thickeners, colorants, spreaders, sticking/spreading agents, antifreezing agents, anti-caking agents, disintegrants and stabilizing agents. If needed, preservatives, plant fragments, etc. may also be used as the additive. One of these additives may be used alone, and also two or more of them may be used in combination.

Examples of the solid carriers include natural minerals, such as quartz, clay, kaolinite, pyrophyllite, sericite, talc, bentonite, acid clay, attapulgite, zeolite and diatomite; inorganic salts, such as calcium carbonate, ammonium sulfate, sodium sulfate and potassium chloride; organic solid carriers, such as synthetic silicic acid, synthetic silicates, starch, cellulose and plant powders (for example, sawdust, coconut shell, corn cob, tobacco stalk, etc.); plastics carriers, such as polyethylene, polypropylene and polyvinylidene chloride; urea; hollow inorganic materials; hollow plastic materials; and fumed silica (white carbon) . One of these solid carriers may be used alone, and also two or more of them may be used in combination.

Examples of the liquid carriers include alcohols including monohydric alcohols, such as methanol, ethanol, propanol, isopropanol and butanol, and polyhydric alcohols, such as ethylene glycol, diethylene glycol, propylene glycol, hexylene glycol, polyethylene glycol, polypropylene glycol and glycerin; polyol compounds, such as propylene glycol ether; ketones, such as acetone, methyl ethyl ketone, methyl isobutyl ketone, diisobutyl ketone and cyclohexanone; ethers, such as ethyl ether, dioxane, ethylene glycol monoethyl ether, dipropyl ether and tetrahydrofuran (THF); aliphatic hydrocarbons, such as normal paraffin, naphthene, isoparaffin, kerosene and mineral oil; aromatic hydrocarbons, such as benzene, toluene, xylene, solvent naphtha and alkyl naphthalene; halogenated hydrocarbons, such as dichloromethane, chloroform and carbon tetrachloride; esters, such as ethyl acetate, diisopropyl phthalate, dibutyl phthalate, dioctyl phthalate and dimethyl adipate; lactones, such as γ-butyrolactone; amides, such as dimethylformamide, diethylformamide, dimethylacetamide and N-alkyl pyrrolidinone; nitriles, such as acetonitrile; sulfur compounds, such as dimethyl sulfoxide; vegetable oils, such as soybean oil, rapeseed oil, cotton seed oil and castor oil; and water. One of these liquid

carriers may be used alone, and also two or more of them may be used in combination.

Exemplary surfactants used as the dispersant or the wetting/spreading agent include nonionic surfactants, such as sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkyl aryl ether, polyoxyethylene alkyl phenyl ether, polyoxyethylene dialkyl phenyl ether, polyoxyethylene alkyl phenyl ether-formaldehyde condensates, polyoxyethylene-polyoxypropylene block copolymers, polystyrene-polyoxyethylene block polymers, alkyl polyoxyethylene-polypropylene block copolymer ether, polyoxyethylene alkylamine, polyoxyethylene fatty acid amide, polyoxyethylene fatty acid bis(phenyl ether), polyalkylene benzyl phenyl ether, polyoxyalkylene styryl phenyl ether, acetylene diol, polyoxyalkylene-added acetylene diol, polyoxyethylene ether-type silicone, ester-type silicone, fluorosurfactants, polyoxyethylene castor oil and polyoxyethylene hydrogenated castor oil; anionic surfactants, such as alkyl sulfates, polyoxyethylene alkyl ether sulfates, polyoxyethylene alkyl phenyl ether sulfates, polyoxyethylene styryl phenyl ether sulfates, alkylbenzene sulfonates, alkylaryl sulfonates, lignosulfonates, alkyl sulfosuccinates, naphthalene sulfonates, alkylnaphthalene sulfonates, salts of naphthalenesulfonic acid-formaldehyde condensates, salts of alkylnaphthalenesulfonic acid-formaldehyde condensates, fatty acid salts, polycarboxylic acid salts, polyacrylates, N-methyl-fatty acid sarcosinates, resinates, polyoxyethylene alkyl ether phosphates and polyoxyethylene alkyl phenyl ether phosphates; cationic surfactants including alkyl amine salts, such as lauryl amine hydrochloride, stearyl amine hydrochloride, oleyl amine hydrochloride, stearyl amine acetate, stearyl aminopropyl amine acetate, alkyl trimethyl ammonium chloride and alkyl dimethyl benzalkonium chloride; and amphoteric surfactants, such as amino acid-type or betaine-type amphoteric surfactants. One of these surfactants may be used alone, and also two or more of them may be used in combination.

Examples of the binders or the tackifiers include carboxymethyl cellulose or salts thereof, dextrin, soluble starch, xanthan gum, guar gum, sucrose, polyvinyl pyrrolidone, gum arabic, polyvinyl alcohol, polyvinyl acetate, sodium polyacrylate, polyethylene glycols with an average molecular weight of 6,000 to 20,000, polyethylene oxides with an average molecular weight of 100,000 to 5,000,000, phospholipids (for example, cephalin, lecithin, etc.), cellulose powder, dextrin, modified starch, polyaminocarboxylic acid chelating compounds, cross-linked polyvinyl pyrrolidone, maleic acid-styrene copolymers, (meth)acrylic acid copolymers, half esters of polyhydric alcohol polymer and dicarboxylic anhydride, water soluble polystyrene sulfonates, paraffin, terpene, polyamide resins, polyacrylates, polyoxyethylene, waxes, polyvinyl alkyl ether, alkylphenol-formaldehyde condensates and synthetic resin emulsions.

Examples of the thickeners include water soluble polymers, such as xanthan gum, guar gum, diutan gum, carboxymethyl cellulose, polyvinyl pyrrolidone, carboxyvinyl polymers, acrylic polymers, starch compounds and polysaccharides; and inorganic fine powders, such as high grade bentonite and fumed silica (white carbon).

Examples of the colorants include inorganic pigments, such as iron oxide, titanium oxide and Prussian blue; and organic dyes, such as alizarin dyes, azo dyes and metal phthalocyanine dyes.

Examples of the antifreezing agents include polyhydric alcohols, such as ethylene glycol, diethylene glycol, propylene glycol and glycerin.

Examples of the adjuvants serving to prevent caking or facilitate disintegration include polysaccharides (starch, alginic acid, mannose, galactose, etc.), polyvinyl pyrrolidone, fumed silica (white carbon), ester gum, petroleum resin, sodium tripolyphosphate, sodium hexametaphosphate, metal stearates, cellulose powder, dextrin, methacrylate copolymers, polyvinyl pyrrolidone, polyaminocarboxylic acid chelating compounds, sulfonated styrene-isobutylene-maleic anhydride copolymers and starch-polyacrylonitrile graft copolymers.

Examples of the stabilizing agents include desiccants, such as zeolite, quicklime and magnesium oxide; antioxidants, such as phenolic compounds, amine compounds, sulfur compounds and phosphoric acid compounds; and ultraviolet absorbers, such as salicylic acid compounds and benzophenone compounds.

Examples of the preservatives include potassium sorbate and 1,2-benzothiazolin-3-one.

Further, other adjuvants including functional spreading agents, activity enhancers such as metabolic inhibitors (piperonyl butoxide etc.), antifreezing agents (propylene glycol etc.), antioxidants (BHT etc.) and ultraviolet absorbers can also be used if needed.

The amount of the active ingredient compound in the agricultural and horticultural insecticide of the present invention can be adjusted as needed, and basically, the amount of the active ingredient compound is appropriately selected from the range of 0.01 to 90 parts by weight in 100 parts by weight of the agricultural and horticultural insecticide. For example, in the case where the agricultural and horticultural insecticide is a dust, a granule, an emulsifiable concentrate or a wettable powder, it is suitable that the amount of the active ingredient compound is 0.01 to 50 parts by weight (0.01 to 50% by weight relative to the total weight of the agricultural and horticultural insecticide).

The application rate of the agricultural and horticultural insecticide of the present invention may vary with various factors, for example, the purpose, the target pest, the growing conditions of crops, the tendency of pest infestation, the weather, the environmental conditions, the dosage form, the application method, the application site, the application timing, etc., but basically, the application rate of the active ingredient compound is appropriately selected from the range of 0.001 g to 10 kg, and preferably 0.01 g to 1 kg per 10 ares depending on the purpose.

Furthermore, for the expansion of the range of target pests and the appropriate time for pest control, or for dose reduction, the agricultural and horticultural insecticide of the present invention can be used after mixed with other agricultural and horticultural insecticides, acaricides, nematicides, microbicides, biopesticides and/or the like. Further, the agricultural and horticultural insecticide can be used after mixed with herbicides, plant growth regulators, fertilizers and/or the like depending on the situation.

Examples of such additional agricultural and horticultural insecticides, acaricides and nematicides used for the above-mentioned purposes include 3,5-xylyl methylcarbamate (XMC), crystalline protein toxins produced by *Bacillus thuringiensis* such as *Bacillus thuringiensis aizawai, Bacillus thuringiensis israelensis, Bacillus thuringiensis japonensis, Bacillus thuringiensis kurstaki* and *Bacillus thuringiensis tenebrionis,* BPMC, Bt toxin-derived insecticidal compounds, CPCBS (chlorfenson), DCIP (dichlorodiisopropyl ether), D-D (1,3-dichloropropene), DDT, NAC, O-4-dimethylsulfamoylphenyl O,O-diethyl phosphorothioate (DSP), O-ethyl O-4-nitrophenyl phenylphosphonothioate (EPN), tripropylisocyanurate (TPIC), acrinathrin, azadirachtin, azinphos-methyl, acequinocyl, acetamiprid, acetoprole, acephate, abamectin, avermectin-B, amidoflumet, amitraz, alanycarb, aldicarb, aldoxycarb, aldrin, alpha-endosulfan, alpha-cypermethrin, albendazole, allethrin, isazofos, isamidofos, isoamidofos isoxathion, isofenphos, isoprocarb (MIPC), ivermectin, imicyafos, imidacloprid, imiprothrin, indoxacarb, esfenvalerate, ethiofencarb, ethion, ethiprole, etoxazole, ethofenprox, ethoprophos, etrimfos, emamectin, emamectin-benzoate, endosulfan, empenthrin, oxamyl, oxydemeton-methyl, oxydeprofos (ESP), oxibendazole, oxfendazole, potassium oleate, sodium oleate, cadusafos, cartap, carbaryl, carbosulfan, carbofuran, gamma-cyhalothrin, xylylcarb, quinalphos, kinoprene, chinomethionat, cloethocarb, clothianidin, clofentezine, chromafenozide, chlorantraniliprole, chlorethoxyfos, chlordimeform, chlordane, chlorpyrifos, chlorpyrifos-methyl, chlorphenapyr, chlorfenson, chlorfenvinphos, chlorfluazuron, chlorobenzilate, chlorobenzoate, kelthane (dicofol), salithion, cyanophos (CYAP), diafenthiuron, diamidafos, cyantraniliprole, theta-cypermethrin, dienochlor, cyenopyrafen, dioxabenzofos, diofenolan, sigma-cypermethrin, dichlofenthion (ECP), cycloprothrin, dichlorvos (DDVP), disulfoton, dinotefuran, cyhalothrin, cyphenothrin, cyfluthrin, diflubenzuron, cyflumetofen, diflovidazin, cyhexatin, cypermethrin, dimethylvinphos, dimethoate, dimefluthrin, silafluofen, cyromazine, spinetoram, spinosad, spirodiclofen, spirotetramat, spiromesifen, sulfluramid, sulprofos, sulfoxaflor, zeta-cypermethrin, diazinon, tau-fluvalinate, dazomet, thiacloprid, thiamethoxam, thiodicarb, thiocyclam, thiosultap, thiosultap-sodium, thionazin, thiometon, deet, dieldrin, tetrachlorvinphos, tetradifon, tetramethylfluthrin, tetramethrin, tebupirimfos, tebufenozide, tebufenpyrad, tefluthrin, teflubenzuron, demeton-S-methyl, temephos, deltamethrin, terbufos, tralopyril, tralomethrin, transfluthrin, triazamate, triazuron, trichlamide, trichlorphon (DEP), triflumuron, tolfenpyrad, naled (BRP), nithiazine, nitenpyram, novaluron, noviflumuron, hydroprene, vaniliprole, vamidothion, parathion, parathion-methyl, halfenprox, halofenozide, bistrifluron, bisultap, hydramethylnon, hydroxy propyl starch, binapacryl, bifenazate, bifenthrin, pymetrozine, pyraclofos, pyrafluprole, pyridafenthion, pyridaben, pyridalyl, pyrifluquinazon, pyriprole, pyriproxyfen, pirimicarb, pyrimidifen, pirimiphos-methyl, pyrethrins, fipronil, fenazaquin, fenamiphos, bromopropylate, fenitrothion (MEP), fenoxycarb, fenothiocarb, phenothrin, fenobucarb, fensulfothion, fenthion (MPP), phenthoate (PAP), fenvalerate, fenpyroximate, fenpropathrin, fenbendazole, fosthiazate, formetanate, butathiofos, buprofezin, furathiocarb, prallethrin, fluacrypyrim, fluazinam, fluazuron, fluensulfone, flucycloxuron, flucythrinate, fluvalinate, flupyrazofos, flufenerim, flufenoxuron, flufenzine, flufenprox, fluproxyfen, flubrocythrinate, flubendiamide, flumethrin, flurimfen, prothiofos, protrifenbute, flonicamid, propaphos, propargite (BPPS), profenofos, profluthrin, propoxur (PHC), bromopropylate, beta-cyfluthrin, hexaflumuron, hexythiazox, heptenophos, permethrin, benclothiaz, bendiocarb, bensultap, benzoximate, benfuracarb, phoxim, phosalone, fosthiazate, fosthietan, phosphamidon, phosphocarb, phosmet (PMP), polynactins, formetanate, formothion, phorate, machine oil, malathion, milbemycin, milbemycin-A, milbemectin, mecarbam, mesulfenfos, methomyl, metaldehyde, metaflumizone, methamidophos, metam-ammonium, metam-sodium, methiocarb, methidathion (DMTP), methylisothiocyanate, methylneodecanamide, methylparathion, metoxadiazone, methoxychlor, methoxyfenozide, metofluthrin, methoprene, metolcarb, meperfluthrin, mevinphos, monocrotophos, monosultap, lambda-cyhalothrin, ryanodine, lufenuron, resmethrin, lepimectin, rotenone, levamisole hydrochloride, fenbutatin oxide, morantel tartarate, methyl bromide, tricyclohexyltin hydroxide (cyhexatin), calcium cyanamide, calcium polysulfide, sulfur and nicotine-sulfate.

Exemplary agricultural and horticultural microbicides used for the same purposes as above include aureofungin, azaconazole, azithiram, acypetacs, acibenzolar, acibenzolar-S-methyl, azoxystrobin, anilazine, amisulbrom, ampropylfos, ametoctradin, allyl alcohol, aldimorph, amobam, isotianil, isovaledione, isopyrazam, isoprothiolane, ipconazole, iprodione, iprovalicarb, iprobenfos, imazalil, iminoctadine, iminoctadine-albesilate, iminoctadine-triacetate, imibenconazole, uniconazole, uniconazole-P, echlomezole, edifenphos, etaconazole, ethaboxam, ethirimol, etem, ethoxyquin, etridiazole, enestroburin, epoxiconazole, oxadixyl, oxycarboxin, copper-8-quinolinolate, oxytetracycline, copper-oxinate, oxpoconazole, oxpoconazole-fumarate, oxolinic acid, octhilinone, ofurace, orysastrobin, soil fungicides such as metam-sodium, kasugamycin, carbamorph, carpropamid, carbendazim, carboxin, carvone, quinazamid, quinacetol, quinoxyfen, quinomethionate, captafol, captan, kiralaxyl, quinconazole, quintozene, guazatine, cufraneb, cuprobam, glyodin, griseofulvin, climbazole, cresol, kresoxim-methyl, chlozolinate, clotrimazole, chlobenthiazone, chloraniformethan, chloranil, chlorquinox, chloropicrin, chlorfenazole, chlorodinitronaphthalene, chlorothalonil, chloroneb, zarilamid, salicylanilide, cyazofamid, diethyl pyrocarbonate, diethofencarb, cyclafuramid, diclocymet, dichlozoline, diclobutrazol, dichlofluanid,

cycloheximide, diclomezine, dicloran, dichlorophen, dichlone, disulfiram, ditalimfos, dithianon, diniconazole, diniconazole-M, zineb, dinocap, dinocton, dinosulfon, dinoterbon, dinobuton, dinopenton, dipyrithione, diphenylamine, difenoconazole, cyflufenamid, diflumetorim, cyproconazole, cyprodinil, cyprofuram, cypendazole, simeconazole, dimethirimol, dimethomorph, cymoxanil, dimoxystrobin, methyl bromide, ziram, silthiofam, streptomycin, spiroxamine, sultropen, sedaxane, zoxamide, dazomet, thiadiazin, tiadinil, thiadifluor, thiabendazole, tioxymid, thiochlorfenphim, thiophanate, thiophanate-methyl, thicyofen, thioquinox, chinomethionat, thifluzamide, thiram, decafentin, tecnazene, tecloftalam, tecoram, tetraconazole, debacarb, dehydroacetic acid, tebuconazole, tebufloquin, dodicin, dodine, dodecyl benzensulfonate bis-ethylene diamine copper(II) (DBEDC), dodemorph, drazoxolon, triadimenol, triadimefon, triazbutil, triazoxide, triamiphos, triarimol, trichlamide, tricyclazole, triticonazole, tridemorph, tributyltin oxide, triflumizole, trifloxystrobin, triforine, tolylfluanid, tolclofos-methyl, natamycin, nabam, nitrothal-isopropyl, nitrostyrene, nuarimol, copper nonylphenol sulfonate, halacrinate, validamycin, valifenalate, harpin protein, bixafen, picoxystrobin, picobenzamide, bithionol, bitertanol, hydroxyisoxazole, hydroxyisoxazole-potassium, binapacryl, biphenyl, piperalin, hymexazol, pyraoxystrobin, pyracarbolid, pyraclostrobin, pyrazophos, pyrametostrobin, pyriofenone, pyridinitril, pyrifenox, pyribencarb, pyrimethanil, pyroxychlor, pyroxyfur, pyroquilon, vinclozolin, famoxadone, fenapanil, fenamidone, fenaminosulf, fenarimol, fenitropan, fenoxanil, ferimzone, ferbam, fentin, fenpiclonil, fenpyrazamine, fenbuconazole, fenfuram, fenpropidin, fenpropimorph, fenhexamid, phthalide, buthiobate, butylamine, bupirimate, fuberidazole, blasticidin-S, furametpyr, furalaxyl, fluacrypyrim, fluazinam, fluoxastrobin, fluotrimazole, fluopicolide, fluopyram, fluoroimide, furcarbanil, fluxapyroxad, fluquinconazole, furconazole, furconazole-cis, fludioxonil, flusilazole, flusulfamide, flutianil, flutolanil, flutriafol, furfural, furmecyclox, flumetover, flumorph, proquinazid, prochloraz, procymidone, prothiocarb, prothioconazole, propamocarb, propiconazole, propineb, furophanate, probenazole, bromuconazole, hexachlorobutadiene, hexaconazole, hexylthiofos, bethoxazin, benalaxyl, benalaxyl-M, benodanil, benomyl, pefurazoate, benquinox, penconazole, benzamorf, pencycuron, benzohydroxamic acid, bentaluron, benthiazole, benthiavalicarb-isopropyl, penthiopyrad, penflufen, boscalid, phosdiphen, fosetyl, fosetyl-Al, polyoxins, polyoxorim, polycarbamate, folpet, formaldehyde, machine oil, maneb, mancozeb, mandipropamid, myclozolin, myclobutanil, mildiomycin, milneb, mecarbinzid, methasulfocarb, metazoxolon, metam, metam-sodium, metalaxyl, metalaxyl-M, metiram, methyl isothiocyanate, meptyldinocap, metconazole, metsulfovax, methfuroxam, metominostrobin, metrafenone, mepanipyrim, mefenoxam, meptyldinocap, mepronil, mebenil, iodomethane, rabenzazole, benzalkonium chloride, basic copper chloride, basic copper sulfate, inorganic microbicides such as silver, sodium hypochlorite, cupric hydroxide, wettable sulfur, calcium polysulfide, potassium hydrogen carbonate, sodium hydrogen carbonate, sulfur, copper sulfate anhydride, nickel dimethyldithiocarbamate, copper compounds such as copper-8-quinolinolate (oxine copper), zinc sulfate and copper sulfate pentahydrate.

Exemplary herbicides used for the same purposes as above include 1-naphthylacetamide, 2,4-PA, 2,3,6-TBA, 2,4,5-T, 2,4,5-TB, 2,4-D, 2,4-DB, 2,4-DEB, 2,4-DEP, 3,4-DA, 3,4-DB, 3,4-DP, 4-CPA, 4-CPB, 4-CPP, MCP, MCPA, MCPA-thioethyl, MCPB, ioxynil, aclonifen, azafenidin, acifluorfen, aziprotryne, azimsulfuron, asulam, acetochlor, atrazine, atraton, anisuron, anilofos, aviglycine, abscisic acid, amicarbazone, amidosulfuron, amitrole, aminocyclopyrachlor, aminopyralid, amibuzin, amiprophos-methyl, ametridione, ametryn, alachlor, allidochlor, alloxydim, alorac, isouron, isocarbamid, isoxachlortole, isoxapyrifop, isoxaflutole, isoxaben, isocil, isonoruron, isoproturon, isopropalin, isopolinate, isomethiozin, inabenfide, ipazine, ipfencarbazone, iprymidam, imazaquin, imazapic, imazapyr, imazamethapyr, imazamethabenz, imazamethabenz-methyl, imazamox, imazethapyr, imazosulfuron, indaziflam, indanofan, indolebutyric acid, uniconazole-P, eglinazine, esprocarb, ethametsulfuron, ethametsulfuron-methyl, ethalfluralin, ethiolate, ethychlozate-ethyl, ethidimuron, etinofen, ethephon, ethoxysulfuron, ethoxyfen, etnipromid, ethofumesate, etobenzanid, epronaz, erbon, endothal, oxadiazon, oxadiargyl, oxaziclomefone, oxasulfuron, oxapyrazon, oxyfluorfen, oryzalin, orthosulfamuron, orbencarb, cafenstrole, cambendichlor, carbasulam, carfentrazone, carfentrazone-ethyl, karbutilate, carbetamide, carboxazole, quizalofop, quizalofop-P, quizalofop-ethyl, xylachlor, quinoclamine, quinonamid, quinclorac, quinmerac, cumyluron, cliodinate, glyphosate, glufosinate, glufosinate-P, credazine, clethodim, cloxyfonac, clodinafop, clodinafop-propargyl, chlorotoluron, clopyralid, cloproxydim, cloprop, chlorbromuron, clofop, clomazone, chlomethoxynil, chlomethoxyfen, clomeprop, chlorazifop, chlorazine, cloransulam, chloranocryl, chloramben, cloransulam-methyl, chloridazon, chlorimuron, chlorimuron-ethyl, chlorsulfuron, chlorthal, chlorthiamid, chlortoluron, chlornitrofen, chlorfenac, chlorfenprop, chlorbufam, chlorflurazole, chlorflurenol, chlorprocarb, chlorpropham, chlormequat, chloreturon, chloroxynil, chloroxuron, chloropon, saflufenacil, cyanazine, cyanatryn, di-allate, diuron, diethamquat, dicamba, cycluron, cycloate, cycloxydim, diclosulam, cyclosulfamuron, dichlorprop, dichlorprop-P, dichlobenil, diclofop, diclofop-methyl, dichlormate, dichloralurea, diquat, cisanilide, disul, siduron, dithiopyr, dinitramine, cinidon-ethyl, dinosam, cinosulfuron, dinoseb, dinoterb, dinofenate, dinoprop, cyhalofop-butyl, diphenamid, difenoxuron, difenopenten, difenzoquat, cybutryne, cyprazine, cyprazole, diflufenican, diflufenzopyr, dipropetryn, cypromid, cyperquat, gibberellin, simazine, dimexano, dimethachlor, dimidazon, dimethametryn, dimethenamid, simetryn, simeton, dimepiperate, dimefuron, cinmethylin, swep, sulglycapin, sulcotrione, sulfallate, sulfentrazone, sulfosulfuron, sulfometuron, sulfometuron-methyl, secbumeton, sethoxydim, sebuthylazine, terbacil, daimuron, dazomet, dalapon, thiazafluron, thiazopyr, thiencarbazone, thiencarbazone-methyl, tiocarbazil, tioclorim, thiobencarb, thidiazimin, thidiazuron, thifensulfuron, thifensulfuron-methyl, desmedipham, desmetryn, tetrafluron, thenylchlor, tebutam, tebuthiuron, terbumeton, tepraloxydim, tefuryltrione, tembotrione, delachlor, terbacil, terbucarb, terbuchlor, terbuthylazine, terbutryn, topramezone, tralkoxydim, triaziflam, triasulfuron, tri-allate, trietazine, tricamba, triclopyr, tridiphane, tritac, tritosulfuron, triflusulfuron, triflusulfuron-methyl, trifluralin, trifloxysulfuron, tripropindan, tribenuron-methyl, tribenuron, trifop, trifopsime, trimeturon, naptalam, naproanilide, napropamide, nicosulfuron, nitralin, nitrofen, nitrofluorfen, nipyraclofen, neburon, norflurazon, noruron, barban, paclobutrazol, paraquat, parafluron, haloxydine, haloxyfop, haloxyfop-P, haloxyfop-methyl, halosafen, halosulfuron, halosulfuron-methyl, picloram, picolinafen, bicyclopyrone, bispyribac, bispyribac-sodium, pydanon, pinoxaden, bifenox, piperophos, hymexazol, pyraclonil, pyrasulfotole, pyrazoxyfen, pyrazosulfuron, pyrazosulfuron-ethyl, pyrazolate, bilanafos, pyraflufen-ethyl, pyriclor, pyridafol, pyrithiobac, pyrithiobac-sodium, pyridate, pyriftalid, pyributicarb, pyribenzoxim, pyrimisulfan, primisulfuron, pyriminobac-methyl, pyroxasulfone, pyroxsulam, fenasulam, phenisopham, fenuron, fenoxasulfone, fenoxaprop, fenoxaprop-P, fenoxaprop-ethyl, phenothiol, fenoprop, phenobenzuron, fenthiaprop, fenteracol, fentrazamide, phenmedipham, phenmedipham-ethyl, butachlor, butafenacil, butamifos, buthiuron, buthidazole, butylate, buturon, butenachlor, butroxydim, butralin, flazasulfuron, flamprop, furyloxyfen, prynachlor, primisulfuron-methyl, fluazifop, fluazifop-P, fluazifop-butyl, fluazolate, fluroxypyr, fluothiuron, fluometuron, fluoroglycofen, flurochloridone, fluorodifen, fluoronitrofen, fluoromidine, flucarbazone, flucarbazone-sodium, fluchloralin, flucetosulfuron, fluthiacet, fluthiacet-methyl, flupyrsulfuron, flufenacet, flufenican, flufenpyr, flupropacil, flupropanate, flupoxam, flumioxazin, flumiclorac, flumiclorac-pentyl, flumipropyn, flumezin, fluometuron, flumetsulam, fluridone, flurtamone, fluroxypyr, pretilachlor, proxan, proglinazine, procyazine, prodiamine, prosulfalin, prosulfuron, prosulfocarb, propaquizafop, propachlor, propazine, propanil, propyzamide, propisochlor, prohydrojasmon, propyrisulfuron, propham, profluazol, profluralin, prohexadione-calcium, propoxycarbazone, propoxycarbazone-sodium, profoxydim, bromacil, brompyrazon, prometryn, prometon, bromoxynil, bromofenoxim, bromobutide, bromobonil, florasulam, hexachloroacetone, hexazinone, pethoxamid, benazolin, penoxsulam, pebulate, beflubutamid, vernolate, perfluidone, bencarbazone, benzadox, benzipram, benzylaminopurine, benzthiazuron, benzfendizone, bensulide, bensulfuron-methyl, benzoylprop, benzobicyclon, benzofenap, benzofluor, bentazone, pentanochlor, benthiocarb, pendimethalin, pentoxazone, benfluralin, benfuresate, fosamine, fomesafen, foramsulfuron, forchlorfenuron, maleic hydrazide, mecoprop, mecoprop-P, medinoterb, mesosulfuron, mesosulfuron-methyl, mesotrione, mesoprazine, methoprotryne, metazachlor, methazole, metazosulfuron, methabenzthiazuron, metamitron, metamifop, metam, methalpropalin, methiuron, methiozolin, methiobencarb, methyldymron, metoxuron, metosulam, metsulfuron, metsulfuron-methyl, metflurazon, metobromuron, metobenzuron, methometon, metolachlor, metribuzin, mepiquat-chloride, mefenacet, mefluidide, monalide, monisouron, monuron, monochloroacetic acid, monolinuron, molinate, morfamquat, iodosulfuron, iodosulfuron-methyl-sodium, iodobonil, iodomethane, lactofen, linuron, rimsulfuron, lenacil, rhodethanil, calcium peroxide and methyl bromide.

Exemplary biopesticides used for the same purposes as above include viral formulations such as nuclear polyhedrosis viruses (NPV), granulosis viruses (GV), cytoplasmic polyhedrosis viruses (CPV) and entomopox viruses (EPV) ; microbial pesticides used as an insecticide or a nematicide, such as *Monacrosporium phymatophagum, Steinernema carpocapsae, Steinernema kushidai* and *Pasteuria penetrans;* microbial pesticides used as a microbicide, such as *Trichoderma lignorum, Agrobacterium radiobactor,* avirulent *Erwinia carotovora* and *Bacillus subtilis;* and biopesticides used as a herbicide, such as *Xanthomonas campestris.* Such a combined use of the agricultural and horticultural insecticide of the present invention with the foregoing biopesticide as a mixture can be expected to provide the same effect as above.

Other examples of the biopesticides include natural predators such as *Encarsia formosa, Aphidius colemani, Aphidoletes aphidimyza, Diglyphus isaea, Dacnusa sibirica, Phytoseiulus persimilis, Amblyseius cucumeris* and *Orius sauteri;* microbial pesticides such as *Beauveria brongniartii;* and pheromones such as (Z)-10-tetradecenyl acetate, (E,Z)-4,10-tetradecadienyl acetate, (Z)-8-dodecenyl acetate, (Z)-11-tetradecenyl acetate, (Z)-13-icosen-10-one and 14-methyl-1-octadecene.

## EXAMPLES

Hereinafter, representative Examples in connection with the present invention are shown, but the present invention is not limited thereto.

### Production Example 1

Production of 2-(1-ethylsulfonylindol-2-yl)-3-methyl-6-pentafluoroethyl-3H-imidazo[4,5-c]pyridazine (compound number 1-33)

[Chem. 19]

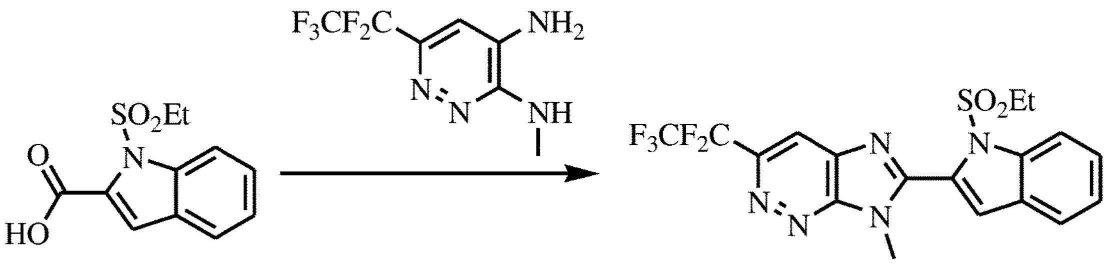

1-Ethylsulfonylindole-2-carboxylic acid (0.32 g, 1.3 mmol) was dissolved in 5 mL of toluene. To this, thionyl chloride (0.19 mL, 2.5 mmol) and three drops of dimethylformamide were added. The mixture was stirred at 100°C for 1.5 hours, and the solvent was evaporated off in vacuo. The residue was dissolved in 3 mL of toluene. To this, 4-amino-3-methylamino-6-pentafluoroethyl pyridazine (0.31 g, 1.3 mmol) synthesized by the method described in the literature (WO 2016/039441) and pyridine (0.20 mL, 2.5 mmol) were added, and the mixture was stirred at 50°C. After the completion of the reaction, the reaction mixture was diluted with ethyl acetate and washed with water and a saturated aqueous sodium chloride solution. The organic layer was dried over anhydrous sodium sulfate, and the solvent was evaporated off. The residue was purified by silica gel column chromatography to give a crude product.

The crude product was dissolved in 5 mL of toluene and 5 mL of acetic acid, and the solution was heated under reflux for 30 minutes. After the completion of the reaction, the reaction mixture was diluted with ethyl acetate and washed with water and a saturated aqueous sodium chloride solution. The organic layer was dried over anhydrous sodium sulfate, and the solvent was evaporated off. The residue was purified by silica gel column chromatography to give the title compound (0.16 g, 0.34 mmol) .

Yield: 27% (overall yield in 2 steps)
Physical property: Melting point 145 to 146°C

Production Example 2

Production of 2-(1-ethylsulfonyl-6-fluoroindol-2-yl)-3-methyl-6-pentafluo roethyl-3H-imidazo[4,5-c]pyridazine (compound number 1-65)

[Chem. 20]

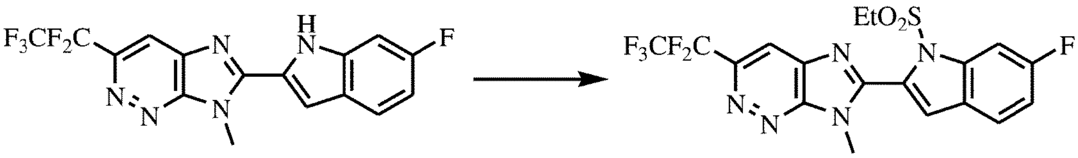

2-(6-Fluoro-1H-indol-2-yl)-3-methyl-6-pentafluoroethyl-3H-imidazo[4,5-c]pyridazine (45.7 mg, 0.119 mmol) was dissolved in 2.0 mL of N,N-dimethylformamide. To this, 60% sodium hydride (7.16 mg, 0.179 mmol) was added under ice-cooling, and the mixture was stirred for 30 minutes. A solution of ethylsulfonyl chloride (30.6 mg, 0.238 mmol) in N,N-dimethylformamide (1.0 mL) was further added dropwise at 0°C, and the mixture was stirred at room temperature for 20 hours . A saturated aqueous ammonium chloride solution was added to the reaction mixture, and extraction with ethyl acetate was performed, followed by washing with water and a saturated aqueous sodium chloride solution. The organic layer was dried over anhydrous sodium sulfate, and the solvent was evaporated off. The residue was purified by silica gel column chromatography to give the title compound (14.5 mg).

Yield: 26%
Physical property: Melting point 82 to 86°C

Production Example 3

Production of 2-(1-ethylsulfonyl-5-trifluoromethylindol-2-yl)-3-methyl-6-trifluoromethyl-3H-imidazo[4,5-b]pyridine (compound number 2-11)

[Chem. 21]

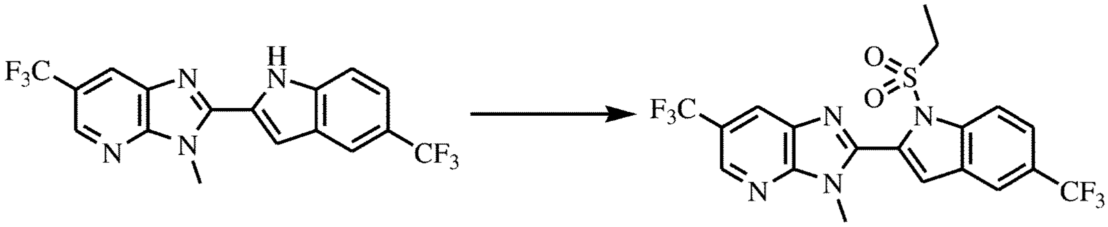

2-(5-Trifluoromethyl-1H-indol-2-yl)-3-methyl-6-trifluor omethyl-3H-imidazo[4,5-b]pyridine (100 mg, 0.260 mmol) was dissolved in 2.6 mL of N,N-dimethylformamide. To this, 60% sodium hydride (20.8 mg, 0.520 mmol) was added under ice-cooling, and the mixture was stirred for 15 minutes. Ethylsulfonyl chloride (73.5 mg, 0.572 mmol) was further added dropwise at 0°C, and the mixture was stirred at room temperature for 15 hours . A saturated aqueous ammonium chloride solution was added to the reaction mixture, and extraction with ethyl acetate was performed, followed by washing with water and a saturated aqueous sodium chloride solution. The organic layer was dried over anhydrous sodium sulfate, and the solvent was evaporated off. The residue was purified by silica gel column chromatography to give the title compound (69.1 mg).

Yield: 50%
Physical property: Melting point 134 to 135°C

Production Example 4

Production of 2-(1-ethylsulfonylindol-2-yl)-5-trifluoromethylthiobenzo[d] oxazole (compound number 3-1)

[Chem. 22]

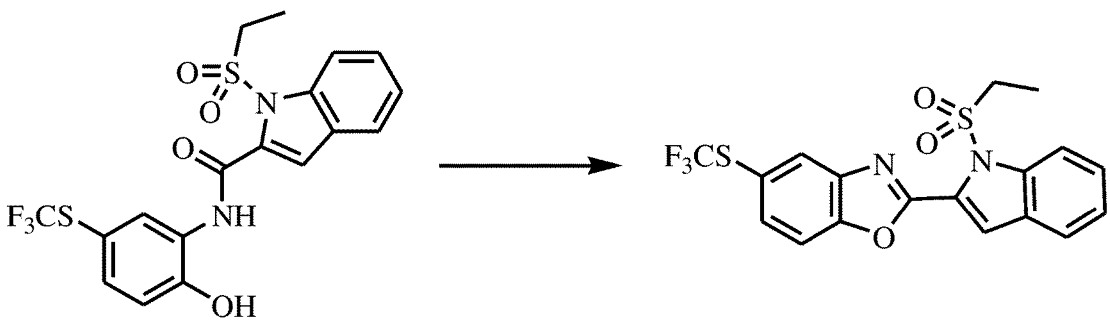

N-(2-Hydroxy-5-trifluoromethylthiophenyl)-1-ethylsulfon ylindole-2-carboxylic amide (114 mg, 0.257 mmol) and triphenylphosphine (101 mg, 0.385 mmol) were dissolved in 2.5 mL of tetrahydrofuran. To this, bis(2-methoxyethyl) azodicarboxylate (0.270 mg, 4.50 mmol) was added at 60°C. The mixture was stirred at the same temperature for 1 hour and then allowed to cool. To the reaction mixture, an aqueous ammonium chloride solution was added. Extraction with ethyl acetate was performed, followed by washing with water and a saturated aqueous sodium chloride solution. The organic layer was dried over anhydrous sodium sulfate, and the solvent was evaporated off. The residue was purified by silica gel column chromatography to give the title compound (22 mg).

Yield: 26%
Physical property: Melting point 118 to 119°C

Reference Example 1

Production of methyl 1-ethylsulfonylindole-2-carboxylate

[Chem. 23]

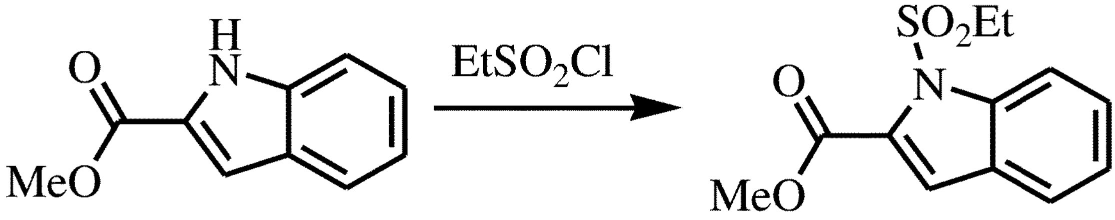


Methyl 1H-indole-2-carboxylate (3.5 g, 20 mmol) synthesized by the method described in the literature (WO 1999/007351) was dissolved in 30 mL of THF. To this, 60% sodium hydride (0.88 g, 22 mmol) was added under ice-cooling, and the mixture was stirred at ordinary temperature for 10 minutes. After ice-cooling again, ethanesulfonyl chloride (3.1 g, 24 mmol) was added dropwise, and the mixture was stirred at ordinary temperature for 15 minutes. Water was added under ice-cooling to stop the reaction. The reaction mixture was diluted with ethyl acetate and washed with water and a saturated aqueous sodium chloride solution. The organic layer was dried over anhydrous sodium sulfate, and the solvent was evaporated off. The residue was purified by silica gel column chromatography to give the title compound (2.4 g, 9.0 mmol).

Yield: 45%
Physical property: Melting point 57 to 58°C

Reference Example 2

Production of 1-ethylsulfonylindole-2-carboxylic acid

[Chem. 24]

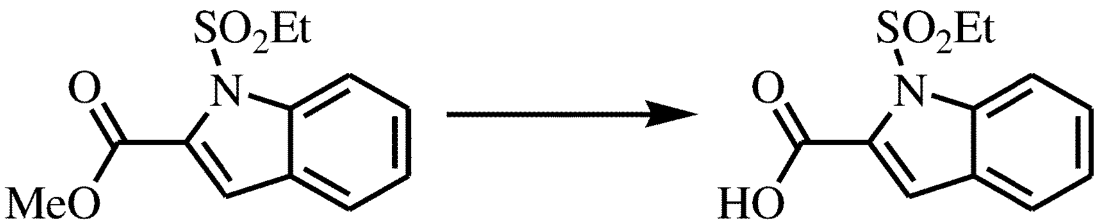

Methyl 1-ethylsulfonylindole-2-carboxylate (1.7 g, 6.2 mmol) was dissolved in 15 mL of THF. To this, a saturated aqueous sodium hydroxide solution containing sodium hydroxide (1.2 g, 30 mmol) was slowly added under ice-cooling, and the mixture was stirred at ordinary temperature. After the completion of the reaction, 2 M hydrochloric acid (15 mL) was added under ice-cooling. Extraction with ethyl acetate was performed, followed by washing with a saturated aqueous sodium chloride solution. The organic layer was dried over anhydrous sodium sulfate, and the solvent was evaporated off to give the title compound (1.5 g, 5.9 mmol) as a crude product.
Physical property: Melting point 136 to 140°C

Reference Example 3

Production of methyl

1-methoxymethyl-6-fluoroindole-2-carboxylate

[Chem. 25]

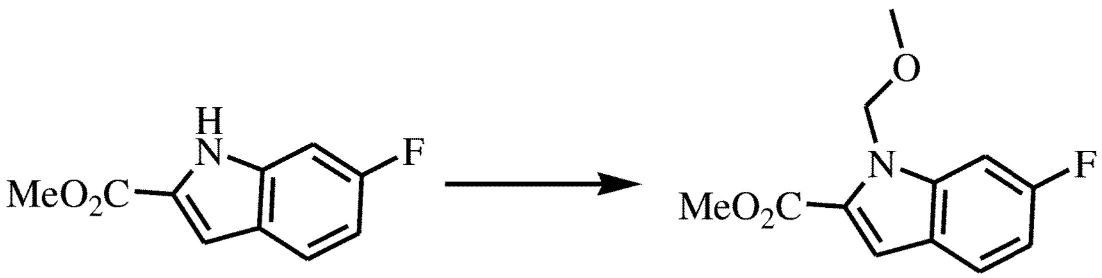

Methyl 6-fluoro-1H-indole-2-carboxylate (316 mg, 1.64 mmol) synthesized according to the method described in the literature (J. O. C., 2001, 66, 3474.) was dissolved in N,N-dimethylformamide (5.5 mL). To this, 60% sodium hydride (72.2 mg, 1.96 mmol) was added under ice-cooling, and the mixture was stirred for 10 minutes. Chloromethyl methyl ether (158 mg, 1.80 mmol) was added dropwise, and the mixture was stirred at room temperature for 1 hour. An aqueous ammonium chloride solution was added under ice-cooling, and extraction with ethyl acetate was performed, followed by washing with water and a saturated aqueous sodium chloride solution. The organic layer was dried over anhydrous sodium sulfate, and the solvent was evaporated off. The residue was purified by silica gel column chromatography to give the title compound (380 mg).

Yield: 98%
Physical property: Melting point 85°C

Reference Example 4

Production of N-(3-methylamino-6-pentafluoroethylpyridazin-4-yl)-1-methox ymethyl-6-fluoroindole-2-carboxylic amide

[Chem. 26]

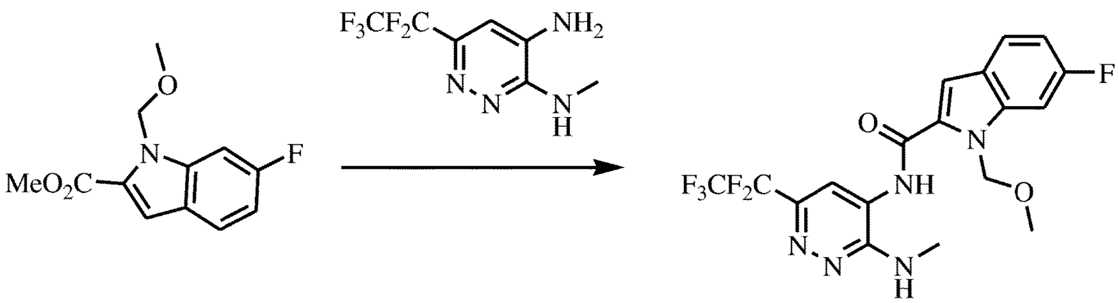

Methyl 1-methoxymethyl-6-fluoroindole-2-carboxylate (357 mg, 1.50 mmol) and 4-amino-3-methylamino-6-pentafluoroethyl pyridazine (413 mg, 1.43 mmol, purity 84%) synthesized by the method described in the literature (WO 2016/039441) were dissolved in 14.3 mL of tetrahydrofuran. To this, 60% sodium hydride (68.6 mg, 1.72 mmol) was added under ice-cooling. The mixture was stirred at the same temperature for 10 minutes, heated to 35°C, and further stirred for 3 hours. An aqueous ammonium chloride solution was added to the reaction mixture under ice-cooling, and extraction with ethyl acetate was performed, followed by washing with water and a saturated aqueous sodium chloride solution. The organic layer was dried over anhydrous sodium sulfate, and the solvent was evaporated off. The residue

was purified by silica gel column chromatography to give the title compound (217 mg).

Reference Example 5

Production of 2-(1-methoxymethyl-6-fluoroindol-2-yl)-3-methyl-6-pentafluo roethyl-3H-imidazo[4,5-c]pyridazine

[Chem. 27]

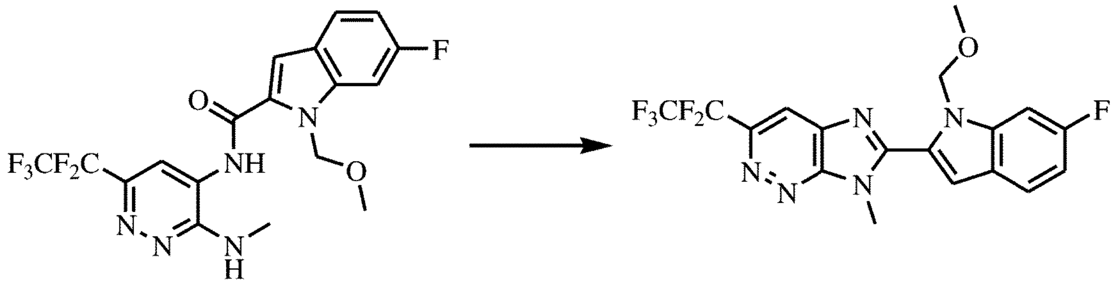

N-(3-Methylamino-6-pentafluoroethylpyridazin-4-yl)-1-me thoxymethyl-6-fluoroindole-2-carboxylic amide (217 mg, 0.485 mmol) was dissolved in 8.0 mL of toluene and 1.5 mL of acetic acid. The solution was heated under reflux for 1.5 hours. The reaction mixture was allowed to cool and concentrated in vacuo. The resulting solid was washed to give the title compound (187 mg) .

Yield: 90%
Physical property: Melting point 165 to 166°C

Reference Example 6

Production of 2-(6-fluoro-1H-indol-2-yl)-3-methyl-6-pentafluoroethyl-3H-i midazo[4,5-c]pyridazine

[Chem. 28]

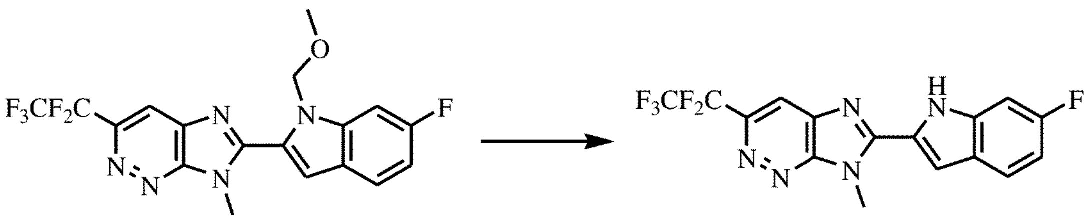

2-(1-Methoxymethyl-6-fluoroindol-2-yl)-3-methyl-6-hepta fluoropropyl-3H-imidazo[4,5-c]pyridazine (165 mg, 0.384 mmol) was dissolved in 2.0 mL of trifluoroacetic acid. The solution was stirred at room temperature for 15 hours. The reaction mixture was concentrated in vacuo and diluted with ethyl acetate . To this, a saturated aqueous sodium bicarbonate solution was added. Extraction with ethyl acetate was performed, followed by washing with water and a saturated aqueous sodium chloride solution. The organic layer was dried over anhydrous sodium sulfate, and the solvent was evaporated off. The residue was purified by silica gel column chromatography to give the title compound (67.7 mg).

Yield: 46%
Physical property: $^1$H-NMR δ 9.62 (s, 1H), 8.06 (s, 1H), 7.71 (dd, 1H), 7.37 (dd, 1H), 7.19 (dd, 1H), 7.01 (ddd, 1H), 4.40 (s, 1H)

Reference Example 7

Production of methyl 1-benzyloxymethyl-5-trifluoromethylindole-2-carboxylate

[Chem. 29]

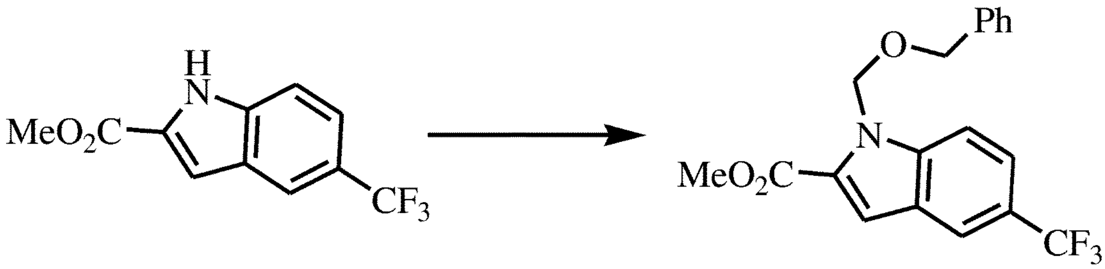

Methyl 5-trifluoromethyl-1H-indole-2-carboxylate (1.30 g, 5.35 mmol) synthesized according to the method described in the literature (WO 2014/073627) was dissolved in 17.8 mL of N,N-dimethylformamide. To this, 60% sodium hydride (0.235 g, 5.89 mmol) was added under ice-cooling, and the mixture was stirred for 5 minutes. Benzyl chloromethyl ether (1.00 g, 6.42 mmol) was added dropwise, and the mixture was stirred at room temperature for 1 hour. An aqueous ammonium chloride solution was added under ice-cooling, and extraction with ethyl acetate was performed, followed by washing with water and a saturated aqueous sodium chloride solution. The organic layer was dried over anhydrous sodium sulfate, and the solvent was evaporated off. The residue was purified by silica gel column chromatography to give the title compound (1.99 g).

Yield: quantitative
Physical property: Refractive index 1.4900 (26.8°C)

Reference Example 8

Production of N-(2-methylamino-5-trifluoromethylpyridin-3-yl)-1-benzyloxy methyl-5-trifluoromethylindole-2-carboxylic amide

[Chem. 30]

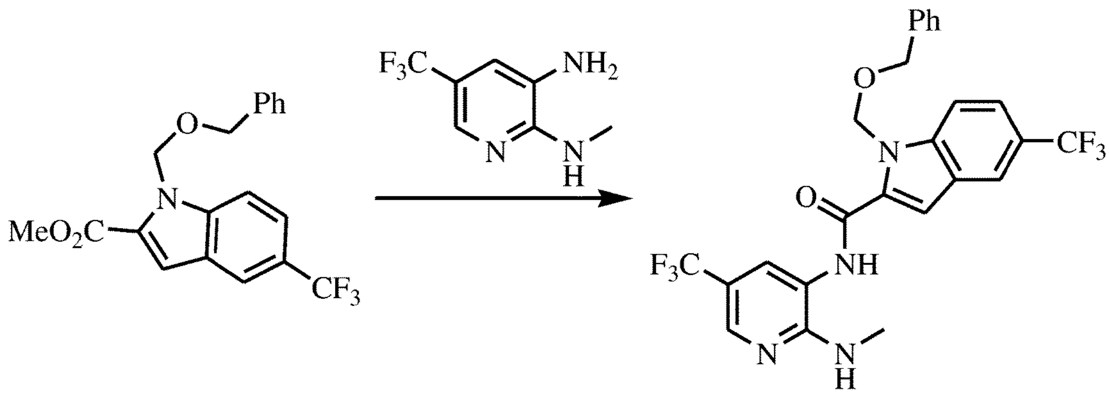

Methyl 1-benzyloxymethyl-5-trifluoromethylindole-2-carboxylate (1.27 g, 3.50 mmol) and 3-amino-2-methylamino-5-trifluoromethylpyridine (0.669 g, 3.50 mmol) were dissolved in 17.5 mL of tetrahydrofuran. To this, 60% sodium hydride (0.168 g, 4.20 mmol) was added under ice-cooling. The mixture was stirred at the same temperature for 15 minutes, heated to 35°C, and further stirred for 1 hour. An aqueous ammonium chloride solution was added to the reaction mixture under ice-cooling, and extraction with ethyl acetate was performed, followed by washing with water and

a saturated aqueous sodium chloride solution. The organic layer was dried over anhydrous sodium sulfate, and the solvent was evaporated off. The residue was purified by silica gel column chromatography to give the title compound (0.95 g).

Reference Example 9

Production of 2-(5-trifluoromethyl-1H-indol-2-yl)-3-methyl-6-trifluoromet hyl-3H-imidazo[4,5-b]pyridine

[Chem. 31]

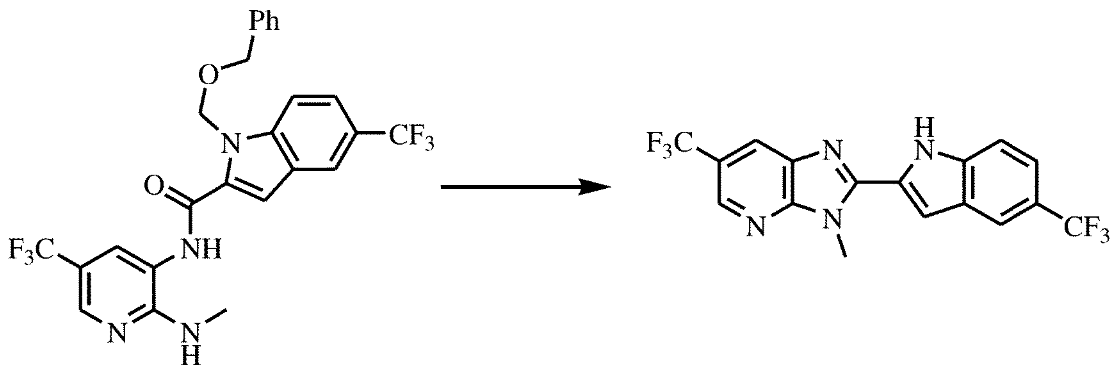

N-(2-Methylamino-5-trifluoromethylpyridin-3-yl)-1-benzy loxymethyl-5-trifluoromethylindole-2-carboxylic amide (0.95 g, 1.82 mmol) was dissolved in 10 mL of N-methyl-2-pyrrolidone. To this, p-toluenesulfonic acid monohydrate (1.04 g, 5.46 mmol) was added, and the mixture was stirred at 170°C for 3 hours. The reaction mixture was allowed to cool. A saturated aqueous sodium bicarbonate solution was added to the reaction mixture, and extraction with ethyl acetate was performed, followed by washing with water and a saturated aqueous sodium chloride solution. The organic layer was dried over anhydrous sodium sulfate, and the solvent was evaporated off. The residue was purified by silica gel column chromatography. The resulting solid was washed with hexane to give the title compound (0.227 g)

Yield: 33%
Physical property: $^1$H-NMR δ 9.83 (s, 1H), 8.69 (d, 1H), 8.24 (d, 1H), 8.05 (s, 1H), 7.57 (s, 2H), 7.29 (d, 1H), 4.26 (s, 3H)

Reference Example 10

Production of N-(2-hydroxy-5-trifluoromethylthiophenyl)-1-ethylsulfonylin dole-2-carboxylic amide

[Chem. 32]

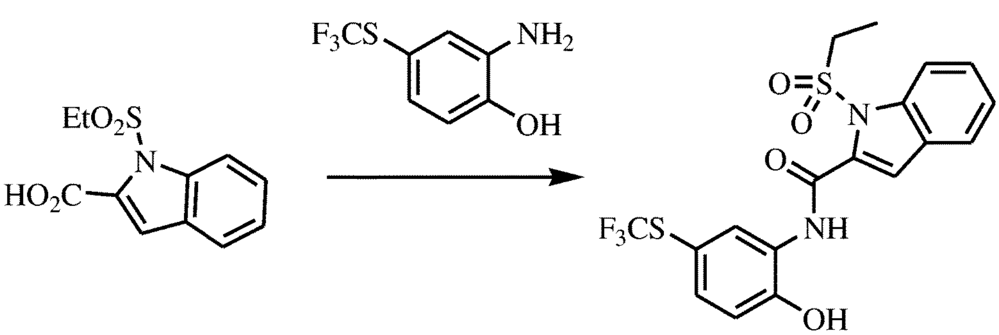

1-Ethylsulfonylindole-2-carboxylic acid (249 mg, 0.98 mmol), 2-hydroxy-5-trifluoromethyl thioaniline (205 mg, 0.98 mmol) and 1-(3-dimethylaminopropyl)-3-ethylcarbodiimide hydrochloride (226 mg, 1.18 mmol) were dissolved in

5.0 mL of pyridine. The solution was stirred at room temperature for 14 hours . Water was added to the reaction mixture, and extraction with ethyl acetate was performed, followed by washing with water and a saturated aqueous sodium chloride solution. The organic layer was dried over anhydrous sodium sulfate, and the solvent was evaporated off. The residue was purified by silica gel column chromatography to give the title compound (114 mg).
Yield: 26%

Hereinafter, formulation examples are shown, but the present invention is not limited thereto. In the formulation examples, "part" means part by weight.

Formulation Example 1

| | |
|---|---|
| Compound of the present invention | 10 parts |
| Xylene | 70 parts |
| N-methylpyrrolidone | 10 parts |
| Mixture of polyoxyethylene nonylphenyl ether and calcium alkylbenzene sulfonate | 10 parts |

The above ingredients are uniformly mixed for dissolution to give an emulsifiable concentrate formulation.

Formulation Example 2

| | |
|---|---|
| Compound of the present invention | 3 parts |
| Clay powder | 82 parts |
| Diatomite powder | 15 parts |

The above ingredients are uniformly mixed and then pulverized to give a dust formulation.

Formulation Example 3

| | |
|---|---|
| Compound of the present invention | 5 parts |
| Mixture of bentonite powder and clay powder | 90 parts |
| Calcium lignosulfonate | 5 parts |

The above ingredients are uniformly mixed. After addition of an appropriate volume of water, the mixture is kneaded, granulated and dried to give a granular formulation.

Formulation Example 4

| | |
|---|---|
| Compound of the present invention | 20 parts |
| Kaolin and synthetic high-dispersion silicic acid | 75 parts |
| Mixture of polyoxyethylene nonylphenyl ether and calcium alkylbenzene sulfonate | 5 parts |

The above ingredients are uniformly mixed and then pulverized to give a wettable powder formulation.

Hereinafter, biological test examples are shown, but the present invention is not limited thereto.

Test Example 1

Test for control efficacy on *Myzus persicae*

Chinese cabbage plants were planted in plastic pots (diameter: 8 cm, height: 8 cm), Green peach aphids (*Myzus persicae*) were propagated on the plants, and the number of surviving Green peach aphids in each pot was counted. The compound represented by the general formula (1) of the present invention or salts thereof were separately dispersed in water and diluted to 500 ppm. The agrochemical dispersions were applied to the foliage of the potted Chinese cabbage plants. After the plants were air-dried, the pots were kept in a greenhouse. At 6 days after the foliar application, the number of surviving Green peach aphids on the Chinese cabbage plant in each pot was counted, the control rate was calculated according to the formula shown below, and the control efficacy was evaluated according to the criteria shown below.

[Math. 1]

$$\text{Control rate} = 100 - \{(T \times Ca)/(Ta \times C)\} \times 100$$

Ta: the number of survivors before the foliar application in a treatment plot
T: the number of survivors after the foliar application in a treatment plot
Ca: the number of survivors before the foliar application in a non-treatment plot
C: the number of survivors after the foliar application in a non-treatment plot

Criteria

A: the control rate is 100%.
B: the control rate is 90 to 99%.
C: the control rate is 80 to 89%.
D: the control rate is 50 to 79%.

As a result, among the compounds represented by the general formula (1) of the present invention, the compounds 1-1, 1-33, 1-35, 1-36, 1-43, 1-63, 1-65, 2-1, 2-3, 2-5, 2-10, 2-11, 2-31, 2-64, 2-65, 2-66, 2-67, 6-1, 6-2, 6-3, 7-1, 8-18 and 8-20 showed excellent insecticidal efficacy on *Myzus persicae,* which was evaluated as A.

Test Example 2

Insecticidal test on *Laodelphax striatellus*

The compounds represented by the general formula (1) of the present invention or salts thereof were separately dispersed in water and diluted to 500 ppm. Rice plant seedlings (variety: Nihonbare) were dipped in the agrochemical dispersions for 30 seconds. After air-dried, each seedling was put into a separate glass test tube and inoculated with ten 3rd-instar larvae of *Laodelphax striatellus,* and then the glass test tubes were capped with cotton plugs. At 8 days after the inoculation, the numbers of surviving larvae and dead larvae were counted, the corrected mortality rate was calculated according to the formula shown below, and the insecticidal efficacy was evaluated according to the criteria shown below.

[Math. 2]

$$\text{Corrected mortality rate (\%)} = 100 \times (\text{Survival rate in a non-treatment plot} - \text{Survival rate in a treatment plot})/\text{Survival rate in a non-treatment plot}$$

Criteria

A: the corrected mortality rate is 100%.
B: the corrected mortality rate is 90 to 99%.
C: the corrected mortality rate is 80 to 89%.
D: the corrected mortality rate is 50 to 79%.

As a result, the compounds 1-33, 1-35, 1-36, 1-43, 1-63, 1-65, 1-66, 2-1, 2-3, 2-5, 2-10, 2-11, 2-31, 2-64, 2-65, 2-67, 6-1, 7-1 and 8-20 of the present invention showed the activity level evaluated as A.

Test Example 3

Insecticidal test on *Plutella xylostella*

Adults of *Plutella xylostella* were released onto Chinese cabbage seedlings and allowed to lay eggs thereon. At 2 days after the release of the adults, the Chinese cabbage seedlings with laid eggs were dipped for about 30 seconds in agrochemical formulations diluted to 500 ppm, each of which contained a different compound represented by the general formula (1) of the present invention as an active ingredient. After air-dried, the seedlings were kept in a thermostatic chamber at 25°C. At 6 days after the dip treatment, the number of hatched larvae per plot was counted, the mortality rate was calculated according to the formula shown below, and the insecticidal efficacy was evaluated according to the criteria of Test Example 2. This test was conducted in triplicate using 10 adults of *Plutella xylostella* per plot.

[Math. 3]

Corrected mortality rate (%)

= 100 × (Number of hatched larvae in a non-treatment plot - Number of hatched larvae in a treatment plot)/Number of hatched larvae in a non-treatment plot

As a result, the compounds 1-1, 1-33, 1-35, 1-36, 1-43, 1-63, 1-65, 1-66, 2-1, 2-3, 2-5, 2-10, 2-11, 2-15, 2-31, 2-64, 2-65, 2-66, 2-67, 3-1, 3-4, 3-11, 6-1, 6-2, 6-3, 7-1, 8-18, 8-19, 8-20, 8-21 and 8-22 of the present invention showed the activity level evaluated as A.

INDUSTRIAL APPLICABILITY

The compound of the present invention or a salt thereof is highly effective as an agricultural and horticultural insecticide.

## Claims

1. A compound represented by the general formula (1):

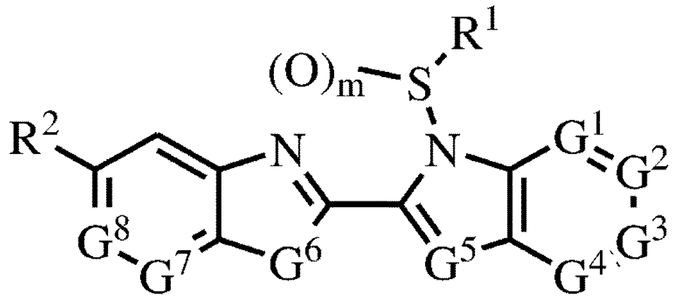

(1)

wherein

$R^1$ represents

(a1) a ($C_1$-$C_6$) alkyl group;
(a2) a ($C_3$-$C_6$) cycloalkyl group;
(a3) a ($C_2$-$C_6$) alkenyl group; or
(a4) a ($C_2$-$C_6$) alkynyl group,

$R^2$ represents

(b1) a halogen atom;
(b2) a cyano group;
(b3) a nitro group;
(b4) a ($C_1$-$C_6$) alkyl group;
(b5) a ($C_1$-$C_6$) alkoxy group;
(b6) a ($C_2$-$C_6$) alkenyloxy group;
(b7) a ($C_2$-$C_6$) alkynyloxy group;
(b8) a halo ($C_1$-$C_6$) alkyl group;
(b9) a halo ($C_1$-$C_6$) alkoxy group;
(b10) a halo ($C_2$-$C_6$) alkenyloxy group;
(b11) a halo ($C_2$-$C_6$) alkynyloxy group;
(b12) a ($C_1$-$C_6$) alkylthio group;
(b13) a ($C_1$-$C_6$) alkylsulfinyl group;
(b14) a ($C_1$-$C_6$) alkylsulfonyl group;
(b15) a halo ($C_1$-$C_6$) alkylthio group;
(b16) a halo ($C_1$-$C_6$) alkylsulfinyl group; or
(b17) a halo ($C_1$-$C_6$) alkylsulfonyl group,

$G^1$, $G^2$, $G^3$ and $G^4$ independently represent C-$R^3$ or N (wherein each $R^3$ independently represents

(c1) a hydrogen atom;
(c2) a halogen atom;
(c3) a hydroxyl group;
(c4) a formyl group;
(c5) a cyano group;
(c6) a ($C_1$-$C_6$) alkyl group;
(c7) a ($C_3$-$C_6$) cycloalkyl group;
(c8) a ($C_1$-$C_6$) alkoxy group;
(c9) a halo ($C_1$-$C_6$) alkyl group;
(c10) a halo ($C_3$-$C_6$) cycloalkyl group;
(c11) a halo ($C_1$-$C_6$) alkoxy group;
(c12) a ($C_1$-$C_6$) alkylthio group;
(c13) a ($C_1$-$C_6$) alkylsulfinyl group;
(c14) a ($C_1$-$C_6$) alkylsulfonyl group;
(c15) a halo ($C_1$-$C_6$) alkylthio group;
(c16) a halo ($C_1$-$C_6$) alkylsulfinyl group;
(c17) a halo ($C_1$-$C_6$) alkylsulfonyl group;
(c18) a ($C_3$-$C_6$) cycloalkyl ($C_1$-$C_6$) alkoxy group;
(c19) an $R^9(R^{10})N$ group (wherein $R^9$ and $R^{10}$ independently represent a hydrogen atom; a ($C_1$-$C_6$) alkyl group; a ($C_3$-$C_6$) cycloalkyl ($C_1$-$C_6$) alkyl group; a halo ($C_1$-$C_6$) alkyl group; a ($C_1$-$C_6$) alkylcarbonyl group; a ($C_1$-$C_6$) alkoxycarbonyl group; or a phenylcarbonyl group);
(c20) a mono ($C_1$-$C_6$) alkylaminocarbonyl NH group;
(c21) a mono ($C_1$-$C_6$) alkylaminothiocarbonyl NH group;
(c22) an $R^{10}ON=C(R^9)$ group (wherein $R^9$ and $R^{10}$ are as defined above) ;
(c23) a carboxyl group;
(c24) a ($C_1$-$C_6$) alkoxycarbonyl group;

(c25) an $R^9(R^{10})N$ carbonyl group (wherein $R^9$ and $R^{10}$ are as defined above);
(c26) an aryl group;
(c27) an aryl group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c28) an aryl ($C_1$-$C_6$) alkyl group;
(c29) an aryl ($C_1$-$C_6$) alkyl group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c30) an aryl ($C_1$-$C_6$) alkoxy group;
(c31) an aryl ($C_1$-$C_6$) alkoxy group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c32) an aryl ($C_1$-$C_6$) alkyl NH group;
(c33) an aryl ($C_1$-$C_6$) alkyl NH group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c34) a heterocyclic group;
(c35) a heterocyclic group having, on the ring, 1 to 3 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group; or
(c36) a ($C_1$-$C_6$) alkoxycarbonyl NH group),

$G^5$ represents C-$R^4$ or N
(wherein
$R^4$ represents

(d1) a hydrogen atom;
(d2) a halogen atom;
(d3) a ($C_1$-$C_6$) alkyl group;
(d4) a ($C_3$-$C_6$) cycloalkyl group;
(d5) a ($C_2$-$C_6$) alkenyl group;
(d6) a ($C_2$-$C_6$) alkynyl group;
(d7) a ($C_1$-$C_6$) alkoxy group;
(d8) a halo ($C_1$-$C_6$) alkyl group;
(d9) a halo ($C_3$-$C_6$) cycloalkyl group;
(d10) a halo ($C_2$-$C_6$) alkenyl group;
(d11) a halo ($C_2$-$C_6$) alkynyl group;
(d12) a halo ($C_1$-$C_6$) alkoxy group;
(d13) a cyano group;
(d14) an $R^9(R^{10})N$ group (wherein $R^9$ and $R^{10}$ are as defined above);
(d15) a ($C_1$-$C_6$) alkylthio group;
(d16) a ($C_1$-$C_6$) alkylsulfinyl group;
(d17) a ($C_1$-$C_6$) alkylsulfonyl group;
(d18) a ($C_1$-$C_6$) alkylthio group;
(d19) a formyl group;
(d20) a ($C_1$-$C_6$) alkoxycarbonyl group;
(d21) an $R^9(R^{10})N$ carbonyl group (wherein $R^9$ and $R^{10}$ are as defined above); or
(d22) an $R^{10}ON=C(R^9)$ group (wherein $R^9$ and $R^{10}$ are as defined above)),

$G^6$ represents N-$R^5$; O; or S (wherein
$R^5$ represents

(e1) a hydrogen atom;
(e2) a ($C_1$-$C_6$) alkyl group;
(e3) a ($C_3$-$C_6$) cycloalkyl group;
(e4) a ($C_2$-$C_6$) alkenyl group; or
(e5) a ($C_2$-$C_6$) alkynyl group),

$G^7$ and $G^8$ independently represent C-H or N, and
m represents an integer of 0 to 2, or

a salt thereof.

**2.** The compound or the salt thereof according to claim 1, wherein

$R^1$ is (a1) a ($C_1$-$C_6$) alkyl group,
$R^2$ is

(b8) a halo ($C_1$-$C_6$) alkyl group;
(b15) a halo ($C_1$-$C_6$) alkylthio group; or
(b17) a halo ($C_1$-$C_6$) alkylsulfonyl group,

each $R^3$ is independently

(c1) a hydrogen atom;
(c2) a halogen atom;
(c4) a formyl group;
(c5) a cyano group;
(c6) a ($C_1$-$C_6$) alkyl group;
(c7) a ($C_3$-$C_6$) cycloalkyl group;
(c8) a ($C_1$-$C_6$) alkoxy group;
(c9) a halo ($C_1$-$C_6$) alkyl group;
(c11) a halo ($C_1$-$C_6$) alkoxy group;
(c12) a ($C_1$-$C_6$) alkylthio group;
(c13) a ($C_1$-$C_6$) alkylsulfinyl group;
(c14) a ($C_1$-$C_6$) alkylsulfonyl group;
(c15) a halo ($C_1$-$C_6$) alkylthio group;
(c19) an $R^9$($R^{10}$)N group (wherein $R^9$ and $R^{10}$ are as defined above);
(c22) an $R^{10}$ON=C($R^9$) group (wherein $R^9$ and $R^{10}$ are as defined above);
(c24) a ($C_1$-$C_6$) alkoxycarbonyl group;
(c25) an $R^9$($R^{10}$)N carbonyl group (wherein $R^9$ and $R^{10}$ are as defined above);
(c27) an aryl group having, on the ring, 1 to 5 substituting groups independently selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group; or
(c36) a ($C_1$-$C_6$) alkoxycarbonyl NH group,

$R^4$ is

(d1) a hydrogen atom;
(d2) a halogen atom;
(d3) a ($C_1$-$C_6$) alkyl group;
(d4) a ($C_3$-$C_6$) cycloalkyl group;
(d7) a ($C_1$-$C_6$) alkoxy group;
(d12) a halo ($C_1$-$C_6$) alkoxy group;
(d13) a cyano group;

(d14) an $R^9(R^{10})N$ group (wherein $R^9$ and $R^{10}$ are as defined above);
(d15) a ($C_1$-$C_6$) alkylthio group;
(d16) a ($C_1$-$C_6$) alkylsulfinyl group;
(d17) a ($C_1$-$C_6$) alkylsulfonyl group;
(d18) a ($C_1$-$C_6$) alkylthio group;
(d19) a formyl group;
(d20) a ($C_1$-$C_6$) alkoxycarbonyl group;
(d21) an $R^9(R^{10})N$ carbonyl group (wherein $R^9$ and $R^{10}$ are as defined above); or
(d22) an $R^{10}ON=C(R^9)$ group (wherein $R^9$ and $R^{10}$ are as defined above),

$G^6$ is N-$R^5$ or O, and
$R^5$ is (e2) a ($C_1$-$C_6$) alkyl group.

**3.** The compound or the salt thereof according to claim 1 or 2, wherein

$R^2$ is

(b8) a halo ($C_1$-$C_6$) alkyl group or
(b15) a halo ($C_1$-$C_6$) alkylthio group,

each $R^3$ is independently

(c1) a hydrogen atom;
(c2) a halogen atom;
(c8) a ($C_1$-$C_6$) alkoxy group;
(c9) a halo ($C_1$-$C_6$) alkyl group; or
(c11) a halo ($C_1$-$C_6$) alkoxy group, and

$R^4$ is

(d1) a hydrogen atom or
(d2) a halogen atom.

**4.** An insecticide comprising the compound or the salt thereof according to any of claims 1 to 3 as an active ingredient.

**5.** An agricultural and horticultural insecticide comprising the compound or the salt thereof according to any of claims 1 to 3 as an active ingredient.

**6.** An animal ectoparasite control agent comprising the compound or the salt according to any of claims 1 to 3 as an active ingredient.

**7.** A method for using an insecticide, comprising treating plants or soil with an effective amount of the insecticide according to claim 4.

**8.** The compound or the salt thereof according to claim 1 wherein

$R^1$ represents

(a1) a ($C_1$-$C_6$) alkyl group;
(a2) a ($C_3$-$C_6$) cycloalkyl group;
(a3) a ($C_2$-$C_6$) alkenyl group; or
(a4) a ($C_2$-$C_6$) alkynyl group,

$R^2$ represents

(b1) a halogen atom;
(b2) a cyano group;
(b3) a nitro group;

(b4) a ($C_1$-$C_6$) alkyl group;
(b5) a ($C_1$-$C_6$) alkoxy group;
(b6) a ($C_2$-$C_6$) alkenyloxy group;
(b7) a ($C_2$-$C_6$) alkynyloxy group;
(b8) a halo ($C_1$-$C_6$) alkyl group;
(b9) a halo ($C_1$-$C_6$) alkoxy group;
(b10) a halo ($C_2$-$C_6$) alkenyloxy group;
(b11) a halo ($C_2$-$C_6$) alkynyloxy group;
(b12) a ($C_1$-$C_6$) alkylthio group;
(b13) a ($C_1$-$C_6$) alkylsulfinyl group;
(b14) a ($C_1$-$C_6$) alkylsulfonyl group;
(b15) a halo ($C_1$-$C_6$) alkylthio group;
(b16) a halo ($C_1$-$C_6$) alkylsulfinyl group; or
(b17) a halo ($C_1$-$C_6$) alkylsulfonyl group,

$G^1$, $G^2$, $G^3$ and $G^4$ may be the same or different and each represent C-$R^3$ or N
(wherein
$R^3$s may be the same or different and represents

(c1) a hydrogen atom;
(c2) a halogen atom;
(c3) a hydroxyl group;
(c4) a formyl group;
(c5) a cyano group;
(c6) a ($C_1$-$C_6$) alkyl group;
(c7) a ($C_3$-$C_6$) cycloalkyl group;
(c8) a ($C_1$-$C_6$) alkoxy group;
(c9) a halo ($C_1$-$C_6$) alkyl group;
(c10) a halo ($C_3$-$C_6$) cycloalkyl group;
(c11) a halo ($C_1$-$C_6$) alkoxy group;
(c12) a ($C_1$-$C_6$) alkylthio group;
(c13) a ($C_1$-$C_6$) alkylsulfinyl group;
(c14) a ($C_1$-$C_6$) alkylsulfonyl group;
(c15) a halo ($C_1$-$C_6$) alkylthio group;
(c16) a halo ($C_1$-$C_6$) alkylsulfinyl group;
(c17) a halo ($C_1$-$C_6$) alkylsulfonyl group;
(c18) a ($C_3$-$C_6$) cycloalkyl ($C_1$-$C_6$) alkoxy group;
(c19) an $R^9$($R^{10}$)N group (wherein $R^9$ and $R^{10}$ may be the same or different and each represent a hydrogen atom; a ($C_1$-$C_6$) alkyl group; a ($C_3$-$C_6$) cycloalkyl ($C_1$-$C_6$) alkyl group; a halo ($C_1$-$C_6$) alkyl group; a ($C_1$-$C_6$) alkylcarbonyl group; a ($C_1$-$C_6$) alkoxycarbonyl group; or a phenylcarbonyl group);
(c20) a mono ($C_1$-$C_6$) alkylaminocarbonyl NH group;
(c21) a mono ($C_1$-$C_6$) alkylaminothiocarbonyl NH group;
(c22) a C($R^9$)=NO$R^{10}$ group (wherein $R^9$ and $R^{10}$ are as defined above);
(c23) a carboxyl group;
(c24) a ($C_1$-$C_6$) alkoxycarbonyl group;
(c25) an $R^9$($R^{10}$)N carbonyl group (wherein $R^9$ and $R^{10}$ are as defined above);
(c26) an aryl group;
(c27) an aryl group having, on the ring, 1 to 5 substituting groups which may be the same or different and are selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c28) an aryl ($C_1$-$C_6$) alkyl group;
(c29) an aryl ($C_1$-$C_6$) alkyl group having, on the ring, 1 to 5 substituting groups which may be the same or different and are selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;

(c30) an aryl ($C_1$-$C_6$) alkoxy group;
(c31) an aryl ($C_1$-$C_6$) alkoxy group having, on the ring, 1 to 5 substituting groups which may be the same or different and are selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c32) an aryl ($C_1$-$C_6$) alkyl NH group;
(c33) an aryl ($C_1$-$C_6$) alkyl NH group having, on the ring, 1 to 5 substituting groups which may be the same or different and are selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group;
(c34) a heterocyclic group; or
(c35) a heterocyclic group having, on the ring, 1 to 3 substituting groups which may be the same or different and are selected from the group consisting of a halogen atom, a ($C_1$-$C_6$) alkyl group, a halo ($C_1$-$C_6$) alkyl group, a ($C_1$-$C_6$) alkoxy group, a halo ($C_1$-$C_6$) alkoxy group, a ($C_1$-$C_6$) alkylthio group, a halo ($C_1$-$C_6$) alkylthio group, a ($C_1$-$C_6$) alkylsulfinyl group, a halo ($C_1$-$C_6$) alkylsulfinyl group, a ($C_1$-$C_6$) alkylsulfonyl group and a halo ($C_1$-$C_6$) alkylsulfonyl group),

$G^5$ represents C-$R^4$ or N
(wherein
$R^4$ represents

(d1) a hydrogen atom;
(d2) a halogen atom;
(d3) a ($C_1$-$C_6$) alkyl group;
(d4) a ($C_3$-$C_6$) cycloalkyl group;
(d5) a ($C_2$-$C_6$) alkenyl group;
(d6) a ($C_2$-$C_6$) alkynyl group;
(d7) a ($C_1$-$C_6$) alkoxy group;
(d8) a halo ($C_1$-$C_6$) alkyl group;
(d9) a halo ($C_3$-$C_6$) cycloalkyl group;
(d10) a halo ($C_2$-$C_6$) alkenyl group;
(d11) a halo ($C_2$-$C_6$) alkynyl group; or
(d12) a halo ($C_1$-$C_6$) alkoxy group),

$G^6$ represents N-$R^5$, O or S
(wherein
$R^5$ represents

(e1) a hydrogen atom;
(e2) a ($C_1$-$C_6$) alkyl group;
(e3) a ($C_3$-$C_6$) cycloalkyl group;
(e4) a ($C_2$-$C_6$) alkenyl group; or
(e5) a ($C_2$-$C_6$) alkynyl group),

$G^7$ and $G^8$ may be the same or different and each represent C-H or N, and
m represents an integer of 0 to 2.

**9.** The compound or the salt thereof according to claim 8, wherein

$R^1$ represents (a1) a ($C_1$-$C_6$) alkyl group,
$R^2$ is

(b8) a halo ($C_1$-$C_6$) alkyl group;
(b15) a halo ($C_1$-$C_6$) alkylthio group; or
(b17) a halo ($C_1$-$C_6$) alkylsulfonyl group,

$G^1$, $G^2$, $G^3$ and $G^4$ each represent C-$R^3$
(wherein
$R^3$s may be the same or different and represents

(c1) a hydrogen atom;
(c2) a halogen atom;
(c4) a formyl group;
(c5) a cyano group;
(c6) a ($C_1$-$C_6$) alkyl group;
(c7) a ($C_3$-$C_6$) cycloalkyl group;
(c8) a ($C_1$-$C_6$) alkoxy group;
(c9) a halo ($C_1$-$C_6$) alkyl group;
(c12) a ($C_1$-$C_6$) alkylthio group;
(c15) a halo ($C_1$-$C_6$) alkylthio group;
(c19) an $R^9$($R^{10}$)N group (wherein $R^9$ and $R^{10}$ may be the same or different and each represent a hydrogen atom; a ($C_1$-$C_6$) alkyl group; a ($C_3$-$C_6$) cycloalkyl ($C_1$-$C_6$) alkyl group; a halo ($C_1$-$C_6$) alkyl group; a ($C_1$-$C_6$) alkylcarbonyl group; a ($C_1$-$C_6$) alkoxycarbonyl group; or a phenylcarbonyl group);
(c22) a C($R^9$)=NOR$^{10}$ group (wherein $R^9$ and $R^{10}$ are as defined above);
(c24) a ($C_1$-$C_6$) alkoxycarbonyl group; or
(c25) an $R^9$ ($R^{10}$) N carbonyl group (wherein $R^9$ and $R^{10}$ are as defined above)),

$G^5$ represents C-$R^4$ (wherein $R^4$ represents (d1) a hydrogen atom), and
$G^6$ represents N-$R^5$, O or S (wherein $R^5$ represents (e2) a ($C_1$-$C_6$) alkyl group) .

**10.** The compound or the salt thereof according to claim 8 or 9, wherein

$R^2$ is

(b8) a halo ($C_1$-$C_6$) alkyl group or
(b15) a halo ($C_1$-$C_6$) alkylthio group,

$G^1$, $G^2$, $G^3$ and $G^4$ each represent C-$R^3$
(wherein
$R^3$s may be the same or different and represents

(c1) a hydrogen atom;
(c2) a halogen atom; or
(c9) a halo ($C_1$-$C_6$) alkyl group), and

$G^6$ represents N-$R^5$ or O (wherein $R^5$ represents (e2) a ($C_1$-$C_6$) alkyl group) .

**11.** An agricultural and horticultural insecticide comprising the compound or the salt thereof according to any of claims 8 to 10 as an active ingredient.

**12.** A method for using an agricultural and horticultural insecticide, comprising treating plants or soil with an effective amount of the agricultural and horticultural insecticide according to claim 11.

**Patentansprüche**

**1.** Verbindung, repräsentiert durch die allgemeine Formel (1)

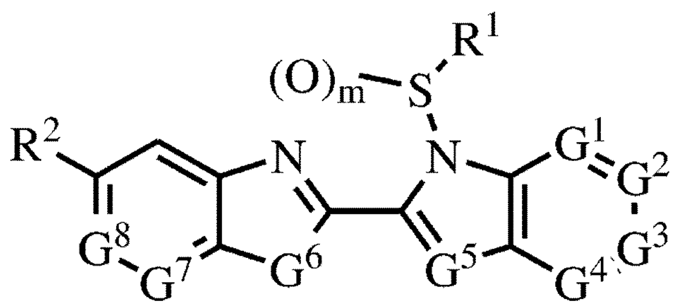

(1)

wobei

$R^1$ steht für

(a1) eine ($C_1$-$C_6$) Alkylgruppe;
(a2) eine ($C_3$-$C_6$) Cycloalkylgruppe;
(a3) eine ($C_2$-$C_6$) Alkenylgruppe; oder
(a4) eine ($C_2$-$C_6$) Alkinylgruppe,

$R^2$ steht für

(b1) ein Halogenatom;
(b2) eine Cyanogruppe;
(b3) eine Nitrogruppe;
(b4) eine ($C_1$-$C_6$) Alkylgruppe;
(b5) eine ($C_1$-$C_6$) Alkoxygruppe;
(b6) eine ($C_2$-$C_6$) Alkenyloxygruppe;
(b7) eine ($C_2$-$C_6$) Alkinyloxygruppe;
(b8) eine Halogen ($C_1$-$C_6$) Alkylgruppe;
(b9) eine Halogen ($C_1$-$C_6$) Alkoxygruppe;
(b10) eine Halogen ($C_2$-$C_6$) Alkenyloxygruppe;
(b11) eine Halogen ($C_2$-$C_6$) Alkinyloxygruppe;
(b12) eine ($C_1$-$C_6$) Alkylthiogruppe;
(b13) eine ($C_1$-$C_6$) Alkylsulfinylgruppe;
(b14) eine (C1-C6) Alkylsulfonylgruppe;
(b15) eine Halogen ($C_1$-$C_6$) Alkylthiogruppe;
(b16) eine Halogen ($C_1$-$C_6$) Alkylsulfinylgruppe; oder
(b17) eine Halogen ($C_1$-$C_6$) Alkylsulfonylgruppe,

$G^1$, $G^2$, $G^3$ und $G^4$ stehen unabhängig voneinander für C-$R^3$ oder N
(wobei
jedes $R^3$ steht unabhängig voneinander für

(c1) ein Wasserstoffatom;
(c2) ein Halogenatom;
(c3) eine Hydroxylgruppe;
(c4) eine Formylgruppe;
(c5) eine Cyanogruppe;
(c6) eine ($C_1$-$C_6$) Alkylgruppe;
(c7) eine ($C_3$-$C_6$) Cycloalkylgruppe;
(c8) eine ($C_1$-$C_6$) Alkoxygruppe;
(c9) eine Halogen ($C_1$-$C_6$) Alkylgruppe;
(c10) eine Halogen ($C_3$-$C_6$) Cycloalkylgruppe;
(c11) eine Halogen ($C_1$-$C_6$) Alkoxygruppe;
(c12) eine ($C_1$-$C_6$) Alkylthiogruppe;
(c13) eine ($C_1$-$C_6$) Alkylsulfinylgruppe;
(c14) eine ($C_1$-$C_6$) Alkylsulfonylgruppe;

(c15) eine Halogen ($C_1$-$C_6$) Alkylthiogruppe;
(c16) eine Halogen ($C_1$-$C_6$) Alkylsulfinylgruppe;
(c17) eine Halogen ($C_1$-$C_6$) Alkylsulfonylgruppe;
(c18) eine ($C_3$-$C_6$) Cycloalkyl ($C_1$-$C_6$) Alkoxygruppe;
(c19) eine $R^9(R^{10})$N-Gruppe (wobei $R^9$ und $R^{10}$ unabhängig voneinander stehen für ein Wasserstoffatom; eine ($C_1$-$C_6$) Alkylgruppe; eine ($C_3$-$C_6$) Cycloalkyl ($C_1$-$C_6$) Alkylgruppe; eine Halogen ($C_1$-$C_6$) Alkylgruppe; eine ($C_1$-$C_6$) Alkylcarbonylgruppe; eine ($C_1$-$C_6$) Alkoxycarbonylgruppe; oder eine Phenylcarbonylgruppe);
(c20) eine Mono ($C_1$-$C_6$) Alkylaminocarbonyl-NH-Gruppe;
(c21) eine Mono ($C_1$-$C_6$) Alkylaminothiocarbonyl-NH-Gruppe;
(c22) eine $R^{10}ON=C(R^9)$-Gruppe (wobei $R^9$ und $R^{10}$ wie oben definiert sind);
(c23) eine Carboxylgruppe;
(c24) eine ($C^1$-$C^6$) Alkoxycarbonylgruppe;
(c25) eine $R^9(R^{10})$N-Carbonylgruppe (wobei $R^9$ und $R^{10}$ wie oben definiert sind);
(c26) eine Arylgruppe;
(c27) eine Arylgruppe, die am Ring 1 bis 5 Substitutionsgruppen aufweist, die unabhängig voneinander ausgewählt sind aus der Gruppe bestehend aus einem Halogenatom, einer ($C_1$-$C_6$) Alkylgruppe, einer Halogen ($C_1$-$C_6$) Alkylgruppe, einer ($C_1$-$C_6$) Alkoxygruppe, einer Halogen ($C_1$-$C_6$) Alkoxygruppe, einer ($C_1$-$C_6$) Alkylthiogruppe, einer Halogen ($C_1$-$C_6$) Alkylthiogruppe, einer ($C_1$-$C_6$) Alkylsulfinylgruppe, einer Halogen ($C_1$-$C_6$) Alkylsulfinylgruppe, einer ($C_1$-$C_6$) Alkylsulfonylgruppe und einer Halogen ($C_1$-$C_6$) Alkylsulfonylgruppe;
(c28) eine Aryl ($C_1$-$C_6$) Alkylgruppe;
(c29) eine Aryl (C1-C6) Alkylgruppe, die am Ring 1 bis 5 Substitutionsgruppen aufweist, die unabhängig voneinander ausgewählt sind aus der Gruppe bestehend aus einem Halogenatom, einer ($C_1$-$C_6$) Alkylgruppe, einer Halogen ($C_1$-$C_6$) Alkylgruppe, einer ($C_1$-$C_6$) Alkoxygruppe einer Halogen ($C_1$-$C_6$) Alkoxygruppe, einer ($C_1$-$C_6$) Alkylthiogruppe, einer Halogen ($C_1$-$C_6$) Alkylthiogruppe, einer ($C_1$-$C_6$) Alkylsulfinylgruppe, einer Halogen ($C_1$-$C_6$) Alkylsulfinylgruppe, einer ($C_1$-$C_6$) Alkylsulfonylgruppe und einer Halogen ($C_1$-$C_6$) Alkylsulfonylgruppe;
(c30) eine Aryl ($C_1$-$C_6$) Alkoxygruppe;
(c31) eine Aryl ($C_1$-$C_6$) Alkoxygruppe, die am Ring 1 bis 5 Substitutionsgruppen aufweist, die unabhängig voneinander ausgewählt sind aus der Gruppe bestehend aus einem Halogenatom, einer ($C_1$-$C_6$) Alkylgruppe, einer Halogen ($C_1$-$C_6$) Alkylgruppe, einer ($C_1$-$C_6$) Alkoxygruppe einer Halogen ($C_1$-$C_6$) Alkoxygruppe, einer ($C_1$-$C_6$) Alkylthiogruppe, einer Halogen ($C_1$-$C_6$) Alkylthiogruppe, einer ($C_1$-$C_6$) Alkylsulfinylgruppe, einer Halogen-($C_1$-$C_6$) Alkylsulfinylgruppe, einer ($C_1$-$C_6$) Alkylsulfonylgruppe und einer Halogen ($C_1$-$C_6$) Alkylsulfonylgruppe;
(c32) eine Aryl ($C_1$-$C_6$) Alkyl-NH-Gruppe;
(c33) eine Aryl ($C_1$-$C_6$)-Alkyl-NH-Gruppe, die am Ring 1 bis 5 Substitutionsgruppen aufweist, die unabhängig voneinander ausgewählt sind aus der Gruppe bestehend aus einem Halogenatom, einer ($C_1$-$C_6$) Alkylgruppe, einer Halogen ($C_1$-$C_6$) Alkylgruppe, einer ($C_1$-$C_6$) Alkoxygruppe einer Halogen ($C_1$-$C_6$) Alkoxygruppe, einer ($C_1$-$C_6$) Alkylthiogruppe, einer Halogen ($C_1$-$C_6$) Alkylthiogruppe, einer ($C_1$-$C_6$) Alkylsulfinylgruppe, einer Halogen ($C_1$-$C_6$) Alkylsulfinylgruppe, einer ($C_1$-$C_6$) Alkylsulfonylgruppe und einer Halogen ($C_1$-$C_6$)-Alkylsulfonylgruppe;
(c34) eine heterocyclische Gruppe;
(c35) eine heterocyclische Gruppe, die am Ring 1 bis 3 Substituentengruppen aufweist, die unabhängig voneinander ausgewählt sind aus der Gruppe bestehend aus einem Halogenatom, einer ($C_1$-$C_6$) Alkylgruppe, einer Halogen ($C_1$-$C_6$) Alkylgruppe, einer ($C_1$-$C_6$) Alkoxygruppe, einer Halogen ($C_1$-$C_6$) Alkoxygruppe, einer ($C_1$-$C_6$) Alkylthiogruppe, einer Halogen ($C_1$-$C_6$) Alkylthiogruppe, einer ($C_1$-$C_6$) Alkylsulfinylgruppe, einer Halogen-($C_1$-$C_6$) Alkylsulfinylgruppe, einer ($C_1$-$C_6$) Alkylsulfonylgruppe und einer Halogen ($C_1$-$C_6$)-Alkylsulfonylgruppe; oder
(c36) eine ($C_1$-$C_6$)-Alkoxycarbonyl-NH-Gruppe),

$G^5$ steht für C-$R^4$ oder N
(wobei
$R^4$ steht für

(d1) ein Wasserstoffatom;
(d2) ein Halogenatom;
(d3) eine ($C_1$-$C_6$) Alkylgruppe;
(d4) eine ($C_3$-$C_6$) Cycloalkylgruppe;

(d5) eine ($C_2$-$C_6$) Alkenylgruppe;
(d6) eine ($C_2$-$C_6$) Alkinylgruppe;
(d7) eine ($C_1$-$C_6$) Alkoxygruppe;
(d8) eine Halogen ($C_1$-$C_6$) Alkylgruppe;
(d9) eine Halogen ($C_3$-$C_6$) cycloalkylgruppe;
(d10) eine Halogen ($C_2$-$C_6$) Alkenylgruppe;
(d11) eine Halogen ($C_2$-$C_6$) Alkinylgruppe;
(d12) eine Halogen ($C_1$-$C_6$) Alkoxygruppe;
(d13) eine Cyanogruppe;
(d14) eine $R^9(R^{10})N$-Gruppe (wobei $R^9$ und $R^{10}$ wie oben definiert sind);
(d15) eine ($C_1$-$C_6$) Alkylthiogruppe;
(d16) eine ($C_1$-$C_6$) Alkylsulfinylgruppe;
(d17) eine ($C_1$-$C_6$) Alkylsulfonylgruppe;
(d18) eine ($C_1$-$C_6$) Alkylthiogruppe;
(d19) eine Formylgruppe;
(d20) eine ($C_1$-$C_6$) Alkoxycarbonylgruppe;
(d21) eine $R^9(R^{10})N$-Carbonylgruppe (wobei $R^9$ und $R^{10}$ wie oben definiert sind); oder
(d22) eine $R^{10}ON{=}C(R^9)$-Gruppe (wobei $R^9$ und $R^{10}$ wie oben definiert sind)),

$G^6$ steht für N-$R^5$; O; oder S
(wobei
$R^5$ steht für

(e1) ein Wasserstoffatom;
(e2) eine ($C_1$-$C_6$) Alkylgruppe;
(e3) eine ($C_3$-$C_6$) Cycloalkylgruppe;
(e4) eine ($C_2$-$C_6$) Alkenylgruppe; oder
(e5) eine ($C_2$-$C_6$) Alkinylgruppe),

$G^7$ und $G^8$ stehen unabhängig voneinander für C-H oder N, und
m stellt eine ganze Zahl von 0 bis 2 dar, oder

ein Salz davon.

**2.** Verbindung oder das Salz davon nach Anspruch 1, wobei

$R^1$ ist (a1) eine ($C_1$-$C_6$)-Alkylgruppe,
$R^2$ ist

(b8) eine Halogen ($C_1$-$C_6$) Alkylgruppe;
(b15) eine Halogen ($C_1$-$C_6$) Alkylthiogruppe; oder
(b17) eine Halogen ($C_1$-$C_6$) Alkylsulfonylgruppe,

jedes $R^3$ ist unabhängig

(c1) ein Wasserstoffatom;
(c2) ein Halogenatom;
(c4) eine Formylgruppe;
(c5) eine Cyanogruppe;
(c6) eine ($C_1$-$C_6$) Alkylgruppe;
(c7) eine ($C_3$-$C_6$) Cycloalkylgruppe;
(c8) eine ($C_1$-$C_6$) Alkoxygruppe;
(c9) eine Halogen ($C_1$-$C_6$) Alkylgruppe;
(c11) eine Halogen ($C_1$-$C_6$) Alkoxygruppe;
(c12) eine ($C_1$-$C_6$) Alkylthiogruppe;
(c13) eine ($C_1$-$C_6$) Alkylsulfinylgruppe;
(c14) eine ($C_1$-$C_6$) Alkylsulfonylgruppe;
(c15) eine Halogen ($C_1$-$C_6$) Alkylthiogruppe;

(c19) eine $R_9(R^{10})N$-Gruppe (wobei $R^9$ und $R^{10}$ wie oben definiert sind);
(c22) eine $R^{10}ON{=}C(R^9)$-Gruppe (wobei $R^9$ und $R^{10}$ wie oben definiert sind);
(c24) eine ($C_1$-$C_6$) Alkoxycarbonylgruppe;
(c25) eine $R^9(R^{10})N$-Carbonylgruppe (wobei $R^9$ und $R^{10}$ wie oben definiert sind);
(c27) eine Arylgruppe mit 1 bis 5 Substitutionsgruppen am Ring, die unabhängig voneinander aus der Gruppe ausgewählt sind bestehend aus einem Halogenatom, einer ($C_1$-$C_6$) Alkylgruppe, einer Halogen ($C_1$-$C_6$) Alkylgruppe, einer ($C_1$-$C_6$) Alkoxygruppe einer Halogen ($C_1$-$C_6$) Alkoxygruppe, einer ($C_1$-$C_6$) Alkylthiogruppe, einer Halogen ($C_1$-$C_6$) Alkylthiogruppe, einer ($C_1$-$C_6$) Alkylsulfinylgruppe, einer Halogen ($C_1$-$C_6$) Alkylsulfinylgruppe, einer ($C_1$-$C_6$) Alkylsulfonylgruppe und einer Halogen (C1-$C_6$) Alkylsulfonylgruppe; oder (c36) eine ($C_1$-$C_6$) Alkoxycarbonyl-NH-Gruppe,

$R^4$ ist

(d1) ein Wasserstoffatom;
(d2) ein Halogenatom;
(d3) eine ($C_1$-$C_6$) Alkylgruppe;
(d4) eine ($C_3$-$C_6$) Cycloalkylgruppe;
(d7) eine ($C_1$-$C_6$) Alkoxygruppe;
(d12) eine Halogen ($C_1$-$C_6$) Alkoxygruppe;
(d13) eine Cyanogruppe;
(d14) eine $R^9(R^{10})N$-Gruppe (wobei $R^9$ und $R^{10}$ wie oben definiert sind);
(d15) eine ($C_1$-$C_6$) Alkylthiogruppe;
(d16) eine ($C_1$-$C_6$) Alkylsulfinylgruppe;
(d17) eine ($C_1$-$C_6$) Alkylsulfonylgruppe;
(d18) eine ($C_1$-$C_6$) Alkylthiogruppe;
(d19) eine Formylgruppe;
(d20) eine ($C_1$-$C_6$) Alkoxycarbonylgruppe;
(d21) eine $R^9(R^{10})N$-Carbonylgruppe (wobei $R^9$ und $R^{10}$ wie oben definiert sind); oder
(d22) eine $R^{10}ON{=}C(R^9)$-Gruppe (wobei $R^9$ und $R^{10}$ wie oben definiert sind),

$G^6$ ist N-$R^5$ oder O, und
$R^5$ ist (e2) eine ($C_1$-$C_6$)-Alkylgruppe.

**3.** Die Verbindung oder das Salz davon nach Anspruch 1 oder 2, wobei

$R^2$ ist

(b8) eine Halogen ($C_1$-$C_6$) Alkylgruppe oder
(b15) eine Halogen ($C_1$-$C_6$) Alkylthiogruppe ist,

jedes $R^3$ ist unabhängig voneinander

(c1) ein Wasserstoffatom;
(c2) ein Halogenatom;
(c8) eine ($C_1$-$C_6$) Alkoxygruppe;
(c9) eine Halogen ($C_1$-$C_6$) Alkylgruppe; oder
(c11) eine Halogen ($C_1$-$C_6$) Alkoxygruppe, und

$R^4$ ist

(d1) ein Wasserstoffatom oder
(d2) ein Halogenatom.

**4.** Insektizid, das die Verbindung oder das Salz davon nach einem der Ansprüche 1 bis 3 als Wirkstoff aufweist.

**5.** Insektizid für Landwirtschaft und Gartenbau, das die Verbindung oder das Salz davon nach einem der Ansprüche 1 bis 3 als Wirkstoff aufweist.

6. Mittel zur Bekämpfung von Ektoparasiten bei Tieren, das die Verbindung oder das Salz nach einem der Ansprüche 1 bis 3 als Wirkstoff aufweist.

7. Verfahren zur Verwendung eines Insektizids, das die Behandlung von Pflanzen oder Boden mit einer wirksamen Menge des Insektizids nach Anspruch 4 aufweist.

8. Die Verbindung oder das Salz davon nach Anspruch 1 wobei

$R^1$ steht für

(a1) eine ($C_1$-$C_6$) Alkylgruppe;
(a2) eine ($C_3$-$C_6$) Cycloalkylgruppe;
(a3) eine ($C_2$-$C_6$) Alkenylgruppe; oder
(a4) eine ($C_2$-$C_6$) Alkinylgruppe,

$R^2$ steht für

(b1) ein Halogenatom;
(b2) eine Cyanogruppe;
(b3) eine Nitrogruppe;
(b4) eine ($C_1$-$C_6$) Alkylgruppe;
(b5) eine ($C_1$-$C_6$) Alkoxygruppe;
(b6) eine ($C_2$-$C_6$) Alkenyloxygruppe;
(b7) eine ($C_2$-$C_6$) Alkinyloxygruppe;
(b8) eine Halogen ($C_1$-$C_6$) Alkylgruppe;
(b9) eine Halogen ($C_1$-$C_6$) Alkoxygruppe;
(b10) eine Halogen ($C_2$-$C_6$) Alkenyloxygruppe;
(b11) eine Halogen ($C_2$-$C_6$) Alkinyloxygruppe;
(b12) eine ($C_1$-$C_6$) Alkylthiogruppe;
(b13) eine ($C_1$-$C_6$) Alkylsulfinylgruppe;
(b14) eine ($C_1$-$C_6$) Alkylsulfonylgruppe;
(b15) eine Halogen ($C_1$-$C_6$) Alkylthiogruppe;
(b16) eine Halogen ($C_1$-$C_6$)Alkylsulfinylgruppe; oder
(b17) eine Halogen ($C_1$-$C_6$)Alkylsulfonylgruppe,

$G^1$, $G^2$, $G^3$ und $G^4$ können gleich oder verschieden sein und stellen jeweils C-$R^3$ oder N dar (wobei
$R^3$s gleich oder verschieden sein kann und steht für

(c1) ein Wasserstoffatom;
(c2) ein Halogenatom;
(c3) eine Hydroxylgruppe;
(c4) eine Formylgruppe;
(c5) eine Cyanogruppe;
(c6) eine ($C_1$-$C_6$) Alkylgruppe;
(c7) eine ($C_3$-$C_6$) Cycloalkylgruppe;
(c8) eine ($C_1$-$C_6$) Alkoxygruppe;
(c9) eine Halogen ($C_1$-$C_6$) Alkylgruppe;
(c10) eine Halogen ($C_3$-$C_6$) Cycloalkylgruppe;
(c11) eine Halogen ($C_1$-$C_6$) Alkoxygruppe;
(c12) eine ($C_1$-$C_6$) Alkylthiogruppe;
(c13) eine ($C_1$-$C_6$) Alkylsulfinylgruppe;
(c14) eine ($C_1$-$C_6$) Alkylsulfonylgruppe;
(c15) eine Halogen ($C_1$-$C_6$) Alkylthiogruppe;
(c16) eine Halogen ($C_1$-$C_6$) Alkylsulfinylgruppe;
(c17) eine Halogen ($C_1$-$C_6$) Alkylsulfonylgruppe;
(c18) eine ($C_3$-$C_6$) Cycloalkyl ($C_1$-$C_6$) Alkoxygruppe;

(c19) eine $R^9(R^{10})$N-Gruppe (wobei $R^9$ und $R^{10}$ gleich oder verschieden sein können und jeweils stehen für ein Wasserstoffatom; eine ($C_1$-$C_6$) Alkylgruppe; eine ($C_3$-$C_6$) Cycloalkyl ($C_1$-$C_6$) Alkylgruppe; eine Halogen ($C_1$-$C_6$) Alkylgruppe; eine ($C_1$-$C_6$) Alkylcarbonylgruppe; eine ($C_1$-$C_6$) Alkoxycarbonylgruppe; oder eine Phenylcarbonylgruppe darstellen);
(c20) eine Mono ($C_1$-$C_6$) Alkylaminocarbonyl-NH-Gruppe;
(c21) eine Mono ($C_1$-$C_6$) Alkylaminothiocarbonyl-NH-Gruppe;
(c22) eine $C(R^9)=NOR^{10}$-Gruppe (worin $R^9$ und $R^{10}$ wie oben definiert sind);
(c23) eine Carboxylgruppe;
(c24) eine ($C_1$-$C_6$) Alkoxycarbonylgruppe;
(c25) eine $R_9(R_{10})$N-Carbonylgruppe (worin $R^9$ und $R^{10}$ wie oben definiert sind);
(c26) eine Arylgruppe;
(c27) eine Arylgruppe mit 1 bis 5 Substitutionsgruppen am Ring, die gleich oder verschieden sein können und ausgewählt sind aus der Gruppe bestehend aus einem Halogenatom, einer ($C_1$-$C_6$) Alkylgruppe, einer Halogen ($C_1$-$C_6$) Alkylgruppe, einer ($C_1$-$C_6$) Alkoxygruppe einer Halogen ($C_1$-$C_6$) Alkoxygruppe, einer ($C_1$-$C_6$) Alkylthiogruppe, einer Halogen ($C_1$-$C_6$) Alkylthiogruppe, einer ($C_1$-$C_6$) Alkylsulfinylgruppe, einer Halogen ($C_1$-$C_6$) Alkylsulfinylgruppe, einer ($C_1$-$C_6$) Alkylsulfonylgruppe und einer Halogen ($C_1$-$C_6$) Alkylsulfonylgruppe;
(c28) eine Aryl ($C_1$-$C_6$) Alkylgruppe;
(c29) einer Aryl ($C_1$-$C_6$) Alkylgruppe mit 1 bis 5 Substitutionsgruppen am Ring, die gleich oder verschieden sein können und ausgewählt sind aus der Gruppe bestehend aus einem Halogenatom, einer ($C_1$-$C_6$) Alkylgruppe, einer Halogen ($C_1$-$C_6$) Alkylgruppe einer ($C_1$-$C_6$) Alkoxygruppe, einer Halogen ($C_1$-$C_6$) Alkoxygruppe, einer ($C_1$-$C_6$) Alkylthiogruppe, einer Halogen-($C_1$-$C_6$) Alkylthiogruppe, einer ($C_1$-$C_6$) Alkylsulfinylgruppe, einer Halogen ($C_1$-$C_6$) Alkylsulfinylgruppe, einer ($C_1$-$C_6$) Alkylsulfonylgruppe und einer Halogen ($C_1$-$C_6$) Alkylsulfonylgruppe;
(c30) eine Aryl ($C_1$-$C_6$) Alkoxygruppe;
(c31) einer Aryl-($C_1$-$C_6$) Alkoxygruppe mit 1 bis 5 Substitutionsgruppen am Ring, die gleich oder verschieden sein können und ausgewählt sind aus der Gruppe bestehend aus einem Halogenatom, einer ($C_1$-$C_6$) Alkylgruppe, einer Halogen ($C_1$-$C_6$) Alkylgruppe einer ($C_1$-$C_6$) Alkoxygruppe, einer Halogen ($C_1$-$C_6$) Alkoxygruppe, einer ($C_1$-$C_6$) Alkylthiogruppe, einer Halogen ($C_1$-$C_6$) Alkylthiogruppe, einer ($C_1$-$C_6$) Alkylsulfinylgruppe, einer Halogen ($C_1$-$C_6$) Alkylsulfinylgruppe, einer ($C_1$-$C_6$) Alkylsulfonylgruppe und einer Halogen ($C_1$-$C_6$) Alkylsulfonylgruppe;
(c32) eine Aryl ($C_1$-$C_6$) Alkyl-NH-Gruppe;
(c33) einer Aryl ($C_1$-$C_6$) Alkyl-NH-Gruppe mit 1 bis 5 Substitutionsgruppen am Ring, die gleich oder verschieden sein können und ausgewählt sind aus der Gruppe bestehend aus einem Halogenatom, einer ($C_1$-$C_6$) Alkylgruppe, einer Halogen ($C_1$-$C_6$) Alkylgruppe, einer ($C_1$-$C_6$) Alkoxygruppe, einer Halogen ($C_1$-$C_6$) Alkoxygruppe, einer ($C_1$-$C_6$) Alkylthiogruppe, einer Halogen ($C_1$-$C_6$) Alkylthiogruppe, einer ($C_1$-$C_6$) Alkylsulfinylgruppe, einer Halogen ($C_1$-$C_6$) Alkylsulfinylgruppe, einer ($C_1$-$C_6$) Alkylsulfonylgruppe und einer Halogen ($C_1$-$C_6$) Alkylsulfonylgruppe;
(c34) eine heterocyclische Gruppe; oder
(c35) eine heterocyclische Gruppe, die am Ring 1 bis 3 Substitutionsgruppen aufweist, die gleich oder verschieden sein können und ausgewählt sind aus der Gruppe bestehend aus einem Halogenatom, einer ($C_1$-$C_6$) Alkylgruppe, einer Halogen ($C_1$-$C_6$) Alkylgruppe, einer ($C_1$-$C_6$) Alkoxygruppe einer Halogen ($C_1$-$C_6$) Alkoxygruppe, einer ($C_1$-$C_6$) Alkylthiogruppe, einer Halogen ($C_1$-$C_6$) Alkylthiogruppe, einer ($C_1$-$C_6$) Alkylsulfinylgruppe, einer Halogen ($C_1$-$C_6$) Alkylsulfinylgruppe, einer ($C_1$-$C_6$) Alkylsulfonylgruppe und einer Halogen ($C_1$-$C_6$) Alkylsulfonylgruppe),

$G^5$ steht für C-$R^4$ oder N
(wobei
$R^4$ steht für

(d1) ein Wasserstoffatom;
(d2) ein Halogenatom;
(d3) eine ($C_1$-$C_6$) Alkylgruppe;
(d4) eine ($C_3$-$C_6$) Cycloalkylgruppe;
(d5) eine ($C_2$-$C_6$) Alkenylgruppe;
(d6) eine ($C_2$-$C_6$) Alkinylgruppe;
(d7) eine ($C_1$-$C_6$) Alkoxygruppe;
(d8) eine Halogen ($C_1$-$C_6$) Alkylgruppe;

(d9) eine Halogen ($C_3$-$C_6$) Cycloalkylgruppe;
(d10) eine Halogen ($C_2$-$C_6$) Alkenylgruppe;
(d11) eine Halogen ($C_2$-$C_6$) Alkinylgruppe; oder
(d12) eine Halogen ($C_1$-$C_6$) Alkoxygruppe),

$G^6$ steht für N-$R^5$, O oder S
(wobei
$R^5$ steht für

(e1) ein Wasserstoffatom;
(e2) eine ($C_1$-$C_6$) Alkylgruppe;
(e3) eine ($C_3$-$C_6$) Cycloalkylgruppe;
(e4) eine ($C_2$-$C_6$) Alkenylgruppe; oder
(e5) eine ($C_2$-$C_6$) Alkinylgruppe),

$G^7$ und $G^8$ können gleich oder verschieden sein und stehen jeweils für C-H oder N, und
m steht für eine ganze Zahl von 0 bis 2.

**9.** Die Verbindung oder das Salz davon nach Anspruch 8, wobei

$R^1$ (a1) steht für eine ($C_1$-$C_6$) Alkylgruppe,
$R^2$ ist

(b8) eine Halogen ($C_1$-$C_6$) Alkylgruppe;
(b15) eine Halogen ($C_1$-$C_6$) Alkylthiogruppe; oder
(b17) eine Halogen ($C_1$-$C_6$) Alkylsulfonylgruppe,

$G^1$, $G^2$, $G^3$ und $G^4$ stehen jeweils für C-$R^3$
(wobei
$R^3$s können gleich oder verschieden sein und stehen für

(c1) ein Wasserstoffatom;
(c2) ein Halogenatom;
(c4) eine Formylgruppe;
(c5) eine Cyanogruppe;
(c6) eine ($C_1$-$C_6$) Alkylgruppe;
(c7) eine ($C_3$-$C_6$) Cycloalkylgruppe;
(c8) eine ($C_1$-$C_6$) Alkoxygruppe;
(c9) eine Halogen ($C_1$-$C_6$) Alkylgruppe;
(c12) eine ($C_1$-$C_6$) Alkylthiogruppe;
(c15) eine Halogen ($C_1$-$C_6$) Alkylthiogruppe;
(c19) eine $R^9$($R^{10}$)N-Gruppe (worin $R^9$ und $R^{10}$ gleich oder verschieden sein können und jeweils ein Wasserstoffatom; eine ($C_1$-$C_6$) Alkylgruppe; eine ($C_3$-$C_6$) Cycloalkyl ($C_1$-$C_6$) Alkylgruppe; eine Halogen ($C_1$-$C_6$) Alkylgruppe; eine ($C_1$-$C_6$) Alkylcarbonylgruppe; eine ($C_1$-$C_6$) Alkoxycarbonylgruppe; oder eine Phenylcarbonylgruppe darstellen);
(c22) eine C($R^9$)=NOR$^{10}$-Gruppe (worin $R^9$ und $R^{10}$ wie oben definiert sind);
(c24) eine ($C_1$-$C_6$) Alkoxycarbonylgruppe; oder
(c25) eine $R^9$($R^{10}$)N-Carbonylgruppe (worin $R^9$ und $R^{10}$ wie oben definiert sind)),

$G^5$ steht für C-$R^4$ (worin $R^4$ (d1) für ein Wasserstoffatom steht), und
$G^6$ für N-$R^5$, O oder S steht (worin $R^5$ für (e2) eine ($C_1$-$C_6$) Alkylgruppe steht).

**10.** Verbindung oder das Salz davon nach Anspruch 8 oder 9, wobei

$R^2$ ist

(b8) eine Halogen ($C_1$-$C_6$) Alkylgruppe oder
(b15) eine Halogen ($C_1$-$C_6$) Alkylthiogruppe,

$G^1$, $G^2$, $G^3$ und $G^4$ stehen jeweils für C-$R^3$
(wobei
$R^3$s können gleich oder verschieden sein und bedeuten

(c1) ein Wasserstoffatom;
(c2) ein Halogenatom; oder
(c9) eine Halo ($C_1$-$C_6$) Alkylgruppe), und

$G^6$ steht für N-$R^5$ oder O (wobei $R^5$ steht für (e2) eine ($C_1$-$C_6$) Alkylgruppe).

**11.** Insektizid für Landwirtschaft und Gartenbau, das die Verbindung oder das Salz davon nach einem der Ansprüche 8 bis 10 als Wirkstoff enthält.

**12.** Verfahren zur Verwendung eines Insektizids für Landwirtschaft und Gartenbau, aufweisend die Behandlung von Pflanzen oder Boden mit einer wirksamen Menge des Insektizids für Landwirtschaft und Gartenbau gemäß Anspruch 11.

## Revendications

**1.** Composé représenté par la formule générale (1) :

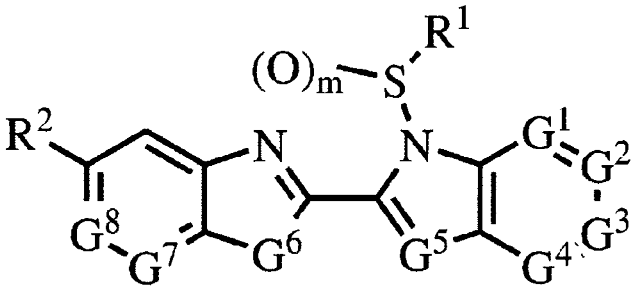

(1)

dans laquelle

$R^1$ représente

(a1) un groupe alkyle en ($C_1$-$C_6$) ;
(a2) un groupe cycloalkyle en ($C_3$-$C_6$) ;
(a3) un groupe alcényle en ($C_2$-$C_6$) ; ou
(a4) un groupe alcynyle en ($C_2$-$C_6$),

$R^2$ représente

(b1) un atome d'halogène ;
(b2) un groupe cyano ;
(b3) un groupe nitro ;
(b4) un groupe alkyle en ($C_1$-$C_6$) ;
(b5) un groupe alcoxy en ($C_1$-$C_6$) ;
(b6) un groupe alcényloxy en ($C_2$-$C_6$) ;
(b7) un groupe alcynyloxy en ($C_2$-$C_6$) ;
(b8) un groupe halogénure d'alkyle en ($C_1$-$C_6$) ;
(b9) un groupe halogénoalcoxy en ($C_1$-$C_6$) ;
(b10) un groupe halogénoalcényloxy en ($C_2$-$C_6$) ;
(b11) un groupe halogénoalcynyloxy en ($C_2$-$C_6$) ;
(b12) un groupe alkylthio en ($C_1$-$C_6$) ;
(b13) un groupe alkylsulfinyle en ($C_1$-$C_6$) ;

(b14) groupe alkylsulfonyle en ($C_1$-$C_6$) ;
(b15) un groupe halogénoalkylthio en ($C_1$-$C_6$) ;
(b16) un groupe halogénoalkylsulfinyle en ($C_1$-$C_6$) ; ou
(b17) un groupe halogénoalkylsulfonyle en ($C_1$-$C_6$),

$G^1$, $G^2$, $G^3$ et $G^4$ représentent indépendamment C-$R^3$ ou N (où chaque $R^3$ représente indépendamment

(c1) un atome d'hydrogène ;
(c2) un atome d'halogène ;
(c3) un groupe hydroxyle ;
(c4) un groupe formyle ;
(c5) un groupe cyano ;
(c6) un groupe alkyle en ($C_1$-$C_6$) ;
(c7) un groupe cycloalkyle en ($C_3$-$C_6$) ;
(c8) un groupe alcoxy en ($C_1$-$C_6$) ;
(c9) un groupe halogénure d'alkyle en ($C_1$-$C_6$) ;
(c10) un groupe halogénocycloalkyle en ($C_3$-$C_6$) ;
(c11) un groupe halogénoalcoxy en ($C_1$-$C_6$) ;
(c12) un groupe alkylthio en ($C_1$-$C_6$) ;
(c13) un groupe alkylsulfinyle en ($C_1$-$C_6$) ;
(c14) un groupe alkylsulfonyle en ($C_1$-$C_6$) ;
(c15) un groupe halogénoalkylthio en ($C_1$-$C_6$) ;
(c16) un groupe halogénoalkylsulfinyle en ($C_1$-$C_6$) ;
(c17) un groupe halogénoalkylsulfonyle en ($C_1$-$C_6$) ;
(c18) un groupe cycloalkyle en ($C_3$-$C_6$)-alcoxy en ($C_1$-$C_6$) ;
(c19) un groupe $R^9(R^{10})N$ (où $R^9$ et $R^{10}$ représentent indépendamment un atome d'hydrogène ; un groupe alkyle en ($C_1$-$C_6$) ; un groupe cycloalkyle en ($C_3$-$C_6$)-alkyle en ($C_1$-$C_6$) ; un groupe halogénure d'alkyle en ($C_1$-$C_6$), un groupe alkylcarbonyle en ($C_1$-$C_6$), un groupe alcoxycarbonyle en ($C_1$-$C_6$) ; ou un groupe phénylcarbonyle) ;
(c20) un groupe mono NH-alkylaminocarbonyle en ($C_1$-$C_6$) ;
(c21) un groupe mono NH-alkylaminothiocarbonyle en ($C_1$-$C_6$) ;
(c22) un groupe $R^{10}ON=C(R^9)$ (où $R^9$ et $R^{10}$ sont tels que définis ci-dessus) ;
(c23) un groupe carboxyle ;
(c24) un groupe alcoxycarbonyle en ($C_1$-$C_6$) ;
(c25) un groupe $R^9(R^{10})N$ carbonyle (où $R^9$ et $R^{10}$ sont tels que définis ci-dessus) ;
(c26) un groupe aryle ;
(c27) un groupe aryle ayant, sur le cycle, 1 à 5 groupes substituants choisis indépendamment dans le groupe constitué par un atome d'halogène, un groupe alkyle en ($C_1$-$C_6$), un groupe halogénure d'alkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$) ;
(c28) un groupe aryl-alkyle en ($C_1$-$C_6$) ;
(c29) un groupe aryl-alkyle en ($C_1$-$C_6$) ayant, sur le cycle, 1 à 5 groupes substituants choisis indépendamment dans le groupe constitué par un atome d'halogène, un groupe alkyle en ($C_1$-$C_6$), un groupe halogénure d'alkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$) ;
(c30) un groupe aryl-alcoxy en ($C_1$-$C_6$) ;
(c31) un groupe aryl-alcoxy en ($C_1$-$C_6$) ayant, sur le cycle, 1-5 groupes substituants choisis indépendamment dans le groupe constitué par un atome d'halogène, un groupe alkyle en ($C_1$-$C_6$), un groupe halogénure d'alkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$) ;
(c32) un groupe NH-alkyle-aryl en ($C_1$-$C_6$);
(c33) un groupe NH-alkyle-aryl en ($C_1$-$C_6$) ayant, sur le cycle, 1 à 5 groupes substituants choisis indépendamment dans le groupe constitué par un atome d'halogène, un groupe alkyle en ($C_1$-$C_6$), un groupe halogénure d'alkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$) ;
(c34) un groupe hétérocyclique ;
(c35) un groupe hétérocyclique ayant, sur le cycle, 1 à 3 groupes substituants choisis indépendamment dans le groupe constitué par un atome d'halogène, un groupe alkyle en ($C_1$-$C_6$), un groupe halogénoalkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$) ; ou
(c36) un groupe NH-alcoxycarbonyle en ($C_1$-$C_6$)),

$G^5$ représente C-$R^4$ ou N (où
$R^4$ représente

(d1) un atome d'hydrogène ;
(d2) un atome d'halogène ;
(d3) un groupe alkyle en ($C_1$-$C_6$) ;
(d4) un groupe cycloalkyle en ($C_3$-$C_6$) ;
(d5) un groupe alcényle en ($C_2$-$C_6$) ;
(d6) un groupe alcynyle en ($C_2$-$C_6$) ;
(d7) un groupe alcoxy en ($C_1$-$C_6$) ;
(d8) un groupe halogénure d'alkyle en ($C_1$-$C_6$) ;
(d9) un groupe halogénocycloalkyle en ($C_3$-$C_6$) ;
(d10) un groupe halogénoalcényle en ($C_2$-$C_6$) ;
(d11) un groupe halogénoalcynyle en ($C_2$-$C_6$) ;
(d12) un groupe halogénoalcoxy en ($C_1$-$C_6$) ;
(d13) un groupe cyano ;
(d14) un groupe $R^9(R^{10})N$ (où $R^9$ et $R^{10}$ sont tels que définis ci-dessus) ;
(d15) un groupe alkylthio en ($C_1$-$C_6$) ;
(d16) un groupe alkylsulfinyle en ($C_1$-$C_6$) ;
(d17) un groupe alkylsulfonyle en ($C_1$-$C_6$) ;
(d18) un groupe alkylthio en ($C_1$-$C_6$) ;
(d19) un groupe formyle ;
(d20) un groupe alcoxycarbonyle en ($C_1$-$C_6$) ;
(d21) un groupe $R^9(R^{10})N$ carbonyle (où $R^9$ et $R^{10}$ sont tels que définis ci-dessus) ; ou
(d22) un groupe $R^{10}ON=C(R^9)$ (où $R^9$ et $R^{10}$ sont tels que définis ci-dessus)),

$G^6$ représente N-$R^5$ ; O ; ou S
(où
$R^5$ représente

(e1) un atome d'hydrogène ;
(e2) un groupe alkyle en ($C_1$-$C_6$) ;
(e3) un groupe cycloalkyle en ($C_3$-$C_6$) ;
(e4) un groupe alcényle en ($C_2$-$C_6$) ; ou
(e5) un groupe alcynyle en ($C_2$-$C_6$)),

$G^7$ et $G^8$ représentent indépendamment C-H ou N, et

m représente un nombre entier compris entre 0 et 2, ou
un sel de celui-ci.

**2.** Composé ou son sel selon la revendication 1, dans lequel

$R^1$ est (a1) un groupe alkyle en ($C_1$-$C_6$),

$R^2$ est

(b8) un groupe halogénure d'alkyle en ($C_1$-$C_6$) ;
(b15) un groupe halogénoalkylthio en ($C_1$-$C_6$) ; ou
(b17) un groupe halogénoalkylsulfonyle en ($C_1$-$C_6$),

chaque $R^3$ est indépendamment

(c1) un atome d'hydrogène ;
(c2) un atome d'halogène ;
(c4) un groupe formyle ;
(c5) un groupe cyano ;
(c6) un groupe alkyle en ($C_1$-$C_6$) ;
(c7) un groupe cycloalkyle en ($C_3$-$C_6$) ;
(c8) un groupe alcoxy en ($C_1$-$C_6$) ;
(c9) un groupe halogénoalkyle en ($C_1$-$C_6$) ;
(c11) un groupe halogénoalcoxy en ($C_1$-$C_6$) ;
(c12) un groupe alkylthio en ($C_1$-$C_6$) ;
(c13) un groupe alkylsulfinyle en ($C_1$-$C_6$) ;
(c14) un groupe alkylsulfonyle en ($C_1$-$C_6$) ;
(c15) un groupe halogénoalkylthio en ($C_1$-$C_6$) ;
(c19) un groupe $R^9(R^{10})N$ (où $R^9$ et $R^{10}$ sont tels que définis ci-dessus) ;
(c22) un groupe $R^{10}ON=C(R^9)$ (où $R^9$ et $R^{10}$ sont tels que définis ci-dessus) ;
(c24) un groupe alcoxycarbonyle en ($C_1$-$C_6$) ;
(c25) un groupe $R^9(R^{10})N$ carbonyle (où $R^9$ et $R^{10}$ sont tels que définis ci-dessus) ;
(c27) un groupe aryle ayant, sur le cycle, 1-5 groupes substituants choisis indépendamment dans le groupe constitué par un atome d'halogène, un groupe alkyle en ($C_1$-$C_6$), un groupe halogénure d'alkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$) ; ou
(c36) un groupe NH-alcoxycarbonyle en ($C_1$-$C_6$),

$R^3$ 4 est

(d1) un atome d'hydrogène ;
(d2) un atome d'halogène ;
(d3) un groupe alkyle en ($C_1$-$C_6$) ;
(d4) un groupe cycloalkyle en ($C_3$-$C_6$) ;
(d7) un groupe alcoxy en ($C_1$-$C_6$) ;
(d12) un groupe halogénoalcoxy en ($C_1$-$C_6$) ;
(d13) un groupe cyano ;
(d14) un groupe $R^9(R^{10})N$ (où $R^9$ et $R^{10}$ sont tels que définis ci-dessus) ;
(d15) un groupe alkylthio en ($C_1$-$C_6$) ;
(d16) un groupe alkylsulfinyle en ($C_1$-$C_6$) ;
(d17) un groupe alkylsulfonyle en ($C_1$-$C_6$) ;
(d18) un groupe alkylthio en ($C_1$-$C_6$) ;
(d19) un groupe formyle ;
(d20) un groupe alcoxycarbonyle en ($C_1$-$C_6$) ;
(d21) un groupe $R^9(R^{10})N$ carbonyle (où $R^9$ et $R^{10}$ sont tels que définis ci-dessus) ; ou
(d22) un groupe $R^{10}ON=C(R^9)$ (où $R^9$ et $R^{10}$ sont tels que définis ci-dessus),

$G^6$ est N-$R^5$ ou O, et
$R^5$ est (e2) un groupe alkyle en ($C_1$-$C_6$).

**3.** Composé ou son sel selon la revendication 1 ou 2, dans lequel

$R^2$ est

(b8) un groupe halogénoalkyle en ($C_1$-$C_6$), ou
(b15) un groupe halogénoalkylthio en ($C_1$-$C_6$),

chaque $R^3$ est indépendamment

(c1) un atome d'hydrogène ;
(c2) un atome d'halogène ;
(c8) un groupe alcoxy en ($C_1$-$C_6$) ;
(c9) un groupe halogénoalkyle en ($C_1$-$C_6$) ; ou
(c11) un groupe halogénoalcoxy en ($C_1$-$C_6$), et

$R^4$ est

(d1) un atome d'hydrogène, ou
(d2) un atome d'halogène.

**4.** Insecticide comprenant le composé ou son sel selon l'une des revendications 1 à 3 en tant qu'ingrédient actif.

**5.** Insecticide agricole et horticole comprenant le composé ou son sel selon l'une des revendications 1 à 3 en tant qu'ingrédient actif.

**6.** Agent de lutte contre les ectoparasites d'animaux comprenant le composé ou son sel selon l'une des revendications 1 à 3 en tant qu'ingrédient actif.

**7.** Procédé d'utilisation d'un insecticide, comprenant le traitement des plantes ou du sol avec une quantité efficace de l'insecticide selon la revendication 4.

**8.** Composé ou son sel selon la revendication 1, dans lequel

$R^1$ représente

(a1) un groupe alkyle en ($C_1$-$C_6$) ;
(a2) un groupe cycloalkyle en ($C_3$-$C_6$) ;
(a3) un groupe alcényle en ($C_2$-$C_6$) ; ou
(a4) un groupe alcynyle en ($C_2$-$C_6$),

$R^2$ représente

(b1) un atome d'halogène ;
(b2) un groupe cyano ;
(b3) un groupe nitro ;
(b4) un groupe alkyle en ($C_1$-$C_6$) ;
(b5) un groupe alcoxy en ($C_1$-$C_6$) ;
(b6) un groupe alcényloxy en ($C_2$-$C_6$) ;
(b7) un groupe alcynyloxy en ($C_2$-$C_6$) ;
(b8) un groupe halogénure d'alkyle en ($C_1$-$C_6$) ;
(b9) un groupe halogénoalcoxy en ($C_1$-$C_6$) ;
(b10) un groupe halogénoalcényloxy en ($C_2$-$C_6$) ;
(b11) un groupe halogénoalcynyloxy en ($C_2$-$C_6$) ;
(b12) un groupe alkylthio en ($C_1$-$C_6$) ;
(b13) un groupe alkylsulfinyle en ($C_1$-$C_6$) ;
(b14) groupe alkylsulfonyle en ($C_1$-$C_6$) ;
(b15) un groupe halogénoalkylthio en ($C_1$-$C_6$) ;
(b16) un groupe halogénoalkylsulfinyle en ($C_1$-$C_6$) ; ou
(b17) un groupe halogénoalkylsulfonyle en ($C_1$-$C_6$),

$G^1$, $G^2$, $G^3$ et $G^4$ peuvent être identiques ou différents et représentent chacun C-$R^3$ ou N (où

$R^3$ peut être identique ou différent et représente

(c1) un atome d'hydrogène ;
(c2) un atome d'halogène ;
(c3) un groupe hydroxyle ;
(c4) un groupe formyle ;
(c5) un groupe cyano ;
(c6) un groupe alkyle en ($C_1$-$C_6$) ;
(c7) un groupe cycloalkyle en ($C_3$-$C_6$) ;
(c8) un groupe alcoxy en ($C_1$-$C_6$) ;
(c9) un groupe halogénure d'alkyle en ($C_1$-$C_6$) ;
(c10) un groupe halogénocycloalkyle en ($C_3$-$C_6$) ;
(c11) un groupe halogénoalcoxy en ($C_1$-$C_6$) ;
(c12) un groupe alkylthio en ($C_1$-$C_6$) ;
(c13) un groupe alkylsulfinyle en ($C_1$-$C_6$) ;
(c14) un groupe alkylsulfonyle en ($C_1$-$C_6$) ;
(c15) un groupe halogénoalkylthio en ($C_1$-$C_6$) ;
(c16) un groupe halogénoalkylsulfinyle en ($C_1$-$C_6$) ;
(c17) un groupe halogénoalkylsulfonyle en ($C_1$-$C_6$) ;
(c18) un groupe cycloalkyle en ($C_3$-$C_6$)-alcoxy en ($C_1$-$C_6$) ;
(c19) un groupe $R^9(R^{10})N$ (où $R^9$ et $R^{10}$ peuvent être identiques ou différents et représentent chacun un atome d'hydrogène ; un groupe alkyle en ($C_1$-$C_6$) ; un groupe cycloalkyle en ($C_3$-$C_6$)-alkyle en ($C_1$-$C_6$) ; un groupe halogénoalkyle en ($C_1$-$C_6$), un groupe alkylcarbonyle en ($C_1$-$C_6$), un groupe alcoxycarbonyle en ($C_1$-$C_6$) ; ou un groupe phénylcarbonyle) ;
(c20) un groupe mono NH-alkylaminocarbonyle en ($C_1$-$C_6$) ;
(c21) un groupe mono NH-alkylaminothiocarbonyle en ($C_1$-$C_6$) ;
(c22) un groupe $C(R^9)=NOR^{10}$ (où $R^9$ et $R^{10}$ sont tels que définis ci-dessus) ;
(c23) un groupe carboxyle ;
(c24) un groupe alcoxycarbonyle en ($C_1$-$C_6$) ;
(c25) un groupe $R^9(R^{10})N$ carbonyle (où $R^9$ et $R^{10}$ sont tels que définis ci-dessus) ;
(c26) un groupe aryle ;
(c27) un groupe aryle ayant, sur le cycle, 1-5 groupes substituants qui peuvent être identiques ou différents et qui sont choisis dans le groupe constitué par un atome d'halogène, un groupe alkyle en ($C_1$-$C_6$), un groupe halogénure d'alkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$) ;
(c28) un groupe aryl-alkyle en ($C_1$-$C_6$) ;
(c29) un groupe aryl-alkyle en ($C_1$-$C_6$) ayant, sur le cycle, 1-5 groupes substituants qui peuvent être identiques ou différents et qui sont choisis dans le groupe constitué par un atome d'halogène, un groupe alkyle en ($C_1$-$C_6$), un groupe halogénure d'alkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$) ;
(c30) un groupe aryl-alcoxy en ($C_1$-$C_6$) ;
(c31) un groupe aryl-alcoxy en ($C_1$-$C_6$) ayant, sur le cycle, 1-5 groupes substituants qui peuvent être identiques ou différents et qui sont choisis dans le groupe constitué par un atome d'halogène, un groupe alkyle en ($C_1$-$C_6$), un groupe halogénure d'alkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$) ;
(c32) un groupe NH-alkyle-aryl en ($C_1$-$C_6$) ;
(c33) un groupe NH-alkyle-aryl en ($C_1$-$C_6$) ayant, sur le cycle, 1-5 groupes substituants qui peuvent être identiques ou différents et qui sont choisis dans le groupe constitué par un atome d'halogène, un groupe alkyle en ($C_1$-$C_6$), un groupe halogénure d'alkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$) ;

(c34) un groupe hétérocyclique ;

(c35) un groupe hétérocyclique ayant, sur le cycle, 1-3 groupes substituants qui peuvent être identiques ou différents et qui sont choisis dans le groupe constitué par un atome d'halogène, un groupe alkyle en ($C_1$-$C_6$), un groupe halogénoalkyle en ($C_1$-$C_6$), un groupe alcoxy en ($C_1$-$C_6$), un groupe halogénoalcoxy en ($C_1$-$C_6$), un groupe alkylthio en ($C_1$-$C_6$), un groupe halogénoalkylthio en ($C_1$-$C_6$), un groupe alkylsulfinyle en ($C_1$-$C_6$), un groupe halogénoalkylsulfinyle en ($C_1$-$C_6$), un groupe alkylsulfonyle en ($C_1$-$C_6$) et un groupe halogénoalkylsulfonyle en ($C_1$-$C_6$),

$G^5$ représente C-$R^4$ ou N
(où
$R^4$ représente

(d1) un atome d'hydrogène ;
(d2) un atome d'halogène ;
(d3) un groupe alkyle en ($C_1$-$C_6$) ;
(d4) un groupe cycloalkyle en ($C_3$-$C_6$) ;
(d5) un groupe alcényle en ($C_2$-$C_6$) ;
(d6) un groupe alcynyle en ($C_2$-$C_6$) ;
(d7) un groupe alcoxy en ($C_1$-$C_6$) ;
(d8) un groupe halogénure d'alkyle en ($C_1$-$C_6$) ;
(d9) un groupe halogénocycloalkyle en ($C_3$-$C_6$) ;
(d10) un groupe halogénoalcényle en ($C_2$-$C_6$) ;
(d11) un groupe halogénoalcynyle en ($C_2$-$C_6$) ;
(d12) un groupe halogénoalcoxy en ($C_1$-$C_6$) ;

$G^6$ représente N-$R^5$, O ou S
(où
$R^5$ représente

(e1) un atome d'hydrogène ;
(e2) un groupe alkyle en ($C_1$-$C_6$) ;
(e3) un groupe cycloalkyle en ($C_3$-$C_6$) ;
(e4) un groupe alcényle en ($C_2$-$C_6$) ; ou
(e5) un groupe alcynyle en ($C_2$-$C_6$)),

$G^7$ et $G^8$ peuvent être identiques ou différents et représentent chacun C-H ou N, et
m représente un nombre entier compris entre 0 et 2.

**9.** Composé ou son sel selon la revendication 8, dans lequel

$R^1$ représente (a1) un groupe alkyle en ($C_1$-$C_6$),
$R^2$ est

(b8) un groupe halogénure d'alkyle en ($C_1$-$C_6$) ;
(b15) un groupe halogénoalkylthio en ($C_1$-$C_6$) ; ou
(b17) un groupe halogénoalkylsulfonyle en ($C_1$-$C_6$),

$G^1$, $G^2$, $G^3$ et $G^4$ représentent chacun C-$R^3$
(où
$R^3$ peut être identique ou différent et représente

(c1) un atome d'hydrogène ;
(c2) un atome d'halogène ;
(c4) un groupe formyle ;
(c5) un groupe cyano ;
(c6) un groupe alkyle en ($C_1$-$C_6$) ;
(c7) un groupe cycloalkyle en ($C_3$-$C_6$) ;
(c8) un groupe alcoxy en ($C_1$-$C_6$) ;

(c9) un groupe halogénure d'alkyle en ($C_1$-$C_6$) ;
(c12) un groupe alkylthio en ($C_1$-$C_6$) ;
(c15) un groupe halogénoalkylthio en ($C_1$-$C_6$) ;
(c19) un groupe $R^9(R^{10})N$ (où $R^9$ et $R^{10}$ peuvent être identiques ou différents et représentent chacun un atome d'hydrogène ; un groupe alkyle en ($C_1$-$C_6$) ; un groupe cycloalkyle en ($C_3$-$C_6$)-alkyle en ($C_1$-$C_6$) ; un groupe halogénure d'alkyle en ($C_1$-$C_6$), un groupe alkylcarbonyle en ($C_1$-$C_6$), un groupe alcoxycarbonyle en ($C_1$-$C_6$) ; ou un groupe phénylcarbonyle) ;
(c22) un groupe $C(R^9)=NOR^{10}$ (où $R^9$ et $R^{10}$ sont tels que définis ci-dessus) ;
(c24) un groupe alcoxycarbonyle en ($C_1$-$C_6$) ;
(c25) un groupe $R^9(R^{10})N$ carbonyle (où $R^9$ et $R^{10}$ sont tels que définis ci-dessus)),

$G^5$ représente C-$R^4$ (où $R^4$ représente (d1) un atome d'hydrogène), et
$G^6$ représente N-$R^5$, O ou S (où $R^5$ représente (e2) un groupe alkyle en ($C_1$-$C_6$)).

**10.** Composé ou son sel selon la revendication 8 ou 9, dans lequel

$R^2$ est

(b8) un groupe halogénure d'alkyle en ($C_1$-$C_6$), ou
(b15) un groupe halogénoalkylthio en ($C_1$-$C_6$),

$G^1$, $G^2$, $G^3$ et $G^4$ représentent chacun C-$R^3$
(où
$R^3$ peut être identique ou différent et représente

(c1) un atome d'hydrogène ;
(c2) un atome d'halogène ; ou
(c9) un groupe halogénoalkyle en ($C_1$-$C_6$), et

$G^6$ représente N-$R^5$ ou O (où $R^5$ représente (e2) un groupe alkyle en ($C_1$-$C_6$).

**11.** Insecticide agricole et horticole comprenant le composé ou son sel selon l'une des revendications 8 à 10 en tant qu'ingrédient actif.

**12.** Procédé d'utilisation d'un insecticide agricole et horticole, comprenant le traitement des plantes ou du sol avec une quantité efficace de l'insecticide agricole et horticole selon la revendication 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009280574 A
- JP 2010275301 A
- JP 2011079774 A
- JP 2012131780 A
- WO 2012086848 A
- WO 2013018928 A
- WO 2014157600 A
- WO 2016091731 A
- WO 2014073627 A
- EP 0374753 A
- WO 9307278 A
- WO 9534656 A
- EP 0427529 A
- EP 451878 A
- WO 03052073 A
- WO 2016039441 A
- WO 1999007351 A


### Non-patent literature cited in the description


- *J. O. C.,* 2001, vol. 66, 3474
- *J. Med. Chem.,* 2004, vol. 47, 6270
- *Proc. Natl. Acad. Sci. USA,* 1990, vol. 87, 7175-7179
- *Weed Science,* 2005, vol. 53, 728-746
- **GURA T.** Repairing the Genome's Spelling Mistakes. *Science,* 1999, vol. 285, 316-318